(19) Europäisches Patentamt
European Patent Office
Office européen des brevets

(11) Veröffentlichungsnummer: **0 165 904 B1**

(12) **EUROPÄISCHE PATENTSCHRIFT**

(45) Veröffentlichungstag der Patentschrift: **10.04.91**

(21) Anmeldenummer: **85810279.1**

(22) Anmeldetag: **17.06.85**

(51) Int. Cl.5: **A61K 31/435**, A61K 31/415, A61K 31/55, A61K 31/395, C07D 471/04, C07D 487/04, //(C07D471/04,235:00,221:00), (C07D487/04,235:00,209:00), (C07D487/04,235:00,223:00)

(54) **Substituierte bicyclische Verbindungen.**

(30) Priorität: **20.06.84 US 622421**

(43) Veröffentlichungstag der Anmeldung: **27.12.85 Patentblatt 85/52**

(45) Bekanntmachung des Hinweises auf die Patenterteilung: **10.04.91 Patentblatt 91/15**

(84) Benannte Vertragsstaaten: **AT BE CH DE FR GB IT LI LU NL SE**

(56) Entgegenhaltungen:
**EP-A- 0 068 386**
**EP-A- 0 114 573**

**Brodie et al , Endocrinology, vol.104, pp 118-121,(1979)**

**Camacho et al., JAMA, vol. 202, pp 114-120 (1967)**

**Foster et al., Journal Med. Chem., vol. 26, pp. 50-54 (1983)**

(73) Patentinhaber: **CIBA-GEIGY AG**
**Klybeckstrasse 141**
**CH-4002 Basel(CH)**

(72) Erfinder: **Browne, Leslie J. Dr.**
**41 Malapardis Road**
**Morris Plains New Jersey 07950(US)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Verwendung von Substanzen, die das Enzym Aromatase hemmen, als Arzneimittel ist bekannt. Man beabsichtigt damit, die Oestrogenproduktion im Körper zu unterdrücken und so auf Oestrogen-abhängige Krankheiten, z.B. Brustkrebs, günstig einzuwirken. Bekannt sind bisher sowohl steroidale Aromatasehemmer [siehe z.B. A.M.H. Brodie et al., Endocrinology 104, 118 (1979)] als auch einzelne nicht-steroidale Aromatasehemmer, insbesondere Aminoglutethimid und Abkömmlinge davon [siehe z.B. A.M. Camacho et al., J. Am. Med. Assoc. 202, 20 (1967); A.B. Foster et al., J. Med. Chem. 26, 50 (1983) oder EP-A-114 033].

Ferner sind aus EP-A-114 573 bestimmte 5-(substituiertes Phenyl)-imidazo[1,5-a]pyridine und 5,6,7,8-Tetrahydroderivate davon bekannt, die eine Hemmwirkung auf die Thromboxan-Synthetase ausüben.

Die Erfindung betrifft die Verwendung von substituierten Imidazo[1,5-a]-pyridin-Derivaten der Formel I

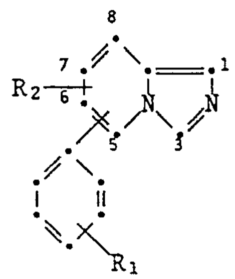

(I),

worin $R_1$ Wasserstoff, Niederalkyl; Niederalkyl, das durch Hydroxy, Niederalkoxy, Niederalkanoyloxy, Niederalkanoyl, Amino, Niederalkylamino, Diniederalkylamino, Halogen, Sulfo, Carboxy, Niederalkoxycarbonyl, Carbamoyl oder Cyan substituiert ist; Nitro, Halogen, Hydroxy, Niederalkoxy, Niederalkanoyloxy, Phenylsulfonyloxy, Niederalkylsulfonyloxy, Mercapto, Niederalkylthio, Niederalkylsulfinyl, Niederalylsulfonyl, Niederalkanoylthio, Amino, Niederalkylamino, Diniederalkylamino, Niederalkylenamino, N-Morpholino, N-Thiomorpholino, gegebenenfalls in 4-Stellung Niederalkyl-substituiertes N-Piperazino, Triniederalkylammonio, Sulfo, Niederalkoxysulfonyl, Sulfamoyl, Niederalkylsulfamoyl, Diniederalkylsulfamoyl, Formyl; Iminomethyl, das gegebenenfalls am Stickstoff durch Hydroxy, Niederalkoxy, Niederalkanoyloxy, Niederalkyl, Phenyl oder Amino substituiert ist; $C_2$-$C_7$-Alkanoyl, Benzoyl, Carboxy, Niederalkoxycarbonyl, Carbamoyl, Niederalkylcarbamoyl, Diniederalkylcarbamoyl, Cyan, 5-Tetrazolyl, gegebenenfalls Niederalkyl-substituiertes 4,5-Dihydro-2-oxazolyl oder Hydroxycarbamoyl bedeutet; und $R_2$ für Wasserstoff, Niederalkyl, phenyl-niederalkyl, Carboxy-niederalkyl, Niederalkoxycarbonylniederalkyl, Halogen, Hydroxy, Niederalkoxy, Niederalkanoyloxy, Mercapto, Niederalkylthio, Phenyl-niederalkylthio, Phenylthio, Niederalkanoylthio, Carboxy, Niederalkoxycarbonyl oder Niederalkanoyl steht; den 7,8-Dihydroderivaten davon; oder von Verbindungen der Formel I*

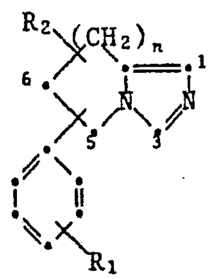

(I*),

worin n für 0, 1, 2, 3 oder 4 steht; und $R_1$ und $R_2$ wie oben unter Formel I definiert sind; wobei der Phenylring in den Resten Phenylsulfonyloxy, Phenyliminomethyl, Benzoyl, Phenyl-niederalkyl, Phenyl-niederalkylthio und Phenylthio unsubstituiert oder durch Niederalkyl, Niederalkoxy oder Halogen substituiert sein kann; wobei in einer Verbindung der Formel I* die beiden Substituenten $C_6H_4$-$R_1$ und $R_2$ an jedem der gesättigten Kohlenstoffatome des gesättigten Rings angeknüpft sein können, entweder beide am selben Kohlenstoffatom oder beide an verschiedenen Kohlenstoffatomen; worin mit "nieder" bezeichnete Reste bis zu 7 Kohlenstoffatome enthalten; Stereoisomeren, Mischungen dieser Stereoisomeren oder pharmazeutisch verwendbaren Salzen davon zur Herstellung von pharmazeutischen präparaten für die Behandlung von Krankheiten die auf eine Hemmung der Aromatase ansprechen.

Ferner betrifft die Erfindung pharmazeutische Präparate, die solche Verbindungen enthalten, neue Verbindungen dieser Art, Verfahren zur Herstellung der letzteren, pharmazeutische Präparate enthaltend die letzteren und die Verwendung der letzteren als pharmazeutische Wirkstoffe oder zur Herstellung von pharmazeutischen Präparaten.

Organische Reste, die mit dem Ausdruck "nieder" bezeichnet sind, enthalten gewöhnlich bis und mit 7, vorzugsweise bis und mit 4, Kohlenstoffatome.

Die Verbindungen der Formel I* sowie bestimmte 7,8-Dihydroderivate der Formel I enthalten wenigstens ein asymmetrisches Kohlenstoffatom. Sie können als R- oder S-Enantiomere sowie als enatiomere Mischungen davon, etwa als Racemat, auftreten. Die vorliegende Erfindung umfasst alle diese Formen, auch alle weiteren Isomere, und Mischungen von wenigstens 2 Isomeren, z.B. Diastereomerengemische oder Enantiomerengemische, die dann vorliegen können, wenn ein oder mehrere weitere asymmetrischen Zentren im Molekül vorliegen.

Niederalkyl ist z.B. n-Propyl, Isopropyl, n-Butyl, Isobutyl, sek-Butyl oder tert-Butyl, ferner n-Pentyl, Isopentyl, Neopentyl, n-Hexyl, Isohexyl oder n-Heptyl, vorzugsweise jedoch Ethyl und im besonderen Methyl.

Halogen ist z.B. Brom oder Jod, vorzugsweise Fluor und insbesondere Chlor.

In einem Aza-niederalkylenamino rest ist das Azastickstoffatom gegebenenfalls z.B. durch gegebenenfalls z.B. durch Niederalkyl substituiert. Beispiele für Oxa-, Thia- oder Aza-niederal= kylenamino sind N-Morpholino, N-Thiomorpholino oder gegebenenfalls in 4-Stellung durch Niederalkyl substituiertes N-Piperazino.

Triniederalkylammonio umfasst z.B. quaternäre Ammoniumsalze, die sich von Diniederalkylaminogruppen ableiten und die als quaternären Substituenten Niederalky enthalten. Triniederalkylammonio. ist z.B. Trimethylammonio. Die Ammoniumsalze entsprechen den unten definierten Salzen, insbesondere den Salzen, die als pharmazeutisch verwendbare, nicht toxische Säureadditionsalze angegeben sind, und vor allem den Salzen, die mit Halogenwasserstoffsäuren, Schwefelsäure oder Phosphorsäure gebildet werden.

Sulfo steht für eine $-SO_3H$ -Gruppe.

$C_1$-Alkanoyl entspricht Formyl.

Niederalkoxy ist vorzugsweise Methoxy oder Ethoxy, ferner z.B. n-Propoxy, Isopropoxy, n-Butoxy, Isobutoxy oder tert-Butoxy.

Niederalkanoyloxy ist z.B. Formyloxy, Acetoxy, Propionyloxy oder Pivaloyloxy.

Niederalkanoyl ist z.B. Formyl, Acetyl, Propionyl oder Pivaloyl. Halogen-$C_2$-$C_7$-Alkanoyl ist vorzugsweise Trifluoracetyl. Niederalkanoylamino ist vorzugsweise Acetylamino oder Propionylamino, kann aber z.B. auch Formylamino sein.

Niederalkoxycarbonyl ist vorzugsweise Methoxycarbonyl oder Ethoxycarbonyl. Niederalkoxycarbonyloxy ist z.B. Methoxycarbonyloxy oder Ethoxycarbonyloxy.

Niederalkylamino bedeutet z.B. Methylamino, Ethylamino, n-Propylamino oder Isopropylamino. Diniederalkylamino ist z.B. Dimethylamino, Ethylmethylamino oder Diethylamino. Niederalkylenamino enthält z.B. 2 bis 7, vorzugsweise 4 bis 6, Ringkohlenstoffatome und ist z.B. N-Pyrrolidino oder N-Piperidino.

Niederalkylthio ist z.B. Methylthio, Ethylthio, n-Propylthio oder Isopropylthio, während Niederalkylsulfinyl z.B. Methylsulfinyl bedeutet und Niederalkylsulfonyl z.B. für Methylsulfonyl oder Ethylsulfonyl steht. Niederalkanoylthio ist vorzugsweise Formylthio oder Acetylthio.

Niederalkoxysulfonyl ist z.B. Methoxysulfonyl oder Ethoxysulfonyl. Niederalkylsulfamoyl ist z.B. N-Methyl- oder N-Ethylsulfamoyl, während Diniederalkylsulfamoyl z.B. Dimethyl- oder Diethylsulfamoyl bedeutet.

Niederalkylcarbamoyl ist z.B. N-Methylcarbamoyl oder N-Ethylcarbamoyl, während Diniederalkylcarbamoyl z.B. Dimethyl- oder Diethylcarbamoyl bedeutet.

Die erfindungsgemässen Verbindungen bilden Säureadditionssalze. Diese werden vorzugsweise mit solchen anorganischen oder organischen Säuren, die pharmazeutisch verwendbare Säureadditionssalze ergeben, hergestellt. Solche Säuren sind z.B. starke Mineralsäuren, wie Salzsäure, Schwefelsäure oder Phosphorsäure, oder organische Säuren, insbesondere aliphatische oder aromatische Carbon- oder Sulfonsäuren, z.B. Ameisen-, Essig-, Propion-, Bernsteinsäure-, Glykol-, Milch-, Hydroxybernsteinsäure-, Wein-, Zitronen-, Malein-, Fumar-, Hydroxymalein-, Brenztrauben-, Phenylessig-, Benzoe-, 4-Aminobenzoe-, Anthranil-, 4-Hydroxybenzoe-, Salicyl-, 4-Aminosalicyl-, Pamoe-, Glukon-, Nikotin-, Methansulfon-, Ethansulfon-, Halogenbenzolsulfon-, p-Toluolsulfon-, Naphthalinsulfon-, Sulfanil- oder Cyclohexylsulfaminsäure; oder andere saure organische Substanzen, z.B. Ascorbinsäure. Ferner können Salze auch z.B. mit Aminosäuren, wie Arginin oder Lysin, gebildet werden.

Verbindungen der Erfindung, die saure Gruppen enthalten, z.B. eine freie Carboxy- oder Sulfogruppe, bilden insbesondere Metall- oder Ammoniumsalze, wie Alkalimetall- oder Erdalkalimetallsalze, z.B. Natrium-,

Kalium-, Magnesium- oder Calciumsalze, ferner Ammoniumsalze, die von Ammoniak oder geeigneten organischen Aminen abgeleitet sind. Hier kommen insbesondere aliphatische, cycloaliphatische, cycloaliphatisch-aliphatische oder araliphatische primäre, sekundäre oder tertiäre Mono-, Di- oder Polyamine in Frage, wie Niederalkylamine, z.B. Di- oder Triethylamin, Hydroxy-niederalkylamine, z.B. 2-Hydroxyethylamin, Bis-(2-hydroxyethyl)-amin oder Tris-(2-hydroxyethyl)-amin, basische aliphatische Ester oder Carbonsäuren, z.B. 4-Aminobenzoesäure-2-diethylaminoethylester, Niederalkylenamine, z.B. 1-Ethylpiperidin, Cycloalkylamine, z.B. Dicyclohexylamin, Benzylamine, z.B. N,N'-Dibenzylethylendiamin; ferner heterocyclische Basen, etwa vom Pyridintyp, z.B. Pyridin, Collidin oder Chinolin.

Bei Gegenwart mehrerer saurer oder basischer Gruppen können Mono- oder Polysalze gebildet werden. Erfindungsgemässe Verbindungen, die eine saure und eine basische Gruppe aufweisen, können ferner in Form von inneren Salzen, d.h. als Zwitterionen, vorliegen oder ein Teil des Moleküls kann als inneres Salz vorliegen und ein anderer Teil des Moleküls als normales Salz. Die oben erwähnten pharmazeutisch verwendbaren Salze sind bevorzugt. Zur Isolierung oder Reinigung können auch pharmazeutisch ungeeignete Salze Verwendung finden, z.B. die Pikrate.

Die Verbindungen der Erfindung weisen wertvolle pharmakologische Eigenschaften auf, z.B. hemmen sie die Aromatase bei Säugetieren einschliesslich Menschen. Beispielsweise hemmen diese Verbindungen die metabolische Umwandlung von Androgenen zu Oestrogenen. Daher sind Verbindungen der Formel I und I* z.B. nützlich bei der Behandlung von Gynäkomastie, d.h. männlicher Brustentwicklung, dadurch, dass die Aromatisierung der Steroide bei solchen Männchen gehemmt wird. Ferner sind Verbindungen der Formel I und I* z.B. nützlich bei der Behandlung von Krankheiten, die Oestrogen-abhängig sind, einschliesslich Oestrogen-abhängigem Brustkrebs, insbesondere bei postmenopausalen Weibchen, dadurch dass die Oestrogen-Synthese gehemmt wird. Diese Wirkungen können durch in vitro-Versuche oder in vivo-Tierversuche, vorzugsweise an Säugetieren, z.B. an Meerschweinchen, Mäusen, Ratten, Katzen, Hunden oder Affen, nachgewiesen werden.

Die in vitro-Hemmung der Aromataseaktivität kann z.B. mit der Methode, die in J. Biol. Chem. 249 , 5364 (1974) beschrieben ist, gezeigt werden. Ferner können $IC_{50}$-Werte für die Aromatasehemmung z.B. in vitro aus enzymkinetischen Studien, die die Hemmung der Umwandlung von $4\text{-}^{14}C$-Androstendion zu $4\text{-}^{14}C$-Oestron in menschlichen plazentalen Mikrosomen betreffen, erhalten werden. Die $IC_{50}$-Werte der erfindungsgemässen Verbindungen liegen etwa zwischen $10^{-5}$ und etwa $10^{-9}$ mol/l. In vivo kann die Aromatasehemmung z.B. durch die Unterdrückung des ovarialen Oestrogengehalts weiblicher Ratten gezeigt werden, die zunächst mit Stutenserumgonadotropin und 2 Tage später mit menschlichem Choriongonadotropin injiziert werden, am folgenden Tag p.o. mit einer Verbindung der Erfindung behandelt werden und 1h später mit Androstendion. Die minimale effektive Dosis der erfindungsgemässen Verbindungen liegt zwischen ungefähr 0,01 und ungefähr 10 mg/kg oder weniger. Die Antitumorwirkung, insbesondere bei Oestrogenabhängigen Tumoren, kann in vivo z.B. bei DMBA-induzierten Mammatumoren an weiblichen Sprague-Dawley-Ratten gezeigt werden. Die Anwendung von erfindungsgemässen Verbindungen bewirkt eine fast vollständige Regression der Tumoren und unterdrückt weiterhin das Auftreten neuer Tumoren bei täglichen Dosen von ungefähr 1 bis ungefähr 20 mg/kg p.o. oder weniger.

Ueberraschenderweise fehlt den erfindungsgemässen Verbindungen, die in vitro und in vivo als effektive Aromatasehemmer wirken, eine Hemmwirkung auf die Spaltung der Cholesterin-Seitenkette in vivo, da sie keine adrenale Hypertrophie induzieren, was durch endokrine Organuntersuchungen gezeigt werden kann.

Aufgrund ihrer pharmakologischen Eigenschaften als Aromatasehemmer können die erfindungsgemässen Verbindungen als Medikamente verwendet werden, z.B. in Form von pharmazeutischen Präparaten, z.B. für die Behandlung hormoneller Krankheiten, wie Oestrogen-abhängiger Tumoren, im besonderem Mammakarzinom, und Anomalien, z.B. Gynäkomatie, bei Warmblütern einschliesslich Menschen. Die neuen Verbindungen sind ferner wertvolle Zwischenprodukte zur Herstellung anderer pharmazeutisch wirksamer Verbindungen.

Im besonderen betrifft die Erfindung die oben angegebere Verwendung von Verbindungen der Formel I, worin $R_1$ Niederalkyl; Niederalkyl, das durch Hydroxy, Amino, Diniederalkylamino, 1-5 Fluoratome, Carboxy, Niederalkoxycarbonyl, Carbamoyl oder Cyan substituiert ist; Nitro, Halogen, Hydroxy, Niederalkoxy, Amino, Niederalkylamino, Diniederalkylamino, Sulfo, Sulfamoyl, Formyl, Iminomethyl; Iminomethyl, das am Stickstoff durch Hydroxy, Niederalkoxy, Niederalkanoyloxy, Niederalkyl oder Phenyl substituiert ist; Carboxy, Niederalkoxycarbonyl, Carbamoyl, Niederalkylcarbamoyl, Diniederalkylcarbamoyl oder Cyan bedeutet; und $R_2$ für Wasserstoff, Niederalkyl, Niederalkoxy oder Halogen steht; oder von Verbindungen der Formel I*, worin n für 1, 2 oder 3 steht; $R_1$ wie oben unter Formel I definiert ist und $R_2$ Wasserstoff, Niederalkyl, Phenyl-niederalkyl, Carboxy-niederalkyl, Niederalkoxycarbonyl-niederalkyl, Halogen, Niederalkoxy, Niederalkylthio, Phenyl-niederalkylthio, Phenylthio, Carboxy, Niederalkoxycarbonyl oder Niederalkanoyl bedeutet;

wobei in einer Verbindung der Formel I* die beiden Substituenten $C_6H_4$-$R_1$ und $R_2$ an jedem der gesättigten Kohlenstoffatome des gesättigten Rings angeknüpft sein können, entweder beide am selben Kohlenstoffatom oder beide an verschiedenen Kohlenstoffatomen; Stereoisomeren, Mischungen dieser Stereoisomeren oder pharmazeutisch verwendbaren Salzen davon.

In ersten Linie betrifft die Erfindung die oben angegebere Verwendung von Verbindungen der Formel I, worin $R_1$ Niederalkyl, Hydroxy-niederalkyl, Halogen, Amino, Formyl, Carboxy, Niederalkoxycarbonyl, Carbamoyl, N-Niederalkylcarbamoyl oder Cyan bedeutet; und $R_2$ Wasserstoff ist; oder von Verbindungen der Formel I*, worin n für 1, 2 oder 3 steht; $R_1$ wie oben unter Formel I definiert ist und $R_2$ Wasserstoff, Niederalkylthio, Niederalkoxycarbonyl, Phenyl-niederalkyl, Carboxy-niederalkyl oder Niederalkoxycarbonyl-niederalkyl bedeutet; wobei in einer Verbindung der Formel I* die beiden Substituenten $C_6H_4$-$R_1$ und $R_2$ an jedem der gesättigten Kohlenstoffatome des gesättigten Rings angeknüpft sein können, entweder beide am selben Kohlenstoffatom oder beide an verschiedenen Kohlenstoffatomen; Stereoisomeren, Mischungen dieser Stereoisomeren oder pharmazeutisch verwendbaren Salzen davon.

Die Erfindung betrifft ferner pharmazeutische, die eine Verbindung der Formel I, worin $R_1$ Halogen, Hydroxy, Mercapto oder Niederalkyl, das durch Niederalkoxy, Niederalkanoyloxy oder Halogen substituiert ist, bedeutet; und R2 für Wasserstoff, Niederalkyl, Niederalkoxy oder Halogen steht; oder eine Verbindung der Formel I*, worin n für 2 steht, $R_1$ wie oben unter Formel I definiert ist und $R_2$ Wasserstoff, Niederalkyl, Phenyl-niederalkyl, Carboxy-niederalkyl, Niederalkoxycarbonyl-niederalkyl, Halogen, Niederalkoxy, Niederalkylthio, Phenyl-niederalkylthio, Phenylthio, Carboxy, Niederalkoxycarbonyl oder Niederalkanoyl bedeutet; wobei in einer Verbindung der Formel I* die beiden Substituenten $C_6H_4$-$R_1$ und $R_2$ an jedem der gesättigten Kohlenstoffatome des gesättigten Rings angeknüpft sein können, entweder beide am selben Kohlenstoffatom oder beide an verschiedenen Kohlenstoffatomen; wobei mit "nieder" bezeichnete Reste bis zu 7 Kohlenstoffatome enthalten; ein Stereoisomer, eine Mischung dieser Stereoisomeren oder ein pharmazeutisch verwendbares Salz davon zusammen mit einem oder mehreren pharmazeutisch annehmbaren Trägermateriglier enthelten.

Die Erfindung betrifft ferner Verbindungen der Formel I, worin $R_1$ Wasserstoff, Niederalkyl; $C_2$-$C_7$-Alkyl, das durch Hydroxy, Niederalkoxy, Halogen oder Niederalkanoyloxy substituiert ist; Niederalkyl, das durch Niederalkanoyl, Amino, Niederalkylamino, Diniederalkylamino, Sulfo, Niederalkoxycarbonyl, Carbamoyl oder Cyan substituiert ist; Nitro, Niederalkoxy, Niederalkanoyloxy, Phenylsulfonyloxy, Niederalkylsulfonyloxy, Niederalkylthio, Niederalkylsulfinyl, Niederalkylsulfonyl, Niederalkanoylthio, Amino, Niederalkylamino, Diniederalkylamino, Niederalkylenamino, N-Morpholino, N-Thiomorpholino, gegebenenfalls in 4-Stellung durch Niederalkyl substituiertes N-Piperazino, Triniederalkylammonio, Sulfo, Niederalkoxysulfonyl, Sulfamoyl, Niederalkylsulfamoyl, Diniederalkylsulfamoyl; Iminomethyl, das gegebenenfalls am Stickstoff durch Hydroxy, Niederalkoxy, Niederalkanoyloxy, Niederalkyl, Phenyl oder Amino substituiert ist; $C_2$-$C_7$-Alkanoyl oder Benzoyl bedeutet, und $R_2$ für Wasserstoff, Niederalkyl, Phenyl-niederalkyl, Carboxy-niederalkyl, Niederalkoxycarbonyl-niederalkyl, Halogen, Hydroxy, Niederalkoxy, Niederalkanoyloxy, Mercapto, Niederalkylthio, Phenyl-niederalkylthio, Phenylthio, Niederalkanoylthio, Carboxy, Niederalkoxycarbonyl oder Niederalkanoyl steht; die 7,8-Dihydroderivate davon und ferner solche 7,8-Dihydroderivate, worin $R_1$ Hydroxymethyl, Niederalkoxymethyl, Halogenmethyl, Niederalkanoyloxymethyl, Carboxy-niederalkyl, Halogen, Hydroxy, Mercapto, Formyl, Carboxy, Niederalkoxycarbonyl, Carbamoyl, Niederalkylcarbamoyl, Diniederalkylcarbamoyl, Cyan, 5-Tetrazolyl, gegegebenenfalls durch Niederalkyl substituiertes 4,5-Dihydro-2-oxazolyl oder Hydroxycarbamoyl bedeutet und $R_2$ wie oben unter Formel I definiert ist; und Verbindungen der Formel I*, worin n für 0, 1, 2, 3 oder 4 steht; $R_1$ wie oben unter Formel I definiert ist oder $R_1$ zusätzlich Hydroxymethyl, Niederalkoxymethyl, Halogenmethyl, Niederalkanoyloxymethyl, Carboxy-niederalkyl, Halogen, Hydroxy, Mercapto, Formyl, Carboxy, Niederalkoxycarbonyl, Carbamoyl, Niederalkylcarbamoyl, Diniederalkylcarbamoyl, Cyan, 5-Tetrazolyl, gegebenenfalls durch Niederalkyl substituiertes 4,5-Dihydro-2-oxazolyl oder Hydroxycarbamoyl bedeuten kann, wenn n für 0, 1, 3 oder 4 steht oder wenn n für 2 steht und $R_2$ Phenyl-niederalkyl, Carboxy-niederalkyl, Niederalkoxycarbonyl-niederalkyl, Niederalkanoyloxy, Mercapto, Niederalkylthio, Phenyl-niederalkylthio, Phenylthio, Niederalkanoylthio, Carboxy, Niederalkoxycarbonyl oder Niederalkanoyl bedeutet; und $R_2$ wie oben unter Formel I definiert ist; wobei der Phenylring in den Resten Phenylsulfonyloxy, Phenyliminomethyl, Benzoyl, Phenyl-niederalkyl, Phenyl-niederalkylthio und Phenylthio unsubstituiert oder durch Niederalkyl, Niederalkoxy oder Halogen substituiert sein kann; wobei in einer Verbindung der Formel I* die beiden Substituenten $C_6H_4$-$R_1$ und $R_2$ an jedem der gesättigten Kohlenstoffatome des gesättigten Rings angeknüpft sein können, entweder beide am selben Kohlenstoffatom oder beide an verschiedenen Kohlenstoffatomen; wobei mit "nieder" bezeichnete Reste bis zu 7 Kohlenstoffe enthalten; Stereoisomere, Mischungen dieser Stereoisomeren und Salze davon.

Die Erfindung betrifft besonders Verbindungen der Formel I, worin $R_1$ Niederalkyl, Hydroxy-$C_2$-$C_7$-alkyl; Niederalkyl, das durch Amino, Diniederalkylamino, 2 bis 5 Fluoratome, Niederalkoxycarbonyl, Carbamoyl

oder Cyan substituiert ist; Nitro, Niederalkoxy, Amino, Niederalkylamino, Diniederalkylamino, Sulfo, Sulfamoyl, Iminomethyl oder Iminomethyl, das am Stickstoff durch Hydroxy, Niederalkoxy, Niederalkanoyloxy, Niederalkyl oder Phenyl substituiert ist, bedeutet und $R_2$ für Wasserstoff, Niederalkyl, Niederalkoxy oder Halogen steht; und Verbindungen der Formel I*, worin n für 1, 2 oder 3 steht; $R_1$ wie oben unter Formel I definiert ist oder $R_1$ zusätzlich Hydroxymethyl, Carboxy-niederalkyl, Halogen, Hydroxy, Formyl, Carboxy, Niederalkoxycarbonyl, Carbamoyl, Niederalkylcarbamoyl, Diniederalkylcarbamoyl oder Cyan bedeuten kann, wenn n für 1 oder 3 steht oder wenn n für 2 steht und $R_2$ Phenyl-niederalkyl, Carboxy-niederalkyl, Niederalkoxycarbonyl-niederalkyl, Niederalkylthio, Phenyl-niederalkylthio, Phenylthio, Carboxy, Niederalkoxycarbonyl oder Niederalkanoyl bedeutet ; und $R_2$ Wasserstoff, Niederalkyl, Phenyl-niederalkyl, Carboxy-niederalkyl, Niederalkoxycarbonyl-niederalkyl, Halogen, Niederalkoxy, Niederalkylthio, Phenyl-niederalkylthio, Phenylthio, Carboxy, Niederalkoxycarbonyl oder Niederalkanoyl bedeutet; wobei in einer Verbindung der Formel I* die beiden Substituenten $C_6H_4$-$R_1$ und $R_2$ an jedem der gesättigten Kohlenstoffatome des gesättigten Rings angeknüpft sein können, entweder beide am selben Kohlenstoffatom oder beide an verschiedenen Kohlenstoffatomen; Stereoisomere, Mischungen dieser Stereoisomeren und pharmazeutisch verwendbare Salze davon.

Die Erfindung betrifft ganz besonders Verbindungen der Formel I*, worin n für 1, 2 oder 3 steht; $R_1$ Niederalkyl, Amino, Niederalkylamino oder Diniederalkylamino bedeutet oder $R_1$ zusätzlich Hydroxymethyl, Halogen, Formyl, Carboxy, Niederalkoxycarbonyl, Carbamoyl, Niederalkylcarbamoyl, Diniederalkylcarbamoyl oder Cyan bedeuten kann, wenn n für 1 oder 3 steht oder wenn n für 2 steht und $R_2$ Phenyl-niederalkyl, Carboxy-niederalkyl, Niederalkoxycarbonyl-niederalkyl, Niederalkylthio, Carboxy oder Niederalkoxycarbonyl bedeutet; und $R_2$ Wasserstoff, Niederalkyl, Phenyl-niederalkyl, Carboxy-niederalkyl, Niederalkoxycarbonyl-niederalkyl, Niederalkylthio, Carboxy oder Niederalkoxycarbonyl bedeutet; wobei die beiden Substituenten $C_6H_4$-$R_1$ und $R_2$ an jedem der gesättigten Kohlenstoffatome des gesättigten Rings angeknüpft sein können, entweder beide am selben Kohlenstoffatom oder beide an verschiedenen Kohlenstoffatomen; Stereoisomere, Mischungen dieser Stereoisomeren und pharmazeutisch verwendbare Salze davon. Eine besondere Ausführungsform der Erfindung bilden die Verbindungen der Formel Ia

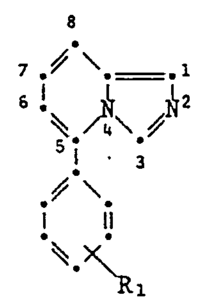

(Ia)

worin $R_1$ Cyan, Nitro oder $C_1$-$C_4$-Alkyl bedeutet, die 7,8-Dihydroderivate davon und die 5,6,7,8-Tetrahydroderivate davon mit der Formel Ib

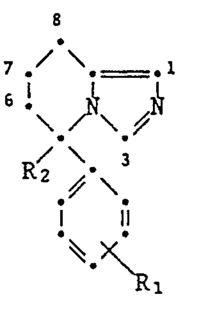

(Ib)

worin $R_1$ wie oben unter Formel Ia definiert ist und $R_2$ Wasserstoff, $C_1$-$C_4$-Alkyl, Benzyl, Halogen, $C_1$-$C_4$-Alkoxy, $C_1$-$C_4$-Alkylthio, Benzylthio, 2-Phenylethylthio, Diphenylmethylthio, Phenylthio, $C_1$-$C_4$-Alkanoyloxy, $C_1$-$C_4$-Alkanoylthio, Carboxy-$C_1$-$C_4$-alkyl, $C_1$-$C_4$-Alkoxycarbonyl-$C_1$-$C_4$-alkyl oder $C_1$-$C_4$-Alkanoyl bedeutet, Stereoisomere, Mischungen dieser Stereoisomeren und Salze dieser Verbindungen.

Die 5,6,7,8-Tetrahydroderivate der Formel Ib besitzen ein chirales Kohlenstoffatom in 5-Stellung. Die 5R- und 5S-Enantiomeren sowie die 5(R,S)-Racemate fallen unter den Umfang der vorliegenden Erfindung.

Die zur Definition der Verbindungen der Formel Ia und Ib verwendeten Allgemeinbegriffe haben

vorzugsweise die folgenden Bedeutungen:

$C_1$-$C_4$-Alkyl $R_1$ oder $R_2$ ist z.B. Ethyl, n-Propyl, Isopropyl, n-Butyl, sek-Butyl oder tert-Butyl, und vorzugsweise Methyl.

Halogen $R_2$ ist z.B. Fluor oder Brom, oder vorzugsweise Chlor.

$C_1$-$C_4$-Alkoxy bedeutet z.B. Methoxy oder Ethoxy, und $C_1$-$C_4$-Alkylthio ist z.B. Methyl- oder Ethylthio.

Carboxy-$C_1$-$C_4$-alkyl $R_2$ ist z.B. Carboxymethyl oder 2-Carboxyethyl.

$C_1$-$C_4$-Alkoxycarbonyl-$C_1$-$C_4$-alkyl $R_2$ ist z.B. Methoxy- oder Ethoxycarbonylmethyl.

$C_1$-$C_4$-Alkanoyl $R_2$ ist z.B. Formyl, Acetyl oder Propionyl.

Die Erfindung betrifft insbesondere die Verbindungen der Formel Ia, worin $R_1$ Cyan bedeutet, die 7,8-Dihydroderivate davon, und die 5,6,7,8-Tetrahydroderivate davon mit der Formel Ib, worin $R_1$ Cyan bedeutet und $R_2$für Wasserstoff, $C_1$-$C_4$-Alkyl, $C_1$-$C_4$-Alkoxy, $C_1$-$C_4$-Alkylthio, Benzylthio, 2-Phenylethylthio, Diphenylmethylthio, Phenylthio oder $C_1$-$C_4$-Alkanoyl steht und pharmazeutisch verwendbare Säureadditionssalze von Verbindungen der Formel Ia und Ib.

Vor allem bevorzugt sind die Verbindungen der Formel Ia, worin $R_1$ Cyan bedeutet, die 7,8-Dihydroderivate davon und die 5,6,7,8-Tetrahydroderivate davon mit der Formel Ib, worin $R_1$ wie oben unter Formel Ia definiert ist und $R_2$ für Wasserstoff steht, und pharmazeutisch verwendbare Säureadditionssalze davon.

In erster Linie betrifft die Erfindung die Verbindungen der Formel Ic

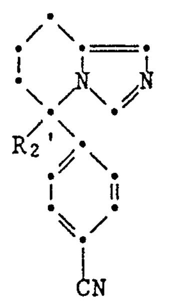

(Ic)

worin $R_2$' Wasserstoff, $C_1$-$C_4$-Alkyl, $C_1$-$C_4$-Alkoxy, $C_1$-$C_4$-Alkylthio, Benzylthio, 2-Phenylethylthio, Diphenylmethylthio, Phenylthio oder $C_1$-$C_4$-Alkanoyl bedeutet, und pharmazeutisch verwendbare Säureadditionssalze davon.

Speziell bevorzugt ist die Verbindung der Formel Ic, worin $R_2$' Wasserstoff bedeutet, mit dem Namen 5-(p-Cyanphenyl)-5,6,7,8-tetrahydroimidazo[1,5-a]pyridin, und pharmazeutisch verwendbare Säureadditionssalze davon.

Weiterhin betrifft die Erfindung Verbindungen der Formel Ia, worin $R_1$ Wasserstoff, p-Toluolsulfonyloxy, Benzolsulfonyloxy, Methylsulfonyloxy, Sulfo, Amino, Carbamoyl, $C_1$-$C_4$-Alkylcarbamoyl oder Hydroxyiminomethyl bedeutet; und die 5,6,7,8-Tetrahydroverbindungen der Formel Ib, worin $R_1$wie oben unter Formel Ia definiert ist oder Halogen bedeutet und $R_2$ für Wasserstoff, $C_1$-$C_4$-Alkyl, Benzyl, Halogen, $C_1$-$C_4$-Alkoxy, $C_1$-$C_4$-Alkanoyloxy, $C_1$-$C_4$-Alkylthio, Benzylthio, 2-Phenylethylthio, Diphenylmethylthio, Phenylthio, $C_1$-$C_4$-Alkanoylthio, Carboxy-$C_1$-$C_4$-alkyl, $C_1$-$C_4$-Alkoxycarbonyl-$C_1$-$C_4$-alkyl oder $C_1$-$C_4$-Alkanoyl steht; und pharmazeutisch verwendbare Salze davon.

Von den letztgehannten Verbindungen der Formel Ia und Ib sind ganz besonders die Verbindungen der Formel Ia und Ib, worin $R_1$ Carbamoyl bedeutet, und die Verbindungen der Formel Ib, worin $R_1$ Halogen bedeutet, von Interesse.

Von den letztgehannten Verbindungen der Formel Ia und Ib sind ferner bevorzugt die Verbindungen der Formel Ia, worin $R_1$ Wasserstoff, p-Toluolsulfonyloxy, Benzolsulfonyloxy, Methylsulfonyloxy, Sulfo, Amino oder Hydroxyiminomethyl bedeutet; und die 5,6,7,8-Tetrahydroverbindungen der Formel Ib, worin $R_1$ wie oben unter Formel Ia angegeben definiert ist und $R_2$ für Wasserstoff steht; oder worin $R_1$ Wasserstoff, Halogen, p-Toluolsulfonyloxy, Benzolsulfonyloxy, Methylsulfonyloxy, Sulfo, Amino, Carboxy, Carbamoyl, $C_1$-$C_4$-Alkylcarbamoyl, Formyl oder Hydroxyiminomethyl bedeutet und $R_2$ für $C_1$-$C_4$-Alkyl, Benzyl, Halogen, $C_1$-$C_4$-Alkoxy, $C_1$-$C_4$-Alkanoyloxy, $C_1$-$C_4$-Alkylthio, Benzylthio, 2-Phenylethylthio, Diphenylmethylthio, Phenylthio, $C_1$-$C_4$-Alkanoylthio, Carboxy-$C_1$-$C_4$-alkyl, $C_1$-$C_4$-Alkoxycarbonyl-$C_1$-$C_4$-alkyl oder $C_1$-$C_4$-Alkanoyl steht; und pharmazeutisch verwendbare Salze davon.

Ganz allgemein sind solche Verbindungen der Erfindung bevorzugt, worin der Substituent $C_6H_4$-$R_1$ in der 5- oder 7-Stellung des bicyclischen Ringsystems angeknüpft ist, und von besonderem Interesse sind die Verbindungen, worin $C_6H_4$-$R_1$ in der 5-Stellung angeknüpft ist. In erfindungsgemässen Verbindungen ist der Substituent $R_1$ vorzugsweise in para- oder meta-Stellung, insbesondere in para-Stellung, des Phenylrings angeknüpft. Die Zahl n in einer Verbindung der Formel I* ist vorzugsweise 1, 2 oder 3, insbesondere 1 oder 2, und vor allem 2. Von den erfindungsgemässen Verbindungen sind die aromatischen, z.B. die mit der Formel I oder Ia, gegenüber den entsprechenden 7,8-Dihydroderivaten bevorzugt. Am meisten bevorzugt sind die Verbindungen mit einem völlig hydrierten Ring, z.B. von der Formel I* oder Ib.

Vor allem sind die in den Beispielen beschriebenen Verbindungen und pharmazeutisch verwendbare Salze davon bevorzugt, ferner pharmazeutische Präparate enthaltend diese Verbindungen und ihre Verwendung als pharmazeutische Wirkstoffe oder zur Herstellung von pharmazeutischen Präparaten.

Die Verbindungen der Formel I oder I*, welche die Verbindungen der Formel Ia und Ib umfassen, werden in an sich bekannter Weise hergestellt, vorzugaweise dadurch, dass man

a) eine Verbindung der Formel II

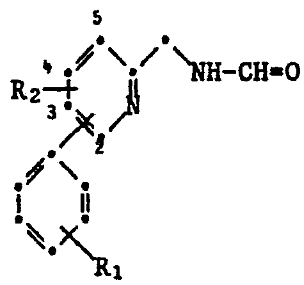

(II)

oder ein 4,5-Dihydroderivat davon, ringschliesst, um eine Verbindung der Formel I bzw. ein 7,8-Dihydroderivat davon zu erhalten, oder

b) eine Verbindung der Formel III

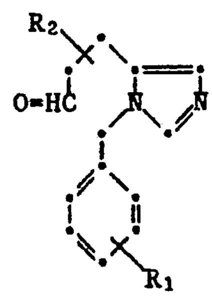

(III),

worin $R_2$ wie angedeutet an jedem der Kohlenstoffatome einschliesslich des Carbonylkohlenstoffs angeknüpft sein kann, ringschliesst, um ein 7,8-Dihydroderivat einer Verbindung der Formel I zu erhalten; worin der Substituent $C_6H_4$-$R_1$ in 5-Stellung angeknüpft ist, oder

c)/f) in einer Verbindung der Formel IV oder VII

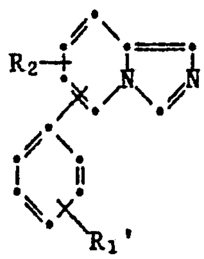

(IV)

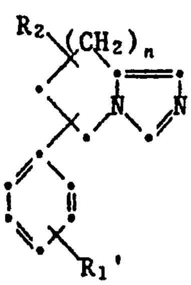

(VII)

oder in einem 7,8-Dihydroderivat der Formel IV, worin $R_1'$ jeweils eine Gruppe bedeutet, die in Cyan umgewandelt werden kann, $R_1'$ in Cyan umwandelt, um eine Verbindung der Formel I, ein 7,8-Dihydroderivat davon bzw. eine Verbindung der Formel I* zu erhalten, worin $R_1$ Cyan bedeutet, oder

d) eine Verbindung der Formel V

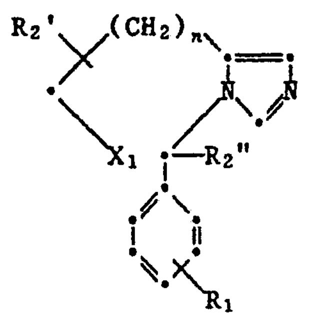

(V),

worin wenigstens einer der Reste $R_2'$ und $R_2''$ Wasserstoff und der andere einen Rest $R_2$ wie unter Formel I* definiert bedeutet, und $X_1$ eine Abgangsgruppe bedeutet, und $R_2'$ an jedem der Kohlenstoffatome wie angedeutet angeknüpft sein kann, ringschliesst, um eine Verbindung der Formel I* zu erhalten, worin der Substituent $C_6H_4$-$R_1$ in 5-Stellung angeknüpft ist; oder $X_1$ eine Gruppe =CH-COOH oder einen Niederalkylester davon bedeutet, $R_2'$ für Wasserstoff steht und $R_2''$ wie unter Formel I* definiert ist, ringschliesst, um eine Verbindung der Formel I* zu erhalten, worin der Substituent $C_6H_4$-$R_1$ in 5-Stellung angeknüpft ist und die 6-Stellung durch Carboxymethyl oder Niederalkoxycarbonylmethyl substituiert ist, oder

e) eine Verbindung der Formel VI

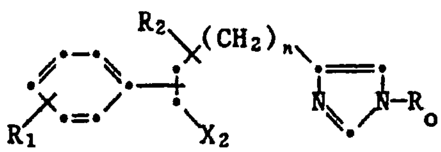

(VI)

worin die Substituenten $C_6H_4$-$R_1$ und $R_2$ an jedem der Kohlenstoffatome wie angedeutet angeknüpft sein können, entweder beide Reste am selben Kohlenstoffatom oder beide an verschiedenen Kohlenstoffatomen, $R_0$ eine NH-Schutzgruppe oder Wasserstoff bedeutet und $X_2$ eine Abgangsgruppe bedeutet, ringschliesst, um eine Verbindung der Formel I* zu erhalten; oder

g) eine Verbindung der Formel IX

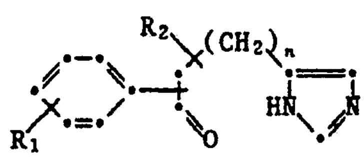

(IX)

worin die Substituenten $C_6H_4$-$R_1$ und $R_2$ an jedem der Kohlenstoffatome einschliesslich dem Carbonylkohlenstoff wie angedeutet angeknüpft sein können, entweder beide Reste am selben Kohlenstoffatom oder beide an verschiedenen Kohlenstoffatomen, gegebenenfalls unter reduktiven Bedingungen, ringschliesst, um ein 7,8-Dihydroderivat einer Verbindung der Formel I oder, im Falle von reduktiven Bedingungen, eine Verbindung der Formel I* erhält, oder

h) eine Verbindung analog zu Formel I, oder ein 7,8-Dihydroderivat davon, oder eine Verbindung analog zu Formel I*, welche jeweils eine zusätzliche Carboxygruppe in 1- oder 3-Stellung enthält, decarboxyliert, um eine Verbindung der Formel I, ein 7,8-Dihydroderivat davon oder eine Verbindung der Formel I* zu erhalten; wobei in den Ausgangsverbindungen der Formel II bis VII und IX die Symbole n, $R_1$ und $R_2$ die unter Formel I bzw. I* angegebenen Bedeutungen haben; und/oder eine Verbindung der Formel I, oder ein 7,8-Dihydroderivat davon, zu einem entsprechenden 5,6,7,8-Tetrahydroderivat der Formel I* reduziert, wobei gegebenenfalls gleichzeitig eine Reduktion des Substituenten $R_1$ und/oder $R_2$ in einen anderen Substituenten $R_1$ und/oder $R_2$ stattfindet; und/oder eine Verbindung der Formel I*, worin $R_2$ Carboxy bedeutet, decarboxyliert, um eine Verbindung der Formel I* zu erhalten, worin $R_2$ Wasserstoff ist; und/oder eine erhaltene Verbindung in eine andere Verbindung der Erfindung umwandelt und/oder ein erhaltenes Salz in die freie Verbindung oder in ein anderes Salz umwandelt und/oder eine erhaltene freie Verbindung in ein Salz umwandelt und/oder ein erhaltenes Gemisch von Isomeren oder Racematen in

die einzelnen Isomeren oder Racematen auftrennt und/oder ein enantiomeren Gemisch, etwa ein Racemat, in die optischen Antipoden aufspaltet.

Die Verbindungen der Formel Ia oder Ib werden vorzugsweise dadurch hergestellt, dass man

a) eine Verbindung der Formel IIa

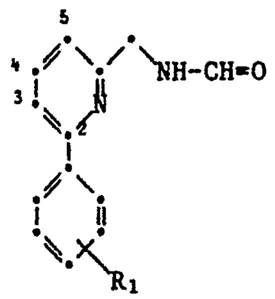

(IIa),

worin $R_1$ wie oben unter Formel Ia definiert ist, oder ein 4,5-Dihydroderivat davon, unter sauren Bedingungen cyclisiert, um eine Verbindung der Formel Ia oder ein 7,8-Dihydroderivat davon zu erhalten, oder

b) zur Herstellung eines 7,8-Dihydroderivates einer Verbindung der Formel Ia, eine Verbindung der Formel IIIa

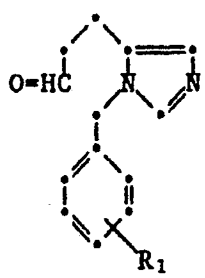

(IIIa),

worin $R_1$ wie oben unter Formel Ia definiert ist, unter basischen Bedingungen cyclisiert, oder

c) in einer Verbindung der Formel IVa

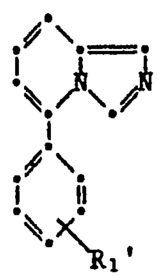

(IVa),

worin $R_1'$ einen Rest bedeutet, der in Cyan umgewandelt werden kann, oder in einem 7,8-Dihydroderivat davon, $R_1'$ in Cyan umwandelt, um eine Verbindung der Formel Ia, oder ein 7,8-Dihydroderivat davon, zu erhalten, oder

d) eine Verbindung der Formel Vb

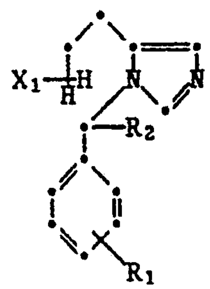

(Vb),

worin $R_1$ und $R_2$ wie oben unter Formel Ib definiert sind und $X_1$ eine Abgangsgruppe bedeutet, in Gegenwart einer Base cyclisiert, um eine Verbindung der Formel Ib zu erhalten, oder

e) eine Verbindung der Formel VIb

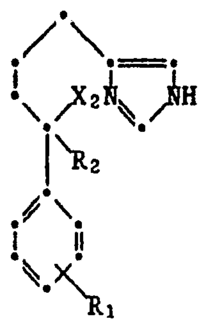

(VIb),

worin $R_1$ und $R_2$ wie oben unter Formel Ib definiert sind und $X_2$ eine Abgangsgruppe bedeutet, in Gegenwart einer Base cyclisiert, um eine Verbindung der Formel Ib zu erhalten, oder

f) in einer Verbindung der Formel VIIb

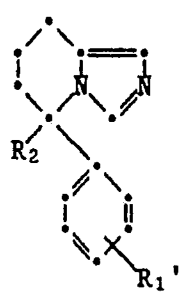

(VIIb),

worin $R_1'$ einen Rest bedeutet, der in Cyan umgewandelt werden kann und worin $R_2$ wie oben unter Formel Ib definiert ist, den Rest $R_1'$ in Cyan umwandelt, um eine Verbindung der Formel Ib zu erhalten, worin $R_1$ Cyan bedeutet; und/oder eine Verbindung der Formel Ia, oder ein 7,8-Dihydroderivat davon, mit Wasserstoff in Gegenwart eines Hydrierungskatalysators zum entsprechenden 5,6,7,8-Tetrahydroderivat der Formel Ib reduziert, und/oder eine erhaltene Verbindung in eine andere Verbindung der Erfindung umwandelt und/oder ein erhaltenes Salz in die freie Verbindung oder in ein anderes Salz umwandelt und/oder eine freie Verbindung, die eine salzbildende Gruppe enthält, in ein Salz umwandelt und/oder eine erhaltenes racemisches Gemisch in die individuellen Enantiomere auftrennt. Verbindungen der Formel Ia oder Ib können ferner z.B. dadurch hergestellt werden, dass man

in einer Variante des Verfahrens e) Verbindungen der Formel VIb verwendet, worin die freie NH-Gruppe durch eine NH-Schutzgruppe wie unten definiert geschützt ist, oder

g) eine Verbindung der Formel IXb

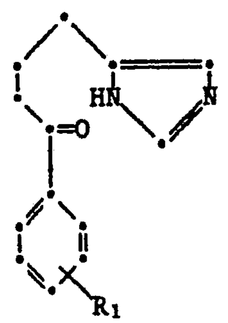

(IXb),

gegebenenfalls unter reduktiven Bedingungen, cyclisiert, um ein 7,8-Dihydroderivat einer Verbindung der Formel Ia oder, im Falle von reduktiven Bedingungen, eine Verbindung der Formel Ib zu erhalten, oder

h) eine Verbindung analog zu Formel Ia, oder ein 7,8-Dihydroderivat davon, oder eine Verbindung analog zu Formel Ib, die jeweils eine zusätzliche Carboxygruppe in 1- oder 3-Stellung enthält, decarboxyliert, um eine Verbindung der Formel Ia, ein 7,8-Dihydroderivat davon oder eine Verbindung der Formel Ib zu erhalten.

Verfahren a): Die Cyclisierung der Formylamino-Verbindung der Formel II oder IIa wird vorteilhaft unter solchen Bedingungen durchgeführt, wie sie für die Cyclisierung von 6-Methyl-2-methylaminopyridin zu 5-Methylimidazo[1,5-a]pyridin in J. Org. Chem. 40 , 1210 (1975) beschrieben sind. Die Cyclisierung unter sauren Bedingungen kann vorteilhaft mit einer Lewis-Säure, wie Polyphosphorsäure, phosphoroxychlorid oder Polyphosphatester, erreicht werden.

Verfahren b): Die Cyclisierung der Formylverbindung der Formel III oder IIIa wird z.B. unter basischen Bedingungen durchgeführt. Als Base kann jede Base, die schnell Protonen aufnimmt, Verwendung finden, z.B. ein Amin, etwa ein tertiäres Amin, z.B. ein Triniederalkylamin, wie Trimethylamin oder Triethylamin, ein cyclisches tertiäres Amin, z.B. N-Methylmorpholin, ein bicyclisches Amidin, etwa ein Diazabicycloalken, z.B. 1,5-Diazabicyclo[4.3.0]non-5-en oder 1,5-Diazabicyclo[5.4.0]undec-5-en (DBU), oder z.B. eine Base vom Pyridintyp, wie Pyridin. Geeignet sind ferner anorganische Basen, z.B. eine Alkalimetallhydroxid oder ein Erdalkalimetallhydroxid, z.B. Natrium-, Kalium- oder Calciumhydroxid. Bevorzugte Basen sind Alkoholate, z.B. ein Alkalimetallalkoholat, wie Natrium- oder Kaliummethylat, -ethylat oder -tert-butylat.

Die Cyclisierung gemäss dem Verfahren a) und b) wird im allgemeinen in inerten organischen Lösungsmitteln durchgeführt, wie geeigneten Alkoholen, z.B. Methanol, Ethanol oder Isopropanol, Ketonen, z.B. Aceton, Ethern, z.B. Dioxan oder Tetrahydrofuran, Nitrilen, z.B. Acetonitril, Kohlenwasserstoffen, z.B. Benzol oder Toluol, halogenierten Kohlenwasserstoffen, z.B. Methylenchlorid, Chloroform oder Tetrachlormethan, Estern, z.B. Essigsäureethylester, oder Amiden, z.B. Dimethylformamid oder Dimethylacetamid, und dergleichen. Die Reaktionstemperatur liegt zwischen Raumtemperatur und dem Siedepunkt des Reaktionsgemisches, vorzugsweise zwischen 60 °C und der Siedetemperatur des Reaktionsgemisches. Weiterhin wird die Cyclisierung vorzugsweise unter einer Inertgasatmosphäre, insbesondere einer Stickstoffatmosphäre, durchgeführt.

Verfahren c)/f): Ein Rest $R_1$' in einer Verbindung der Formel IV, IVa, VII oder VIIb, der in Cyan umgewandelt werden kann, ist z.B. Wasserstoff, verestertes Hydroxy, z.B. Halogen, wie Chlor, Brom oder Jod, oder eine Sulfonyloxygruppe, z.B. p-Toluolsulfonyloxy, Benzolsulfonyloxy oder Mesyloxy, Sulfo, Amino, Carboxy, Carboxy in Form eines funktionellen Derivates, z.B. Carbamoyl, Niederalkylcarbamoyl, wie tert-Butylcarbamoyl, oder Halogenformyl, z.B. Chlor- oder Bromformyl, Formyl, Formyl in Form eines funktionellen Derivates, z.B. Hydroxyiminomethyl, oder eine Halogenmagnesiumgruppe, z.B. Jod-, Brom- oder Chlormagnesium.

Verbindungen der Formel I, Ia, I* oder Ib, worin $R_1$ Cyan bedeutet, können z.B. dadurch erhalten werden, dass man die folgenden Umwandlungen ausführt:

Die Umwandlung einer Verbindung der Formel IV, IVa, VII oder VIIb, worin $R_1$' Wasserstoff ist, zu einer Verbindung der Formel I, Ia oder I* oder Ib wird z.B. gemäss der bekannten Methode von C. Friedel, F.M. Crafts und P. Karrer durch Umsetzung mit Chlorcyan (ClCN) oder Bromcyan durchgeführt oder gemäss dem Verfahren von J. Houben und W. Fisher durch Umsetzung mit z.B. Trichloracetonitril. Vorteilhaft wird der Standardkatalysator Aluminiumtrichlorid bei diesen Reaktionen verwendet, wobei Chlorwasserstoff oder Bromwasserstoff abgespalten wird, der durch Zugabe einer Base, vorzugsweise eines Amins, z.B. Triethylamin oder Pyridin, gebunden werden kann.

Die Umwandlung einer Verbindung der Formel IV, IVa, VII oder VIIb, worin $R_1$' Halogen, z.B. Chlor, Brom oder Jod, bedeutet, in eine Verbindung der Formel I, Ia, I* oder Ib wird z.B. durchgeführt, indem man erstere mit einem Cyanid, vorzugsweise Natrium- oder Kaliumcyanid oder insbesondere Kupfer(I)cyanid

umsetzt. Bevorzugte Lösungsmittel für diese Reaktion sind Pyridin, Chinolin, Dimethylformamid, l-Methyl-2-pyrrolidinon und Hexamethylphosphorsäuretriamid. Hohe Temperaturen, insbesondere die Rückflusstemperatur des Reaktionsgemisches, sind bevorzugt.

Die Umwandlung von Verbindungen der Formel IV, IVa, VII oder VIIb, worin $R_1'$ eine Sulfonyloxygruppe ist, z.B. p-Toluolsulfonyloxy, Benzolsulfonyloxy oder Mesyloxy, in eine Verbindung der Formel I, Ia, I* oder Ib wird z.B. durch Reaktion mit einem Alkalimetall cyanid, vorzugsweise Natrium- oder Kaliumcyanid, durchgeführt. Hohe Temperaturen, insbesondere die Rückflusstemperatur des Reaktionsgemisches, sind bevorzugt.

Die Umwandlung einer Verbindung der Formel IV, IVa, VII oder VIIb, worin $R_1'$ Amino bedeutet, in eine Verbindung der Formel I, Ia, I* oder Ib verläuft über mehrere Stufen. Zunächst wird ein Diazoniumsalz gebildet, etwa durch Reaktion der Aminoverbindung mit einem Alkalimetallnitrit, vorzugsweise Kaliumnitrit. Das Diazoniumsalz kann gemäss der bekannten Sandmeyer-Reaktion in situ z.B. mit Kupfer(I)cyanid oder einem Cyanidkomplex mit labilen Cyanogruppen, vorzugsweise Kaliumcuproammoniumcyanid, oder mit katalytischen Mengen von frisch gefälltem Kupferpulver in Gegenwart eines Alkalimetallcyanids, z.B. Natrium- oder Kaliumcyanid, umgesetzt werden. Einzelheiten dieser Reaktion sind z.B. in Houben-Weyl, Methoden der organischen Chemie, Thieme Stuttgart 1952, Vol. VIII, angegeben.

Eine Carboxygruppe $R_1'$ kann z.B. durch Reaktion mit Chlorsulfonylisocyanat gemäss dem Verfahren von R. Graf, Angew. Chem. 80 , 183 (1968) in Cyan umgewandelt werden. Bevorzugtes Lösungsmittel ist Dimethylformamid; bei dieser Reaktion entwickelt sich $CO_2$ und das Chlorsulfonsäure-Dimethylformamid-Additionssalz wird ausgefällt.

Die Umwandlung einer Verbindung der Formel IV, IVa, VII oder VIIb, worin $R_1'$ Carboxy in Form eines funktionellen Derivates, z.B. Carbamoyl, Niederalkylcarbamoyl, z.B. tert-Butylcarbamoyl, bedeutet, in eine Verbindung der Formel I, Ia, I* oder Ib wird z.B. durch ein starkes Dehydratisierungsmittel, wie Phosphorpentoxid, Phosphoroxychlorid, Thionylchlorid, Phosgen oder Oxalylchlorid, bewirkt.

Ein Halogenformyl-Rest (= Halogencarbonyl) $R_1'$, z.B. Chlor- oder Bromformyl, wird z.B. mit Ammoniak oder einem primären oder sekundären Amin, wie Methyl- oder Dimethylamin, umgesetzt. Das so erhaltene Amid wird zu einem Nitril der Formel I, Ia, I* oder Ib umgewandelt, gegebenenfalls in situ, indem man eines der oben erwähnten Dehydratisierungsmittel verwendet, z.B. Phosphorpentachlorid bei unsubstituierten Amiden oder Phosphoroxychlorid bei Mono- oder Diniederalkylamiden.

Die Dehydratisierung wird vorzugsweise in Gegenwart einer geeigneten Base durchgeführt. Eine geeignete Base ist z.B. ein Amin, etwa ein tertiäres Amin, wie ein Triniederalkylamin, z.B. Trimethylamin, Triethylamin oder Ethyldiisopropylamin, oder ein N-N-Diniederalkylanilin, z.B. N,N-Dimethylanilin, oder ein cyclisches tertiäres Amin, z.B. N-Niederalkyl-morpholin, z.B. N-Methylmorpholin, oder z.B. eine Base vom Pyridintyp, wie Pyridin oder Chinolin.

Die Umwandlung einer Formylgruppe in Cyan wird z.B. durch Umwandlung der Formylgruppe in ein reaktives funktionelles Derivat, z.B. Hydroxyiminomethyl, und Dehydratisierung dieser Gruppe zu Cyan durchgeführt. Geeignete Dehydratisierungsmittel sind die oben genannten anorganischen Dehydratisierungsmittel, z.B. Phosphorpentachlorid, oder vorzugsweise Anhydride von organischen Säuren, z.B. die Anhydride von Niederalkancarbonsäuren, z.B. Essigsäureanhydrid.

Die Umwandlung von Formyl in Hydroxyiminomethyl wird z.B. durch Reaktion mit einem Säureadditionssalz von Hydroxylamin durchgeführt, vorzugsweise mit dem Hydrochlorid.

Eine Verbindung der Formel IV, IVa, VII oder VIIb, worin $R_1'$ Formyl bedeutet, kann direkt zu einer Verbindung der Formel I, Ia, I* oder Ib umgewandelt werden, indem man sie z.B. mit O,N-Bis-(trifluoracetyl)-hydroxylamin in Gegenwart einer Base, z.B. Pyridin, gemäss dem Verfahren von D.T. Mowry, Chem. Rev. 42 , 251 (1948) umsetzt.

Die Umwandlung einer Verbindung der Formel IV, IVa, VII oder VIIb, worin $R_1'$ Halogenmagnesium bedeutet, z.B. Jod-, Brom- oder Chlormagnesium, in eine Verbindung der Formel I, Ia, I* oder Ib wird z.B. dadurch erreicht, dass man das Magnesiumhalogenid mit Halogencyan oder Dicyan umsetzt. Während dieser Reaktion bildet sich Magnesiumhalogenid, etwa Magnesiumchlorid bzw. Magnesiumcyanohalogenid, etwa Magnesiumcyanochlorid. Die "Grignard"-Verbindung, worin $R_1'$ Halogenmagnesium bedeutet, wird in bekannter Weise hergestellt, z.B. durch Reaktion einer Verbindung der Formel IV, IVa, VII oder VIIb, worin $R_1'$ Halogen, z.B. Chlor, Brom oder Jod, ist, mit Magnesium, z.B. in trockenem Ether.

Wenn nicht anders angegeben, wird die Umwandlung einer Verbindung der Formel IV, IVa, VII oder VIIb in eine Verbindung der Formel I, Ia, I* oder Ib vorzugsweise in einem inerten, insbesondere wasserfreien, Lösungsmittel oder Lösungsmittelgemisch durchgeführt, z.B. einem Carbonsäureamid, etwa einem Formamid, wie Dimethylformamid, einem halogenierten Kohlenwasserstoff, z.B. Methylenchlorid, Tetrachlormethan oder Chlorbenzol, einem Keton, z.B. Aceton, einem cyclischen Ether, z.B. Tetrahydrofuran, einem Ester, z.B. Essigsäureethylester, oder einem Nitril, z.B. Acetonitril, oder in Mischungen davon, gegebenenfalls in

Gegenwart eines Alkohols, z.B. Methanol oder Ethanol, oder Wasser, gegebenenfalls bei erniedrigter oder erhöhter Temperatur, z.B. in einem Temperaturbereich von ungefähr -40° C bis +100° C, vorzugsweise zwischen Raumtemperatur und dem Siedepunkt des Reaktionsgemisches und gegebenenfalls unter einer Inertgasatmosphäre, z.B. einer Stickstoffatmosphäre.

Verfahren d): In einem Ausgangsmaterial der Formel V oder Vb bedeutet eine Abgangsgruppe $X_1$ vorzugsweise verestertes Hydroxy, z.B. Niederalkanoyloxy, wie Acetoxy, Mesyloxy, Benzolsulfonyloxy oder Toluolsulfonyloxy, oder insbesondere Halogen, z.B. Chlor oder Brom.

Eine geeignete Base ist z.B. ein Alkalimetall- oder Erdalkalimetallhydroxid, z.B. Natrium-, Kalium- oder Calciumhydroxid, ein bicyclisches Amidin, z.B. 1,5-Diazabicyclo[5.4.O]undec-5-en, vorzugsweise ein Alkoholat, z.B. Natrium- oder Kaliummethylat, -ethylat oder -tert-butylat, ein Alkalimetallamid, z.B. Lithiumdiisopropylamid, oder ein Alkalimetallhydrid, z.B. Natriumhydrid. Falls $R_2$ freies oder funktionell abgewandeltes Carboxy oder Acyl bedeutet, wird die Reaktion bedeutend erleichtert und schwächere Basen, z.B. tertiäre Amine, wie Triniederalkylamine, z.B. Triethylamin, können verwendet werden.

Die Cyclisierung wird in einem aprotischen organischen Lösungsmittel, z.B. einem Ether, wie Diethylether, Dioxan oder Tetrahydrofuran, oder einem Keton, z.B. Aceton, einem Amid, z.B. Dimethylformamid oder Hexamethylphosphorsäuretriamid, oder in Mischungen dieser Lösungsmittel durchgeführt, gegebenenfalls auch in einer Mischung der erwähnten Lösungsmittel mit einem Alkan, z.B. n-Hexan oder Petrolether. Die Reaktionstemperatur liegt zwischen ungefähr -50° und +50° C, vorzugsweise zwischen -10° und Raumtemperatur. Vorzugsweise wird die Reaktion unter einer Inertgasatmosphäre, z.B. Argon oder Stickstoff, durchgeführt.

Verfahren e): In einem Ausgangsmaterial der Formel VI oder VIb ist die Abgangsgruppe $X_2$ vorzugsweise definiert wie eine Abgangsgruppe $X_1$ im Verfahren d). Die Cyclisierung wird vorzugsweise unter Verwendung einer Base durchgeführt, etwa einem tertiären Amin wie oben definiert, z.B. Triethylamin, oder sogar ohne Verwendung einer Base. NH-Schutzgruppen (oder Blockierungsgruppen) $R_0$ sind vorzugsweise Triniederalkylsilyl, z.B. Trimethylsilyl, Niederalkanoyl, z.B. Acetyl, Dialkylcarbamoyl, z.B. Dimethylcarbamoyl, oder Triphenylmethyl.

Verfahren g): Die nichtreduktive Reaktion wird vorzugsweise in Gegenwart eines sauren Katalysators, z.B. p-Toluolsulfonsäure, durchgeführt. Die reduktive Aminierungsreaktion wird z.B. mit Wasserstoff in Gegenwart eines gewöhnlichen Hydrierungskatalysators durchgeführt, z.B. Raney-Nickel, Platin oder Palladium-auf-Kohle, oder mit einem Wasserstoff-liefernden Mittel, z.B. Natriumcyanoborhydrid.

Verfahren h): Die Decarboxylierung kann mit üblichen Decarboxylierungsmitteln durchgeführt werden, z.B. mit Säuren, wie Salzsäure, vorzugsweise bei erhöhter Temperatur.

Nachoperationen: Einer Verbindung der Formel I oder Ia, oder ein 7,8-Dihydroderivat davon, kann zu den weiter hydrierten Derivaten der Formel I*, z.B. den entsprechenden 5,6,7,8-Tetrahydroderivaten der Formel Ib, durch Reduktion, z.B. mit Wasserstoff in Gegenwart eines Hydrierungskatalysators, wie Platin oder Palladium, unter sauren Bedingungen, z.B. in Mineralsäure, wie Salzsäure, oder mit Palladium-auf-Kohle bei atmosphärischem Druck in einem inerten Lösungsmittel, z.B. Ethanol oder Essigsäureethylester, umgewandelt werden.

Weiterhin können Verbindungen der Formel I* oder Ib, worin $R_2$ Carboxy bedeutet, decarboxyliert werden, um andere Verbindungen der Formel I* oder Ib zu erhalten, worin $R_2$ Wasserstoff ist, indem man gewöhnliche Decarboxylierungsmethoden anwendet, z.B. die oben für Verfahren h) beschriebenen. In den erwähnten Verbindungen der Formel I* ist der Carboxy-Substituent $R_2$ vorzugsweise an dasselbe Kohlenstoffatom gebunden wie der Substituent $C_6H_4$-$R_1$.

Eine Verbindung der Formel I*, worin die 5-Stellung durch den Substituenten $C_6H_4$-$R_1$ monosubstituiert ist, z.B. eine Verbindung der Formel Ib oder Ic, worin $R_2$ Wasserstoff bedeutet, kann an diesem Kohlenstoffatom zusätzlich durch eine Gruppe $R_2$ substituiert werden, indem man sie unter basischen Bedingungen mit einem reaktiven Derivat von $R_2$, z.B. einem Niederalkylhalogenid, einem Aryl-niederalkylhalogenid oder einem Niederalkyldisulfid umsetzt. Geeignete Basen sind z.B. Alkalimetallalkoxide, z.B. Kalium-tert-butoxid, Alkalimetallamide, z.B. Lithiumdiisopropylamid, oder Alkalimetallhydride, z.B. Natriumhydrid.

## Herstellung der Ausgangsverbindungen

Verbindungen der Formel II und IIa sind bekannt oder können, falls neu, in an sich bekannter Weise hergestellt werden, z.B. indem man eine Verbindung der Formel VIII oder VIIIa

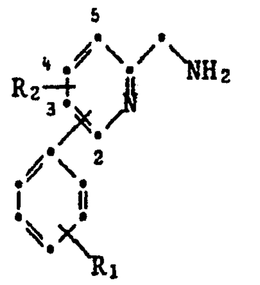 (VIII), 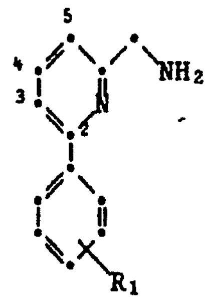 (VIIIa)

worin $R_1$ und $R_2$ wie oben unter Formel I oder Ia definiert sind, mit Ameisensäure oder einem reaktiven, funktionellen Derivat davon, z.B. Ameisensäureessigsäureanhydrid, umsetzt.

Verbindungen der Formel III und IIIa werden z.B. dadurch hergestellt, dass man eine Verbindung der Formel V, worin $X_1$ Hydroxy ist, n für 2 steht, $R_2'$ wie unter Formel V definiert ist und vorzugsweise Wasserstoff bedeutet, und $R_2''$ für Wasserstoff steht, oder eine Verbindung der Formel Vb, worin $X_1$ Hydroxy bedeutet und $R_2$ für Wasserstoff steht, z.B. mit Dimethylsulfoxid in Gegenwart von Dehydratisierungsmitteln, z.B. Säureanhydriden, wie Anhydriden von organischen Carbonsäuren, z.B. aliphatischen oder aromatischen Carbonsäuren oder Dicarbonsäuren, etwa Anhydriden von Niederalkancarbonsäuren, insbesondere Essigsäureanhydrid, gemischten Anhydriden von Niederalkancarbonsäuren oder -dicarbonsäuren mit Mineralsäuren, z.B. Acetyl- oder Oxalylchlorid, ferner Anhydriden von anorganischen Säuren, insbesondere von der Phosphorsäure, z.B. Phosphorpentoxid, umsetzt. Die obengenannten Anhydride, vor allem die von organischen Carbonsäuren, z.B. Oxalylchlorid, werden vorzugsweise in einem Verhältnis von ungefähr 1:1 mit Dimethylsulfoxid angewendet. Weitere Dehydratisierungsmittel oder wasserabsorbierende Mittel sind z.B. Carbodiimide, vor allem Dicyclohexylcarbodiimid, ferner Diisopropylcarbodiimid, oder Ketenimide, z.B. Diphenyl-N-p-tolylketenimin. Diese Reagentien werden vorzugsweise in Gegenwart von sauren Katalysatoren, wie Phosphorsäure, Pyridiniumtrifluoracetat oder Pyridiniumphosphat, angewendet. Schwefeltrioxid kann ebenfalls als Dehydratisierungsmittel oder wasserabsorbierendes Mittel verwendet werden; es wird gewöhnlich in Form eines Komplexes angewendet, z.B. mit Pyridin. Anschliessend wird eine Base hinzugegeben, vorzugsweise eine Base, die oben unter Verfahren c) erwähnt ist, z.B. Triethylamin.

Die Verbindungen der Formel IV und IVa werden vorzugsweise analog dem oben erwähnten Verfahren a) hergestellt.

Verbindungen der Formel V und Vb sind bekannt oder können, falls neu, in an sich bekannter Weise hergestellt werden, indem man z.B. eine andere Verbindung der Formel V oder Vb, worin $X_1$ Hydroxy ist und worin $R_2'$ und $R_2''$ bzw. $R_2$ vorzugsweise Wasserstoff bedeuten, mit einem Halogenierungsmittel umsetzt oder die Hydroxygruppe mit einem reaktiven funktionellen Derivat einer Sulfon- oder Carbonsäure verestert. Die Reaktion mit einem Halogenierungsmittel, z.B. Thionylchlorid oder Phosphorpentachlorid, wird z.B. analog zu dem im US-Patent 4,089,955 beschriebenen Halogenierungsverfahren durchgeführt. Die Reaktion mit einem reaktiven funktionellen Derivat einer Sulfon- oder Carbonsäure, z.B. einem gemischten Anhydrid mit einer Mineralsäure, z.B. Mesylchlorid, Benzolsulfonylchlorid oder p-Toluolsulfonylchlorid, oder Acetylchlorid, wird gemäss bekannten Veresterungsmethoden durchgeführt.

Verbindungen der Formel VI und VIb sind bekannt oder werden, falls neu, in an sich bekannter Weise hergestellt, z.B. indem man eine andere Verbindung der Formel VI oder VIb, worin $X_2$ Hydroxy ist, $R_1$ und $R_2$ wie oben unter Formel I* oder Ib definiert sind, und die Imidazol-NH-Gruppe gegebenenfalls durch eine konventionelle Aminoschutzgruppe, z.B. Triniederalkylsilyl, wie Trimethylsilyl, geschützt ist, mit einem Halogenierungsmittel umsetzt oder die Hydroxygruppe mit einem reaktiven funktionellen Derivat einer Sulfon- oder Carbonsäure verestert. $R_2$ ist vorzugsweise Niederalkyl und im besonderen Wasserstoff. Die Halogenierungsreaktion wird z.B. analog dem im US-Patent 4,089,955 beschriebenen Vefahren durchgeführt. Die Reaktion mit einem reaktiven funktionellen Derivat einer Sulfon- oder Carbonsäure, z.B. einem gemischten Anhydrid mit einer

Mineralsäure, z.B. Mesylchlorid, Benzolsulfonylchlorid oder p-Toluolsulfonylchlorid, oder Acetylchlorid, wird nach bekannten Veresterungsverfahren durchgeführt.

Die Verbindungen der Formel VII oder VIIb werden vorzugsweise analog den Verfahren d) und e), ferner auch g) und h), hergestellt.

Verbindungen der Formel VIII oder VIIIa sind bekannt oder werden, falls neu, in an sich bekannter Weise hergestellt, z.B. durch Hydrierung einer Verbindung der Formel XV oder XVa

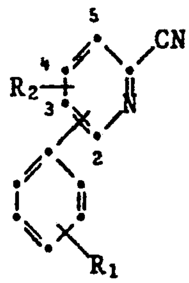 (XV) 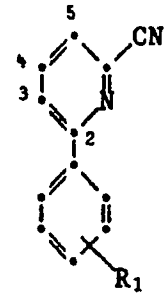 (XVa),

worin $R_1$ und $R_2$ wie oben unter Formel I oder Ia definiert sind.

Die Hydrierung wird vorzugsweise in Gegenwart eines Katalysators durchgeführt, z.B. Platin oder Palladium-auf-Kohle, ferner in Gegenwart einer Mineralsäure, z.B. Salzsäure.

Verbindungen der Formel V oder Vb, worin $X_1$ Hydroxy ist, sind bekannt oder werden, falls neu, in an sich bekannter Weise hergestellt, z.B. indem man eine Verbindung der Formel XVII oder XVIIa

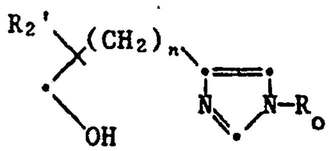 (XVII) 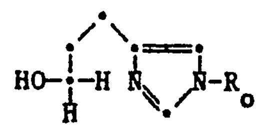 (XVIIa),

worin n und $R_2'$ wie n und $R_2$ unter Formel I* definiert sind, $R_0$ eine NH-Blockierungsgruppe wie oben definiert bedeutet, z.B. Dialkylcarbamoyl, wie Dimethylcarbamoyl, und die Hydroxygruppe durch eine konventionelle Hydroxy-Schutzgruppe geschützt ist, z.B. Trimethylsilyl, mit einer Verbindung der Formel XVIII oder XVIIIa

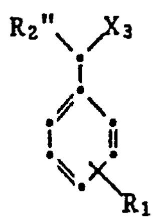 (XVIII) 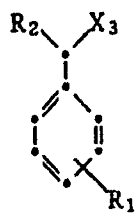 (XVIIIa),

worin $R_1$ wie oben unter Formel I oder Ia definiert ist, $R_2$ wie unter Formel Ib definiert ist und vorzugsweise Wasserstoff bedeutet, $R_2''$ wie $R_2$ unter Formel I* definiert ist und $X_3$ eine Abgangsgruppe bedeutet, z.B. verestertes Hydroxy, wie Halogen, z.B. Chlor oder Brom, oder Sulfonyloxy, z.B. Mesyloxy oder p-Toluolsulfonyloxy, umsetzt.

Verbindungen der Formel VI und VIb, worin $X_2$ Hydroxy ist, $R_2$ vorzugsweise Niederalkyl und insbesondere Wasserstoff ist und der Rest $C_6H_4$-$R_1$ an dasselbe Kohlenstoffatom wie die Gruppe $X_2$ gebunden ist, sind bekannt oder können, falls neu, in an sich bekannter Weise hergestellt werden, z.B. indem man eine Verbindung der Formel XIX oder XIXa

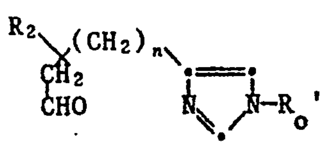 (XIX), 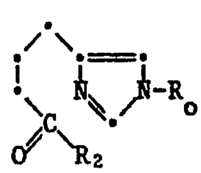 (XIXa),

worin n und $R_2$ wie oben unter Formel I* oder Ib definiert sind, und $R_2$ vorzugsweise Niederalkyl und insbesondere Wasserstoff bedeutet, wobei in der Formel XIX die Gruppe $R_2$ wie angedeutet an jedem Kohlenstoffatom einschliesslich dem Carbonylkohlenstoff angeknüpft sein kann, und worin $R_0'$ vorzugsweise eine konventionelle NH-Schutz gruppe wie oben definiert bedeutet, z.B. Triniederalkylsilyl, etwa Trimethylsilyl, in einer Reaktion mit einer metallorganischen Verbindung der Formel XX

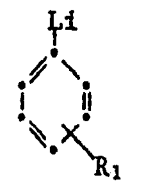 (XX),

worin $R_1$ wie oben unter Formel I oder Ia definiert ist, umsetzt.

Verbindungen der Formel XV und XVa können z.B. dadurch hergestellt werden, dass man eine Verbindung der Formel XXI or XXIa

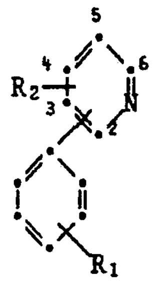 (XXI) 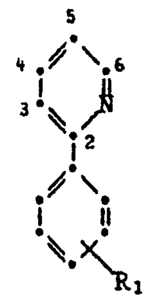 (XXIa)

zunächst mit einem Oxidationsmittel, z.B. Peressigsäure, zum entsprechenden N-Oxid umsetzt, dann das N-Oxid mit einem Methylierungsmittel, z.B. Dimethylsulfat, behandelt, die 6-Stellung mit einem Cyanid-Ion, z.B. unter Verwendung von Kaliumcyanid, substituiert und das N-Oxid zurück zum Pyridinderivat überführt.

Verbindungen der Formeln XVII-XXI, XVIIa-XIXa und XXIa sind bekannt oder können durch Anwendung konventioneller chemischer Verfahren hergestellt werden.

Verbindungen der Formel IX und IXb können z.B. durch die folgende Synthesesequenz hergestellt werden: Zunächst wird eine Verbindung der Formel XIX - oder XIXa, worin $R_2$ Wasserstoff bedeutet - mit gewöhnlichen Oxidationsmitteln, z.B. $KMnO_4$, oxidiert, wobei die entsprechende Säure entsteht, die gegebenenfalls weiter in den entsprechenden Niederalkylester überführt werden kann. Die Umsetzung des letzteren - oder der freien Säure - mit einer Verbindung der Formel XX, oder einem geeigneten organometallischen Aequivalent davon, und die Abspaltung der NH-Schutzgruppe führt zu Verbindungen der Formel IXb und IX, wobei in letzteren der Substituent $C_6H_4$-$R_1$ an den Carbonylkohlenstoff gebunden ist.

Verbindungen der Formel VI und IX, worin der Substituent $C_6H_4$-$R_1$ nicht an dasselbe Kohlenstoffatom wie die Gruppe $X_2$ bzw. an den Carbonylkohlenstoff gebunden ist, können z.B. aus Verbindungen analog zur Formel XVII erhalten werden, die zusätzlich einen Rest $C_6H_4$-$R_1$ in der Seitenkette enthalten. Dies kann nach bekannten Verfahren geschehen, z.B. durch Veresterung der Hydroxygruppe bzw. ihrer Oxidation zu Formyl. Die Ausgangsverbindungen analog zu Formel XVII können durch Anwendung bekannter chemischer Methoden hergestellt werden.

Die Ausgangsverbindungen für Verfahren h), die eine Carboxygruppe in 3-oder 1-Stellung des bicyclischen Ringsystems enthalten, können z.B. dadurch erhalten werden, dass man eine Verbindung der Formel VIII oder VIIIa bzw. eine Verbindung analog zu diesen, die eine zusätzliche Carboxygruppe in $\alpha$-Stellung enthält, mit z.B. Oxalsäureniederalkylesterhalogenid, z.B. Oxalsäureethylesterchlorid, bzw. mit Ameisensäure oder einem Derivat davon, z.B. Ameisensäureessigsäureanhydrid, umsetzt und anschliessend z.B. mit einer Lewissäure, etw Phosphoroxychlorid, den Ring schliesst.

Falls irgendwelche der genannten Zwischenprodukte störende reaktionsfähige Gruppen, z.B. Carboxy-, Hydroxy-, Amino-, Sulfo- oder Mercaptogruppen, enthalten, können solche vorzugsweise vorübergehend, auf jeder Stufe, durch leicht abspaltbare Schutzgruppen geschützt werden. Die Wahl der Schutzgruppe für eine bestimmte Reaktion hängt von verschiedenen Faktoren ab, z.B. von der Art der zu schützenden funktionellen Gruppe, der Struktur und Stabilität des Moleküls, an dem die funktionelle Gruppe sitzt, und den Reaktionsbedingungen. Schutzgruppen, die diese Bedingungen erfüllen, ihre Einführung und Entfernung sind an sich bekannt und beschrieben, z.B. in J.F.W. McOmie, "Protective Groups in Organic Chemistry", Plenum Press, London, New York 1973. So können Carboxy- und auch Sulfogruppen z.B. als Ester geschützt werden, z.B. als unsubstituierte oder substituierte Niederalkylester, wie Methyl- oder Benzylester, wobei es möglich ist, solche Estergruppen unter milden Bedingungen, insbesondere alkalischen Bedingungen, wieder leicht abzuspalten. Amino- und Hydroxy-Schutzgruppen, die unter schonenden

Bedingungen wieder abgespalten werden können, sind z.B. Acylradikale, etwa gegebenenfalls durch Halogen substituiertes Niederalkanoyl, z.B. Formyl oder Trichloracetyl, oder organische Silylgruppen, z.B. Triniederalkylsilyl, etwa Trimethylsilyl.

Salze der erfindungsgemässen Verbindungen können in an sich bekannter Weise hergestellt werden. So können sie z.B. in Uebereinstimmung mit den in den Beispielen beschriebenen Verfahren hergestellt werden. Säureadditionssalze werden in üblicher Weise erhalten, z.B. indem man die freie Verbindung mit einer Säure oder einem geeigneten Anionenaustauschreagens behandelt. Salze können in üblicher Weise in die freien Verbindungen umgewandelt werden, z.B. indem man ein Säureadditionssalz mit einer geeigneten Base, z.B. einem Alkoholat, wie Kalium-tert-butoxid, behandelt. Andererseits können erfindungsgemässe Verbindungen, die saure Gruppen enthalten, z.B. Carboxy, in an sich bekannter Weise in Salze überführt werden, indem man sie mit einer Base behandelt, z.B. mit einem Alkalimetallhydroxid oder -alkoxid, einem Alkalimetall- oder Erdalkalimetallsalz, z.B. Natriumhydrogencarbonat, Ammoniak oder einem geeigneten organischen Amin. Freie Verbindungen können erhalten werden, indem man solche Salze mit einer Säure behandelt. In Folge der engen Beziehung zwischen den Verbindungen in freier Form und in Form ihrer Salze sind im vorausgegangenen und nachfolgend unter den freien Verbindungen oder ihren Salzen sinn- und zweckmässig gegebenenfalls auch die entsprechenden Salze bzw. freien Verbindungen zu verstehen.

Abhängig von der Wahl der Ausgangsstoffe und dem verwendeten Verfahren können die neuen Verbindungen in Form eines der möglichen Isomeren oder als Isomerengemische vorliegen. So können sie z.B. abhängig von der Gegenwart und Anzahl von chiralen Kohlenstoffatomen als optische Isomere, d.h. Antipoden, oder als Gemische von optischen Isomeren, z.B. Racemate, oder als Diastereomerengemische vorliegen.

Erhaltene Diasteromerengemische können auf Grund der physikochemischen Unterschiede ihrer Komponenten in an sich bekannter Weise aufgetrennt werden, z.B. durch Chromatographie und/oder fraktionierte Kristallisation.

Erhaltene Racemate können weiter in die optischen Antipoden in an sich bekannter Weise aufgetrennt werden, z.B. durch Chromatographie unter Verwendung einer optisch aktiven stationären Base, durch Umkristallisation aus einem optisch aktiven Lösungsmittel, mit Hilfe von Mikroorganismen oder z.B. durch Umsetzung eines sauren Zwischenproduktes oder Endstoffes mit einer optisch aktiven Base, die ein Salz mit der racemischen Säure bildet, und Auftrennung der so erhaltenen Salze, z.B. auf Grund ihrer unterschiedlichen Löslichkeit, in die Diastereomeren, aus denen die Antipoden durch Einwirkung geeigneter Agentien freigesetzt werden können. Basische racemische Endstoffe können in gleicher Weise in die Antipoden aufgetrennt werden, z.B. durch Trennung von diastereomeren Salzen davon, z.B. durch fraktionierte Kristallisation der d- oder l-Tartrate.

Die oben genannten Reaktionen werden nach an sich bekannten Methoden, in Gegenwart oder Abwesenheit von Verdünnungsmitteln, vorzugsweise in solchen, die gegenüber den Reagentien inert sind und diese lösen, Katalysatoren, Kondensations- oder anderen obengenannten Mitteln, und/oder einer inerten Atmosphäre, unter Kühlung, bei Raumtemperatur oder erhöhter Temperatur, z.B. in einem Temperaturbereich von $-20^\circ$ bis $+200^\circ C$, vorzugsweise beim Siedepunkt des verwendeten Lösungsmittels, bei normalem oder erhöhtem Druck, durchgeführt. Die bevorzugten Lösungsmittel, Katalysatoren und Reaktionsbedingungen sind in den beigefügten Beispielen offenbart.

Die Verbindungen, einschliesslich ihrer Salze, können auch in Form ihrer Hydrate erhalten werden, oder ihre Kristalle können z.B. das zur Kristallisation verwendete Lösungsmittel einschliessen.

Die Erfindung betrifft ebenfalls Abänderungen des vorliegenden Verfahrens, wonach ein auf irgendeiner Stufe des Verfahrens erhaltenes Zwischenprodukt als Ausgangsmaterial verwendet wird und die verbleibenden Verfahrensschritte durchgeführt werden oder das Verfahren auf irgendeiner Stufe abgebrochen wird, oder wonach ein Ausgangsmaterial unter den Reaktionsbedingungen gebildet, oder worin einer oder mehrere der Ausgangsstoffe in Form eines Salzes oder eines optisch reinen Antipoden verwendet wird. Im Verfahren der vorliegenden Erfindung werden bevorzugt solche Ausgangsstoffe verwendet, die zu den im vorstehenden als besonders wertvoll beschriebenen Verbindungen führen. Die Erfindung betrifft auch neue Ausgangsstoffe und Verfahren zu ihrer Herstellung.

Die Erfindung betrifft weiterhin pharmazeutische Präparate zur enteralen oder parenteralen Verabreichung. Diese Präparate enthalten eine therapeutisch wirksame Menge einer erfindungsgemässen Verbindung, allein oder in Kombination mit einem oder mehreren pharmazeutisch annehmbaren Trägermaterialien. Feste oder flüssige anorganische oder organische Substanzen finden Verwendung als Trägermaterialien. Geeignete Dosiseinheitsformen, insbesondere zur peroralen Verabreichung, z.B. Dragees, Tabletten oder Kapseln, enthalten vorzugsweise ungefähr 5-100 mg, insbesondere ungefähr 10 bis 50 mg einer erfindungsgemässen Verbindung oder eines pharmazeutisch verwendbaren Salzes davon zusammen mit pharmazeutisch verwendbaren Trägerstoffen.

Die täglichen Dosen der erfindungsgemässen Verbindungen liegen bei Säugetieren, abhängig von der Art, und auch bei Personen, abhängig vom Alter, individuellen Zustand und der Art der Verabreichung, zwischen ungefähr 0,1 bis 100 mg/kg, vorzugsweise zwischen ungefähr 0,5 bis 50 mg/kg, des Körpergewichts. Bei parenteraler Verabreichung, z.B. intramuskulären oder subkutanen Injektionen oder intravenösen Infusionen, liegen die Dosen innerhalb des angegebenen Bereiches im allgemeinen niedriger als bei enteraler, z.B. oraler oder rektaler, Verabreichung. Die erfindungsgemässen Verbindungen werden oral oder rektal vorzugsweise in Dosiseinheitsformen wie Tabletten, Dragées, Kapseln oder Suppositorien verabreicht, parenteral insbesondere in Form von injizierbaren Lösungen, Emulsionen oder Suspensionen, oder Infusionslösungen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol. Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidinon, Polyethylenglykol und/oder Titandioxid enthalten, oder Lacklösungen in geeigneten organischen Lösungsmitteln oder Lösungsmittelgemischen, oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Lactose, Bindemitteln, wie Stärken und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können.

Als rektal anwendbare phrmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffes mit einer Grundmasse enthalten; als Grundmassen kommen z.B. flüssige Triglyceride, Polyethylenglykole oder Paraffinkohlenwasserstoffe in Frage.

Zur parenteralen Verabreichung eignen sich in erster Linie Suspensionen des Wirkstoffes, wie entsprechende ölige Injektionssuspensionen oder -lösungen, wobei man geeignete lipophile Lösungsmittel oder Vehikel, wie fette Oele, z.B. Sesamöl, oder synthetische Fettsäureester, z.B. Ethyloleat, oder Triglyceride verwendet, oder wässrige Injektionssuspensionen oder -lösungen, welche viskositätserhöhende Stoffe, z.B. Natriumcarboxymethylcellulose, Sorbit und/oder Dextran, und gegebenenfalls Stabilisatoren enthalten.

Die pharmazeutischen Präparate der vorliegenden Erfindung könnnen in an sich bekannter Weise, z.B. mittels konventioneller Misch-, Granulier-, Dragier-, Konfektionier-, Lösungs- oder Lyophilisierungsverfahren hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Die folgenden Beispiele dienen zur Illustration der Erfindung und sind nicht als Einschränkung ihres Umfangs aufzufassen. Temperaturen werden in Celsiusgraden angegeben, und Angaben über Teile betreffen Gewichtsteile. Wenn nicht anders definiert, wird das Eindampfen von Lösungsmitteln unter vermindertem Druck, vorzugsweise zwischen ungefähr 20 und 130 mbar, durchgeführt.

Beispiel 1: 5-(p-Cyanphenyl)-5,6,7,8-tetrahydroimidazo[1,5-a)-pyridin-Hydrochlorid. Zu einer auf 0° gekühlten Lösung von 8,1 g 5-(3-Chlorpropyl)-1-(p-cyanphenylmethyl)-1H-imidazol in 50 ml Tetrahydrofuran werden 7,0 g Kalium-tert-butoxyd als Feststoff in mehreren Portionen gegeben. Das Gemisch wird 2 h bei Raumtemperatur gerührt, mit 10%iger Essigsäure neutralisiert und zwischen Methylenchlorid und Wasser verteilt. Die organische Phase wird mit Wasser gewaschen, über Magnesiumsulfat getrocknet und eingedampft, wobei man ein Oel erhält, das in wenig Aceton gelöst und mit etherischem Chlorwasserstoff neutralisiert wird. Beim Abkühlen erhält man die Titelverbindung als weissen Feststoff; Smp. 201-203°.

Herstellung der Ausgangsverbindungen:

a) 1-Dimethylcarbamoyl-4-(3-trimethylsilyloxypropyl)-1H-imidazol. Zu einer Suspension von 51,8 g 4-(3-Hydroxy-n-propyl)-1H-imidazol, erhältlich gemäss II Farmaco, Ed. Sc. 29 , 309 (1973), in 500 ml Acetonitril werden 50,0 g Triethylamin gegeben. Dann werden zu diesem Gemisch 48,6 g Dimethylcarbamoylchlorid tropfenweise hinzu gefügt. Nach erfolgter Zugabe wird das Gemisch 21 h unter Rückfluss gekocht. Man kühlt ab auf 0°, wobei Triethylamin-Hydrochlorid ausfällt. Zu diesem Gemisch gibt man 50,0 g Triethylamin und dann 54,0 g Chlortrimethylsilan. Nach erfolgter Zugabe wird eine Stunde weitergerührt. Dann wird das Gemisch mit einem gleichgrossen Volumen an Ether verdünnt und filtriert. Das Filtrat wird zu einem Oel eingedampft, das mit Ether zerrieben und dann wieder filtriert wird, um weiteres Triethylamin-Hydrochlorid zu entfernen. Das Filtrat wird eingedampft und man erhält die Titelverbindung a) als Oel.

b) 1-(p-Cyanphenylmethyl)-5-(3-hydroxypropyl)-1H-imidazol . Eine Lösung von 97,0 g 1-Dimethylcarbamoyl-4-(3-trimethylsilyloxypropyl)-1H-imidazol und 72,0 g 1-Brommethyl-4-cyanbenzol in 500 ml Acetonitril wird 10 h unter Rückfluss gekocht. Man kühlt die Lösung in einem Eisbad auf 0° und leitet für wenige Minuten Ammoniakgas hindurch. Dann wird die Mischung im Vakuum verdampft, und man erhält eine halbfeste Masse, die in 500 ml 1N Salzsäure gelöst wird. Man lässt die Lösung bei Raumtemperatur 15 min stehen und extrahiert sie dann mit Ether. Der pH-Wert der wässrigen Phase wird mit 50%iger Natronlauge auf 9 eingestellt und das Gemisch anschliessend Methylenchlorid extrahiert. Die Methylenchloridextrakte werden mit Wasser gewaschen, über Natriumsulfat getrocknet und eingedampft, wobei man eine halbfeste Masse erhält, die mit kaltem Aceton verrieben wird. Auf diese Weise erhält man die Titelverbindung b) als weissen Feststoff, Smp. 121-123°.

c) 5-(3-Chlorpropyl)-1-(p-cyanphenylmethyl)-1H-imidazol . Zu einer Lösung von 5,2 g Thionylchlorid in 80 ml Methylenchlorid werden 8,4 g 1-(p-Cyanphenylmethyl)-5-(3-hydroxypropyl)-1H-imidazol als Feststoff in mehreren Portionen gegeben. Die Geschwindigkeit der Zugabe wird so reguliert, dass die auftretende Gasentwicklung unter Kontrolle bleibt. Nach vollständiger Zugabe wird die Lösung 1,5 h unter Rückfluss gekocht, mit Eis gekühlt und filtriert, wobei man das Hydrochlorid der Titelverbindung c) als ledergelben Feststoff vom Smp. 190-191° erhält. Das Salz wird zwischen Methylenchlorid und einer gesättigten Natriumhydrogencarbonat-Lösung verteilt. Die organischen Extrakte werden mit Wasser gewaschen, über Natriumsulfat getrocknet und eingedampft, wobei man die freie Base als Oel erhält.

Beispiel 2: 5-(p-Cyanphenyl)-5,6,7,8-tetrahydroimidazo[1,5-a]-pyridin-Hydrochlorid. Eine Lösung von 2,0 g 4-[4-Chlor-4-(p-cyanphenyl)-n-butyl]-1H-imidazol in 50 ml Chloroform wird 4 h unter Stickstoff unter Rückfluss gekocht, abgekühlt und eingedampft, wobei man die Titelverbindung erhält.

Herstellung der Ausgangsverbindungen:

a) 4-(3-Formyl-n-propyl)-1-trimethylsilylimidazol. Eine Lösung von 1,82 g 4-(3-Ethoxycarbonylpropyl)-1H-imidazol in 30 ml Tetrahydrofuran unter Stickstoff wird zunächst 30 min bei 0° mit 0,5 g Natriumhydrid (50 % Oeldispersion) behandelt, und anschliessend 3 h bei 0° mit 1,45 ml Trimethylsilylchlorid. Das Reaktionsgemisch wird mit kalter 0,5 N Natriumhydrogencarbonat-Lösung gewaschen, über Natriumsulfat getrocknet und zur Trockene eingedampft. Das erhaltene Oel wird bei -78° unter Stickstoff in 100 ml Methyenchlorid gelöst und 12,82 ml Diisobutylaluminiumhydrid (1,56 M) tropfenweise hinzugegeben. Das Reaktionsgemisch wird 5 min bei -78° gerührt und die Reaktion durch Zugabe von 1 ml Methanol und dann 10 ml Wasser abgebrochen. Man filtriert durch Celite®. Die organische Phase wird abgetrennt, über Natriumsulfat getrocknet und eingedampft, wobei man die Titelverbindung a) erhält.

b) 4-[4-(p-tert-Butylaminocarbonylphenyl)-4-hydroxy-n-butyl)-1-trimethylsilylimidazol. Eine Lösung von 6,95 g p-tert-Butylaminocarbonyl)-brombenzol wird in 175 ml Tetrahydrofuran bei -70° unter Stickstoff gelöst und 20,1 ml einer Lösung von n-Butyllithium (2,7 M) in Hexan tropfenweise hinzugegeben. Nach einer Reaktionszeit von 30 min wird langsam eine Lösung von 5,69 g 4-(3-Formyl-n-propyl)-1-trimethylsilylimidazol in 10 ml Tetrahydrofuran hinzugegeben. Man lässt das Reaktionsgemisch langsam auf Raumtemperatur erwärmen und gibt 20 ml einer gesättigten Ammoniumchloridlösung hinzu. Die organische Phase wird abgetrennt, über Natriumsulfat getrocknet und eingedampft, wobei man die Titelverbindung b) erhält.

c) 4-[4-Chlor-4-(p-cyanphenyl)-n-butyl]-1H-imidazol. Eine Lösung von 4,5 g 4[4-(p-tert-Butylaminocarbonylphenyl)-4-hydroxy-n-butyl]-1-trimethylsilylimidazol in 50 ml Thionylchlorid wird 1 h unter Rückfluss gekocht, abgekühlt und eingedampft. Der Rückstand wird zwischen Methylenchlorid und einer wässrigen Natriumhydrogencarbonat-Lösung verteilt. Die organische Phase wird abgetrennt, über Natriumsulfat

getrocknet und eingedampft, wobei man die Titelverbindung c) erhält.

Beispiel 3: 5-(p-Cyanphenyl)imidazo[1,5-a]pyridin. Eine Lösung von 0,1 g 5-(p-tert-Butylaminocarbonylphenyl)imidazo[1,5-a]pyridin in 3 ml Toluol wird mit 40 µl Phosphoroxychlorid 5 h bei 90° behandelt. Das Lösungsmittel wird abgedampft und der Rückstand bei 0° in 30 ml Chloroform gelöst. Man gibt eiskalte Ammoniaklösung hinzu und trennt die organische Phase ab, trocknet sie über Natriumsulfat und dampft sie ein. Der Rückstand wird mit Essigsäureethylester über Kieselgel chromatographiert, wobei man die Titelverbindung vom Smp. 117-118° erhält; das entsprechende Hydrochlorid schmilzt bei 255-257°.

Beispiel 4: 5-(p-Ethoxycarbonylphenyl)imidazo[1,5-a]pyridin. Eine Lösung von 9,8 g 2-(p-Ethoxycarbonylphenyl)-6-formylaminomethylpyridin und 11,15 g Phosphoroxychlorid in 26 ml Toluol wird 15 h auf 90° erhitzt. Das Lösungsmittel wird verdampft und der Rückstand in 50 ml Methylenchlorid aufgenommen, auf 0° abgekühlt und mit einem Ueberschuss an eiskalter, gesättigter Ammoniaklösung basisch gemacht. Die organische Phase wird abgetrennt, getrocknet und eingedampft. Der zurückbleibende Feststoff wird mit Essigsäureethylester als Elutionsmittel über 100 g Kieselgel gegeben, wobei man nach Umkristallisation die Titelverbindung vom Smp. 118-119° erhält.

Herstellung der Ausgangsverbindungen:

a) 6-Cyan-2-(p-ethoxycarbonylphenyl)pyridin. 8,9 ml 40 % Peressigsäure werden tropfenweise zu 14,08 g 2-(p-Ethoxycarbonylphenyl)-pyridin gegeben, so dass die Reaktionstemperatur zwischen 80 und 85° gehalten wird. Nach vollständiger Zugabe wird das Reaktionsgemisch 3 h auf 90° erhitzt, worauf man es auf Raumtemperatur abkühlen lässt. Ueberschüssige Peressigsäure wird mit wässriger Natriumsulfit-Lösung zerstört. Das Lösungsmittel wird abgedampft, der Rückstand in Methylenchlorid aufgenommen und durch Celite® filtriert. Nach dem Eindampfen erhält man 2-(p-Ethoxycarbonylphenyl)pyridin-N-oxid, das mit 8,66 g Dimethylsulfat in 62 ml Toluol bei 90° 3 h behandelt wird. Das Lösungsmittel wird abgedampft und der Rückstand in einer eiskalten Mischung aus 8 ml Wasser und 9,3 ml 1N Natronlauge gelöst. Eine Lösung von 13,64 g Kaliumcyanid in 10 ml Wasser wird langsam hinzugegeben und das Reaktionsgemisch 24 h bei 0° gehalten. Durch Extraktion mit Methylenchlorid, Trocknung über Natriumsulfat und Verdampfen des Lösungsmittels erhält man die Titelverbindung a); IR ($CH_2Cl_2$) 2200 $cm^{-1}$.

b) 6-Aminomethyl-2-(p-ethoxycarbonylphenyl)pyridin. 16,23 g 6-Cyan-2-(p-ethoxycarbonylphenyl)pyridin werden bei atmosphärischem Druck in 254 ml Methanol mit 12,9 ml konzentrierter Salzsäure und 2,63 g 10 % Palladium-auf-Kohle hydriert, bis 2 molare Aequivalente Wasserstoff aufgenommen worden sind. 6,9 g Natriummethoxid werden hinzugegeben und der Katalysator abfiltriert. Nach Verdampfen des Lösungsmittels wird der Rückstand in 20 ml Methylenchlorid gelöst und die Salze durch Filtration entfernt. Nach Verdampfen des Lösungsmittels erhält man einen Feststoff, der aus Chloroform umkristallisiert wird. Dieser entspricht der Titelverbindung b), Smp. 141-143°.

c) 2-(p-Ethoxycarbonylphenyl)-6-formylaminomethylpyridin. Eine Lösung von 0,76 g 6-Aminomethyl-2-(p-ethoxycarbonylphenyl)pyridin in 10 ml Ameisensäure wird 15 h auf 90° erhitzt. Das Reaktionsgemisch wird auf 0° abgekühlt, mit einem Ueberschuss gesättigter Ammoniak lösung basisch gemacht und mit Chloroform extrahiert. Die organischen Extrakte werden getrocknet und eingedampft, wobei man die Titelverbindung c) erhält, die aus Toluol umkristallisiert wird; Smp. 119,5-120,5°.

Beispiel 5: 5-(p-Carboxyphenyl)imidazo[1,5-a]pyridin . Eine Lösung von 1,18 g 5-(p-Ethoxycarbonylphenyl)imidazo[1,5-a]pyridin in 10 ml Ethanol und 14 ml 1N Natronlauge wird 3 h unter Rückfluss gekocht, abgekühlt und eingedampft. Der Rückstand wird zwischen Wasser und Essigsäureethylester verteilt. Die wässrige Phase wird abgetrennt und auf pH 5 eingestellt. Der Feststoff wird abfiltriert, mit Wasser gewaschen und getrocknet, wobei man die Titelverbindung vom Smp. 308-310° (Zersetzung) erhält.

Beispiel 6: 5-(p-tert-Butylaminocarbonylphenyl)imidazo[1,5-a]pyridin . Zu einer Aufschlämmung von 0,4 g 5-(p-Carboxyphenyl)imidazo[1,5-a] pyridin in 40 ml Methylenchlorid unter Stickstoff bei Raumtemperatur werden 30 µl N,N-Dimethylformamid gegeben, daran anschliessend 0,16 ml Oxalylchlorid. Das Reaktionsgemisch wird gerührt, bis die Gasentwicklung beendet ist; dann werden 0,46 ml tert-Butylamin tropfenweise zugegeben. Nach 90 min hört man auf zu rühren, und 10 ml gesättigte Natriumhydrogencarbonat-Lösung werden hinzugegeben. Die organische Phase wird abgetrennt, über Natriumsulfat getrocknet und eingedampft, wobei man die Titelverbindung vom Smp. 128-131° erhält.

Beispiel 7: 5-(p-Cyanphenyl)-5,6,7,8-tetrahydroimidazo[1,5-a]pyridin-Hydrochlorid . Eine Lösung von 1,13 g 5-(p-Carbamoylphenyl)-5,6,7,8-tetrahydroimidazo[1,5-a]pyridin und 1,0 ml Phosphoroxychlorid in 30 ml Chloroform wird 15 h unter Rückfluss gekocht, gekühlt und nach Zugabe von Toluol eingedampft. Das erhaltene Oel wird in 30 ml Methylenchlorid gelöst, auf 0° abgekühlt und mit 30 ml einer eiskalten 50%

Ammoniaklösung versetzt. Die organische Phase wird abgetrennt, getrocknet und zu einem Oel eingedampft. Mittels Filtration durch 20 g Kieselgel mit Essigsäureethylester als Elutionsmittel erhält man die freie Titelverbindung, die in 20 ml Aceton gelöst und mit 1,2 ml 3N etherischem Chlorwasserstoff behandelt wird. Auf diese Weise erhält man das entsprechende Hydrochlorid vom Smp. 209-210°.

Beispiel 8: 5-(p-Ethoxycarbonylphenyl)-5-6,7,8-tetrahydroimidazo[1,5-a]pyridin-Hydrochlorid. Eine Lösung von 2,0 g 5-(p-Ethoxycarbonylphenyl)imidazo[1,5-a]pyridin in 120 ml wasserfreiem Ethanol, dass 30 ml konzentrierte Salzsäure enthält, wird mit 1,0 g 10 % Palladium-auf-Kohle bei einem Wasserstoffdruck von 2,76 bar und einer Temperatur von 60° 4 h hydriert. Der Katalysator wird abfiltriert und das Lösungsmittel abgedampft, wobei man einen Feststoff erhält, der aus Isopropanol und Ether umkristallisiert wird und der Titelverbindung vom Smp. 164-166° entspricht.

Beispiel 9: 5-(p-Carboxyphenyl)-5,6,7,8-tetrahydroimidazo[1,5-a]pyridin. Eine Lösung von 0,66 g 5-(p-Ethoxycarbonylphenyl)-5,6,7,8-tetrahydroimidazo[1,5-a]pyridin in 8,0 ml Ethanol und 8,0 ml 1N Natronlauge wird 3 h unter Rückfluss gekocht, abgekühlt und eingedampft. Der Rückstand wird zwischen Wasser und Essigsäureethylester verteilt. Die wässrige Phase wird mit konzentrierter Schwefelsäure auf pH 5 eingestellt, der Feststoff abfiltriert und an der Luft getrocknet. Er entspricht der Titelverbindung, Smp. 309-310° (Zersetzung).

Beispiel 10: 5-(p-Carbamoylphenyl)-5,6,7,8-tetrahydroimidazo[1,5-a]pyridin. Eine Lösung von 5,42 g 5-(p-Carboxyphenyl)-5,6,7,8-tetrahydroimid azo[1,5-a]pyridin in 75 ml Thionylchlorid wird 30 min unter Rückfluss gekocht, abgekühlt und nach Zugabe von Toluol eingedampft. Der Rückstand wird in Methylenchlorid gelöst, auf 0° abgekühlt und mit Ammoniakgas behandelt, bis die Lösung gesättigt ist. Das Reaktionsgemisch wird 10 min unter einer Ammoniakatmosphäre gerührt und der entstandene Feststoff durch Filtration gesammelt, wobei man die Titelverbindung vom Smp. 181-183° erhält. Behandelt man diese mit einem molaren Aequivalent Fumarsäure, gelöst in Ethanol, so erhält man das entsprechende Fumarat vom Smp. 164-166° (Zersetzung).

Beispiel 11: 5-(p-Tolyl)-5,6,7,8-tetrahydroimidazo[1,5-a]pyridin-Hydrochlorid. Eine Lösung von 0,36 g 5-(p-Hydroxymethylphenyl)imidazo[1,5-a]pyridin in 25 ml Ethanol und 6,4 ml konzentrierter Salzsäure wird mit 0,15 g 10 % Palladium-auf-Kohle bei einem Wasserstoffdruck von 2,76 bar und einer Temperatur von 60° 4 h hydriert. Das Reaktionsgemisch wird filtriert und eingedampft, und der Rückstand wird zwischen Methylenchlorid und einer Natriumhydrogencarbonat-Lösung verteilt. Die organische Phase wird über Natriumsulfat getrocknet und zu einem Oel eingedampft, das durch präparative Schichtchromatographie auf Kieselgel mit Essigsäureethylester gereinigt wird. Das entsprechende Hydrochlorid, d.h. die Titelverbindung, wird durch Behandlung mit 1,1 molaren Aequivalenten an etherischem Chlorwasserstoff in Aceton hergestellt; Smp. 173-175°.

Beispiel 12: 5-(p-Hydroxymethylphenyl)imidazo[1,5-a]pyridin. 1 g 5-(p-Ethoxycarbonylphenyl)imidazo-[1,5-a]pyridin wird in 26 ml Methylenchlorid bei -78° unter Stickstoff gelöst, worauf 6,6 ml Diisobutylaluminiumhydrid in Toluol (11,4 mmol) tropfenweise hinzugegeben werden. Nach 1 h Rühren werden 1,5 ml Methanol hinzugegeben, das Kältebad wird entfernt und 15 ml Wasser hinzugefügt. Die Salze werden abfiltriert, die organische Phase über Natriumsulfat getrocknet und eingedampft, wobei man die Titelverbindung vom Smp. 137-138° erhält.

Beispiel 13: 5-(p-Cyanphenyl)-7,8-dihydroimidazo[1,5-a]pyridin. Eine Lösung von 0,24 g 1-(p-Cyanphenylmethyl)-5-(2-formylethyl)-1H-imidazol in 10 ml wasserfreiem Ethanol wird zusammen mit 20 mg Kaliumtert-butoxid 2 h unter Stickstoff unter Rückfluss gekocht, abgekühlt und eingedampft, wobei man die Titelverbindung erhält.

Herstellung der Ausgangsverbindung:

a) 1-(p-Cyanphenylmethyl )-5-(2-formylethyl )-1H-imidazol. Eine Lösung von 0,14 ml Dimethylsulfoxid in 5 ml Methylenchlorid wird unter Stickstoff auf -78° abgekühlt, dann 0,1 ml Oxalylchlorid tropfen weise hinzugefügt. Nach 30 min wird eine Lösung 0,24 g 1-(p-Cyanphenylmethyl)-5-(3-hydroxypropyl)-1H-imidazol in 1 ml Methylenchlorid und 0,2 ml Dimethylsulfoxid langsam hinzugegeben. Das Reaktionsgemisch wird 2 h bei -78° gerührt, dann 1 ml Triethylamin langsam hinzugegeben. Man lässt das Reaktionsgemisch langsam auf Raumtemperatur erwärmen, verdünnt es mit 30 ml Methylenchlorid und wäscht es dreimal mit 10 ml Wasser. Die organische Phase wird über Natriumsulfat getrocknet und eingedampft, wobei man die Titelverbindung a) als Oel erhält; NMR (60 MHZ): δ 5,15 (s, 2H), 9,65 (s, 1H).

Beispiel 14: 5-(p-Cyanphenyl )-5,6,7,8-tetrahydroimidazo[1,5-a]pyridin. Eine Lösung von 1,6 g 5-(p-Cyanphenyl)-7,8-dihydroimidazo[1,5-a]pyridin in 50 ml Essigsäureethylester wird bei atmosphärischem

Druck mit 0,2 g 5 % Palladium-auf-Kohle hydriert, bis die theoretische Wasserstoffmenge aufgenommen ist. Der Katalysator wird abfiltriert und das Lösungsmittel abgedampft, wobei man die Titelverbindung vom Smp. 117-118° erhält.

Beispiel 15: 5-(p-Cyanphenyl)-5,6,7,8-tetrahydroimidazo[1,5-a]pyridin . Eine Lösung von 54 mg 5-(p-Cyanphenyl)imidazo[1,5-a]pyridin-Hydrochlorid in 5,0 ml Methanol wird bei Raumtemperatur und atmosphärischem Druck 30 min mit 0,1 g 10 % Palladium-auf-Kohle hydriert. Der Katalysator wird abfiltriert und 0,21 ml 1N Natronlauge werden hinzugegeben. Das Filtrat wird eingedampft, in 10 ml Methylenchlorid aufgenommen und durch Celite® filtriert. Nach dem Eindampfen erhält man ein Oel, das auf Kieselgel mit Essigsäureethylester chromatographiert wird, wobei man die Titelverbindung von Smp. 117-118° erhält.

Beispiel 16: 5-(p-Cyanphenyl)-5,6,7,8-tetrahydroimidazo[1,5-a]pyridin . Eine Mischung aus 85 mg 5-(p-Bromphenyl)-5,6,7,8-tetrahydroimidazo[1,5-a]pyridin und 74 mg Kupfer(I)cyanid in 1 ml N,N-Dimethylformamid wird unter Stickstoff 11 h auf 120° erhitzt. Das Reaktionsgemisch wird abgekühlt, mit 10 ml Wasser verdünnt und mit Essigsäureethylester extrahiert. Die organischen Extrakte werden über Natriumsulfat getrocknet und eingedampft. Das erhaltene Oel wird mit Essigsäureethylester über Kieselgel chromatographiert, und man erhält die Titelverbindung vom Smp. 117-118°.

Beispiel 17: 5-(p-Bromphenyl )-5,6,7,8-tetrahydroimidazo[1-5-a]pyridin . Eine Lithiumdiisopropylamid-Lösung, hergestellt bei 0° aus 0,12 ml Diisopropylamin und 0,33 ml n-Butyllithium (2,5 M) in 2 ml Tetrahydrofuran unter Stickstoff, wird zu einer Lösung von 0,13 ml N,N,N',N'-Tetramethyl-ethylendiamin und 0,124 g 1-(p-Brombenzyl)-5-(3-chlorpropyl)-1H-imidazol in 2 ml Tetrahydrofuran bei -78° gegeben. Das Reaktionsgemisch wird 3,5 h gerührt, die Reaktion bei -78° mittels Zugabe von gesättigter Ammoniumchlorid-Lösung abgebrochen und das Gemisch mit Methylenchlorid (3x10 ml) extrahiert. Die organischen Extrakte werden über Natriumsulfat getrocknet und eingedampft, wobei man die Titelverbindung erhält, die durch Umwandlung in das entsprechende Hydrochlorid gereinigt wird, Smp. 216°.

Herstellung der Ausgangsverbindungen:

a) 1-(p-Brombenzyl )-5-(3-hydroxypropyl)-1H-imidazol . Eine Lösung von 11,2 g 1-Dimethylcarbamoyl-4-(3-trimethylsilyloxypropyl)-1H-imidazol und 12,49 g p-Brombenzylbromid in 110 ml Acetonitril wird 24 h unter Rückfluss gekocht. Die Lösung wird auf 0° gekühlt und Ammoniakgas 5 min durch das Reaktionsgemisch geleitet. Nach weiteren 45 min bei Raumtemperatur wird das Lösungsmittel abgedampft. Der Rückstand wird in 100 ml 1N Salzsäure aufgenommen und mit 50 ml Ether extrahiert. Die wässrige Phase wird auf pH 8 eingestellt und mit Essigsäureethylester extrahiert (5x50 ml). Die organischen Extrakte werden mit Wasser gewaschen, über Natriumsulfat getrocknet und eingedampft. Das erhaltene Oel wird über 530 g Kieselgel mit Essigsäureethylester: Methanol:gesättigte Ammoniaklösung (90:5:5) chromatographiert, wobei man die Titelverbindung a) als Oel erhält; NMR: $\delta$ 5,00 (s, 2H).

b) 1-(p-Brombenzyl )-5-(3-chlorpropyl)-1H-imidazol . 1-(p-Brombenzyl)-5-(3-hydroxypropyl)-1H-imidazol wird analog zu dem in Beispiel 1c) beschriebenen Verfahren mit Thionylchlorid behandelt, wobei man die Titelverbindung b) erhält.

Beispiel 18: 5-(p-Cyanphenyl )-5,6,7,8-tetrahydroimidazo[1,5-a]pyridin . Eine Lösung von 2,01 g 5-(p-Formylphenyl)-5,6,7,8-tetrahydroimidazo[1,5-a]pyridin und 0,96 g Stickstoffwasserstoffsäure in 30 ml Benzol wird durch externe Kühlung bei Raumtemperatur gehalten, während 0,8 ml konzentrierte Schwefelsäure tropfenweise zugegeben werden. Das Reaktionsgemisch wird 2 h gerührt und dann neutralisiert. Die organische Phase wird abgetrennt, über Natriumsulfat getrocknet und eingedampft, wobei man ein Oel erhält, das mit Essigsäureethylester über Kieselgel chromatographiert wird. Auf diese Weise erhält man die Titelverbindung; Smp. 117-118°.

Beispiel 19: 5-(p-Hydroxymethylphenyl)-5,6,7,8-tetrahydroimidazo[1,5-a]pyridin . Eine Lösung von 0,40 g 5-(p-Ethoxycarbonylphenyl)-5,6,7,8-tetrahydroimidazo[1,5-a]pyridin in 20 ml Methylenchlorid wird unter Stickstoff auf -70° gekühlt, und 4,0 ml 1,53 M Diisobutylaluminium hydrid-Lösung in Toluol werden tropfenweise hinzugegeben. Man lässt das Reaktionsgemisch auf Raumtemperatur erwärmen, bricht die Reaktion durch Zugabe von 3,2 ml Methanol und 15 ml Wasser ab und filtriert durch Celite®. Die Phasen werden getrennt, die organische Phase wird über Natriumsulfat getrocknet und eingedampft, wobei man die Titelverbindung vom Smp. 142-145° erhält.

Beispiel 20: 5-(p-Formylphenyl )-5,6,7,8-tetrahydroimidazo[1,5-a]pyridin . Eine Lösung von 0,16 ml Dimethylsulfoxid in 16 ml Methylenchlorid wird unter Stickstoff auf -70° gekühlt und 0,17 g Oxalylchlorid werden tropfenweise hinzugegeben. Das Reaktionsgemisch wird 30 min gerührt, dann 0,24 g 5-(p-Hydroxymethylphenyl)-5,6,7,8-tetrahydroimidazo[1,5-a]pyridin, gelöst in 4 ml Methylenchlorid, langsam hinzugegeben. Das Reaktionsgemisch wird 2 h bei -70° gerührt, dann werden 0,8 ml Triethylamin tropfenweise hinzugegeben, und man lässt das Reaktionsgemisch langsam auf Raumtemperatur erwärmen. Die Mischung wird mit 20 ml Methylenchlorid verdünnt, mit Wasser gewaschen, über Natriumsulfat getrocknet und eingedampft, wobei man die Titelverbindung erhält, die durch Umwandlung in das entsprechende Fumarat gereinigt wird; Smp. 131°.

Beispiel 21: 5-(p-Cyanphenyl )-5-methylthio-5,6,7,8-tetrahydroimidazo[1,5-a]pyridin-Hydrochlorid . Eine Lösung von Lithiumdiisopropylamid wird aus 0,6 ml n-Butyllithium (2,5 M) und 0,15 g Diisopropylamin in 5 ml trockenem Tetrahydrofuran bei 0° unter Stickstoff hergestellt. Sie wird zu 0,29 g 5-(p-Cyanphenyl)-5,6,7,8-tetrahydroimidazo[1,5-a]pyridin in 10 ml Tetrahydrofuran bei -78° gegeben. Das Reaktionsgemisch wird 30 min gerührt, dann tropfenweise 0,14 g Dimethyldisulfid hinzugegeben. Nach 30 min wird die Kühlung unterbrochen, man lässt das Reaktionsgemisch auf Raumtemperatur erwärmen und bricht die Reaktion durch Zugabe von 10 ml gesättigter Ammoniumchlorid-Lösung ab. Die Phasen werden getrennt, und die organische Phase wird mit kalter 1N Salzsäure gewaschen. Die wässrige Phase wird neutralisiert und mit Essigsäureethylester extrahiert. Die organischen Extrakte werden über Natriumsulfat getrocknet und zu einem Oel eingedampft, das mit 5 % Isopropanol in Essigsäureethylester über Kieselgel chromatographiert wird. Das erhaltene Oel wird in Aceton gelöst und mit 0,1 ml 4N etherischem Chlorwasserstoff behandelt, wobei man die Titelverbindung vom Smp. 204-205° erhält.

Beispiel 22: 5-(p-Cyanphenyl )-5-ethoxycarbonyl-5,6,7,8-tetrahydroimidazo[1,5-a]pyridin . In analoger Weise wie in Beispiel 21 beschrieben erhält man durch Reaktion von 5-(p-Cyanphenyl)-5,6,7,8-tetrahydroimidazo[1,5-a]pyridin mit Chlorameisensäureethylester die Titelverbindung.

Beispiel 23: 5-(p-Cyanphenyl )-5,6,7,8-tetrahydroimidazo[1,5-a]pyridin . Eine Lösung von 1,65 g 5-(p-Cyanphenyl)-5-ethoxycarbonyl-5,6,7,8-tetrahydroimidazo[1,5-a]pyridin in 10 ml Methanol, das 0,2 g Natriumhydroxid enthält, wird 3 h bei Raumtemperatur gerührt, worauf 5 ml 1N Salzsäure hinzugegeben werden. Das Reaktionsgemisch wird 1 h unter Rückfluss gekocht, gekühlt und eingedampft. Der Rückstand wird zwischen Wasser und Essigsäureethylester verteilt. Die organische Phase wird abgetrennt, über Natriumsulfat getrocknet und eingedampft, wobei man die Titelverbindung erhält; Smp. 117-118°.

Herstellung der Ausgangsverbindungen:

a) Eine Lösung von 1,9 g p-Cyanphenylessigsäureethylester in 50 ml Diglyme wird zu einem Gemisch von 0,48 g Natriumhydrid (50 % Oeldispersion) in 10 ml Diglyme gegeben. Das Reaktionsgemisch wird 2 h bei Raumtemperatur gerührt, auf 0° abgekühlt und mit 1,75 g N-Bromsuccinimid, verteilt auf mehrere Portionen, versetzt. Das Lösungsmittel wird im Hochvakuum verdampft und der Rückstand mit Ether über 50 g Kieselgel chromatographiert, wobei man α-Brom-p-cyanphenylessigsäureethylester erhält.

b) Eine Lösung von 97,0 g 4-(3-Trimethylsilyloxypropyl)-1H-imidazol-1-N,N-dimethyl-carboxamid und 72,0 g a-Brom-p-cyanphenylessigsäureethylester in 500 ml Acetonitril wird 10 h unter Rückfluss gekocht. Die Lösung wird in einem Eisbad auf 0° abgekühlt und für wenige Minuten Ammoniakgas eingeleitet. Das Gemisch wird im Vakuum eingedampft, wobei man einen Rückstand erhält, der in 500 ml 1N Salzsäure gelöst wird. Man lässt die Lösung 15 min bei Raumtemperatur stehen und extrahiert sie dann mit Ether. Der pH-Wert der wässrigen Phase wird mit 50 % Natronlauge auf 9 eingestellt, und das Gemisch mit Methylenchlorid extrahiert. Die Methylenchlorid-Extrakte werden mit Wasser gewaschen, über Natriumsulfat getrocknet und eingedampft, wobei man 1-(α-Ethoxycarbonyl-p-cyanbenzyl)-1H-imidazol-5-propanol erhält.

c) Zu einer Lösung von 5,75 g Thionylchlorid in 80 ml Methylenchlorid werden 8,4 g 1-(α-Ethoxycarbonyl-p-cyanbenzyl)-1H-imidazol-5-propanol als Feststoff portionsweise hinzugegeben. Nach erfolgter Zugabe wird die Lösung 1,5 h unter Rückfluss gekocht, im Eisbad abgekühlt und filtriert, wobei man 5-(3-Chlorpropyl)-1-(α-ethoxycarbonyl-p-cyanbenzyl)-1H-imidazol-Hydrochlorid erhält. Das Salz wird zwischen Methylenchlorid und gesättigter Natriumhydrogencarbonatlösung verteilt. Die organischen Extrakte werden mit Wasser gewaschen, über Natriumsulfat getrocknet und eingedampft, wobei man die freie Base erhält.

d) Eine Lösung von 8,1 g 5-(3-Chlorpropyl)-1-(α-ethoxycarbonyl-p-cyanbenzyl)-1H-imidazol in 50 ml Tetrahydrofuran wird in einem Eisbad auf 0° gekühlt. Hierzu gibt man 8,0 g Kalium-tert-butoxid als Feststoff in mehreren Portionen. Das Gemisch wird 2 h bei Raumtemperatur gerührt, mit 10 % Essigsäure neutralisiert und zwischen Methylenchlorid und Wasser verteilt. Die organische Phase wird mit Wasser gewaschen, über Magnesiumsulfat getrocknet und eingedampft, wobei man ein Oel erhält, das in einem kleinen Volumen an Aceton gelöst und mit etherischem Chlorwasserstoff neutralisiert wird. Der Feststoff wird gesammelt, und man erhält 5-(p-Cyanphenyl)-5-ethoxycarbonyl-5,6,7,8-tetrahydroimidazo[1,5-a]pyridin.

Beispiel 24: 5-(p-Cyanphenyl )-5,6,7,8-tetrahydroimidazo[1,5-a]pyridin . Eine Lösung von 2,13 g 5-(p-Aminophenyl)-5,6,7,8-tetrahydroimidazo[1,5-a]pyridin in 4 ml konzentrierter Salzsäure und 10 ml Wasser wird in einem Eisbad gekühlt, worauf eine Lösung von 0,78 g Natriumnitrit in 2 ml Wasser langsam hinzugegeben wird. Dieses Gemisch wird über einen Tropftrichter zu einer eisgekühlten Lösung von 3,0 g Kupfer(I)cyanid in 10 ml Wasser gegeben, wobei die Temperatur zwischen 30 und 40° gehalten wird. Das Reaktionsgemisch wird 1 h auf dem Dampfbad erhitzt, abgekühlt und auf pH 9 gebracht. Die organischen Extrakte werden über Natriumsulfat getrocknet und eingedampft und der Rückstand mit Essigsäureethylester über Kieselgel chromatographiert, wobei man die Titelverbindung erhält.

Beispiel 25: 5(p-Aminophenyl)-5,6,7,8-tetrahydroimidazo[1,5-a]pyridin . Eine Lösung von 2,42 g 5-(p-Carboxyphenyl)-5,6,7,8-tetrahydroimidazo[1,5-a]pyridin in 100 ml Ethylendichlorid wird mit 6 ml konzentrierter Schwefelsäure behandelt. Das Reaktionsgemisch wird auf 40° erhitzt, dann werden 6 ml Stickstoffwasserstoffsäure (2 M in Ethylendichlorid) tropfenweise hinzugegeben. Wenn die Gasentwicklung beendet ist, wird das Reaktionsgemisch eingedampft. Der Rückstand wird in Wasser gelöst und auf pH 10 eingestellt. Die wässrige Phase wird mit Methylenchlorid extrahiert (3x30 ml). Die organischen Extrakte werden über Kaliumcarbonat getrocknet und eingedampft, wobei man die Titelverbindung erhält.

Beispiel 26: Es werden 10.000 Tabletten hergestellt, die je 10 mg des Wirkstoffs enthalten:

Zusammensetzung:

| | |
|---|---|
| 5-(p-Cyanphenyl)-5,6,7,8-tetrahydroimidazo-[1,5-a]pyridin | 100,00 g |
| Milchzucker | 2535,00 g |
| Maisstärke | 125,00 g |
| Polyethylenglykol 6.000 | 150,00 g |
| Magnesiumstearat | 40,00 g |
| Gereinigtes Wasser | quantum satis |

Verfahren:

Alle pulverigen Bestandteile werden durch ein Sieb mit einer Maschenweite von 0,6 mm gesiebt. Dann wird der Wirkstoff, Milchzucker, Magnesiumstearat und die Hälfte der Stärke in einem geeigneten Mischer vermischt. Die andere Hälfte der Stärke wird in 65 ml Wasser suspendiert und die Suspension zur siedenden Lösung des Polyethylenglykols in 260 ml Wasser gegeben. Die gebildete Paste wird den Pulvern zugesetzt und gegebenenfalls unter Zugabe von mehr Wasser granuliert. Das Granulat wird über Nacht bei 35° getrocknet, durch ein Sieb mit 1,2 mm Maschenweite getrieben und zu Tabletten, welche eine Bruchrille aufweisen, gepresst.

Beispiel 27: Es werden 1.000 Kapseln hergestellt, die je 20 mg Wirkstoff enthalten:

Zusammensetzung:

| | |
|---|---|
| 5-(p-Cyanphenyl)-5,6,7,8-tetrahydroimidazo-[1,5-a]pyridin | 20,00 g |
| Milchzucker | 207,00 g |
| Modifizierte Stärke | 80,00 g |
| Magnesiumstearat | 3,00 g |

Verfahren:

Alle pulverigen Bestandteile werden durch ein Sieb mit einer Maschenweite von 0,6 mm gesiebt. Dann wird der Wirkstoff in einem geeigneten Mischer zuerst mit Magnesiumstearat und dann mit dem Milchzukker und der Stärke bis zur Homogenität vermischt. Hartgelatinekapseln Nr. 2 werden mit je 310 mg der erhaltenen Mischung unter Verwendung einer Kapselfüllmaschine gefüllt.

Entsprechend den Beispielen 26 und 27 werden Tabletten und Kapseln hergestellt, die etwa 10 bis 100 mg anderer erfindungsgemässer Verbindungen enthalten, z.B. von jeder anderen in den Beispielen beschriebenen Verbindung.

Beispiel 28: Eine Lösung von 0,52 g 5-(p-Hydroxymethylphenyl)imidazo[1,5-a]pyridin in 10 ml Methylenchlorid wird mit 5,2 g aktiviertem Mangandioxid 24 h unter Rückfluss gekocht. Es werden weitere 5,2 g Mangandioxid hinzugegeben und das Reaktionsgemisch nochmals 6 h gekocht, dann abfiltriert. Nach Verdampfen des Lösungsmittels erhält man 5-(p-Formylphenyl)imidazo[1,5-a]pyridin, Smp. 144-146°.

Beispiel 29: Eine Lösung von 0,18 g 5(p-Carboxyphenyl)imidazo[1,5-a]pyridin-Hydrochlorid in 5 ml Thionylchlorid wird 30 min unter Rückfluss gekocht und zur Trockene eingedampft. Das erhaltene Oel wird in 10 ml Methylenchlorid aufgenommen und 1 h lang bei 0° Ammoniakgas in die Lösung geleitet. Die Lösung wird mit Wasser gewaschen und über Natriumsulfat getrocknet. Nach dem Eindampfen erhält man 5-(p-Carbamoylphenyl)imidazo[1,5-a]pyridin, Smp. 228-230° (Zers.).

Beispiel 30: Eine Lösung von 3,13 g 4-[3-(4-tert-Butylaminocarbonylphenyl)-3-chlorprop-1-yl]-1-tritylimidazol in 150 ml Acetonitril wird 15 h unter Rückfluss gekocht, abgekühlt und mit 150 ml Methanol versetzt. Das Reaktionsgemisch wird weitere 15 h gekocht, dann zur Trockene eingedampft. Der Rückstand wird zwischen Ether und Wasser verteilt. Die etherische Phase wird abgetrennt und mit 1N HCl (2x15 ml) gewaschen. Die vereinigten wässrigen Extrakte werden auf pH 8 eingestellt und mit Methylenchlorid extrahiert. Der Methylenchloridextrakt wird über Natriumsulfat getrocknet, filtriert und zu einem weissen Schaum eingedampft. Nach Umkristallisieren aus Ether erhält man 1,30 g 5H-5-(4-tert-Butylaminocarbonylphenyl) 6,7-dihydropyrrolo[1,2-c]imidazol, Smp. 136-139°.

Herstellung der Ausgangsverbindungen:

a) Eine Lösung von 6,0 g 3-(1H-Imidazol-4-yl)propionsäuremethylester und 11 ml Triethylamin in 31 ml Dimethylformamid wird mit einer Lösung von 9,65 g Triphenylmethylchlorid in 110 ml Dimethylformamid 2 h bei Raumtemperatur unter Stickstoff behandelt. Das Reaktionsgemisch wird auf 700 g Eis gegossen, der entstandene Feststoff durch Filtration gesammelt und aus Ether umkristallisiert, wobei man 13,83 g 3-(1-Tritylimidazol-4-yl)propionsäuremethylester erhält; NMR ($CDCl_3$) $\delta = 2{,}75$ (m,4H), 3,05 (s,3H), 6,5-7,5 (m,17H).

b) Eine Lösung von 44,4 mmol Diisobutylaluminiumhydrid in 29 ml Toluol wird bei -72° unter Stickstoff zu einer Lösung von 8,79 g 3-(1-Tritylimidazol-4-yl)propionsäuremethylester in 175 ml Methylenchlorid gegeben. Nach 5 min wird die Reaktion durch Zugabe von 14 ml Methanol und anschliessend 90 ml Wasser abgebrochen. Man lässt das Reaktionsgemisch auf Raumtemperatur erwärmen und filtriert es durch Celite®. Die organische Phase wird abgetrennt, über Natriumsulfat getrocknet und zu einem gelben Oel eingedampft, das auf 280 g Kieselgel mit Ether chromatographiert wird; man erhält 4,13 g 3-(1-Tritylimidazol-4-yl)propionaldehyd als Oel. IR ($CDCl_3$): 2830, 2740, 1730 $cm^{-1}$.

c) Eine Lösung von 25 mmol n-Butyllithium in 10 ml Hexan wird tropfenweise bei -70° unter Argon zu einer Lösung von 3,19 g N-tert-Butyl-4-brombenzamid in 250 ml Tetrahydrofuran gegeben. Nach 30 min wird langsam eine Lösung von 3,74 g 3-(1-Tritylimidazol-4-yl)propionaldehyd in 100 ml Tetrahydrofuran zugegeben. Das Reaktionsgemisch wird 30 min bei 70° gerührt; dann lässt man es auf 25° erwärmen, rührt es 2,5 h bei 25° und bricht die Reaktion durch Zugabe eines Ueberschusses an gesättigter Ammoniumchloridlösung ab. Die wässrige Phase wird abgetrennt und mit Methylenchlorid (2x100 ml) extrahiert. Die vereinigten organischen Extrakte werden über Natriumsulfat getrocknet und eingedampft. Der Rückstand wird mit 5:1 Ether:Essigsäureethylester über 220 g Kieselgel chromatographiert, wobei man 4-[3-(4-tert-Butylaminocarbonylphenyl)-3-hydroxyprop-1-yl]-1-tritylimidazol als Oel erhält. IR ($CH_2Cl_2$):1660 $cm^{-1}$.

d) Eine Lösung von 3,21 g 4-[3-(4-tert-Butylaminocarbonylphenyl)-3-hydroxyprop-1-yl]-1-tritylimidazol und 1,5 ml Thionylchlorid in 50 ml Methylenchlorid wird 1 h unter Rückfluss gekocht, abgekühlt und in 50 ml einer eiskalten Natriumhydrogencarbonat-Lösung gegossen. Die organische Phase wird abgetrennt, über Natriumsulfat getrocknet und eingedampft, wobei man 4-[3-(4-tert-Butylaminocarbonylphenyl)-3-chlorprop-1-yl]-1-tritylimidazol als weissen Schaum erhält. NMR ($CDCl_3$): $\delta$=1,45 (s,9H), 4,30

(t,J=6,0 Hz, 2H).

Beispiel 31: Eine Lösung von 1,25 g 5H-5-(4-tert-Butylaminocarbonylphenyl)-6,7-dihydropyrrolo[1,2-c]-imidazol in 10 ml Thionylchlorid wird 1 h unter Rückfluss gekocht, abgekühlt und eingedampft. Der Rückstand wird bei 0° in 10 ml Chloroform aufgenommen und 10 ml eiskalter wässriger Ammoniaklösung langsam hinzugegeben. Die wässrige Phase wird abgetrennt, mit Chloroform (3x20 ml) extrahiert und die vereinigten organischen Extrakte über Natriumsulfat getrocknet. Nach dem Filtrieren, Eindampfen und einer Chromatographie mit 5 % wässriger Ammoniaklösung in Essigsäureethylester über 45 g Kieselgel erhält man ein Oel, das mit einem molaren Aequivalent etherischem Chlorwasserstoff behandelt wird und 0,5 g 5H-5-(4-Cyanphenyl)-6,7-dihydropyrrolo[1,2-c]imidazol-Hydrochlorid, Smp. 227-228°, ergibt.

Beispiel 32: Eine Lösung von 1,29 g 5H-5-(4-tert-Butylaminocarbonylphenyl)-6,7,8,9-tetrahydroimidazo-[1,5a]azepin in 10 ml Thionylchlorid wird 1 h unter Rückfluss gekocht, abgekühlt und eingedampft. Der Rückstand wird zwischen Methylenchlorid und einer eiskalten Natriumhydrogencarbonatlösung verteilt. Die wässrige Phase wird abgetrennt und mit Methylenchlorid (3x15 ml) extrahiert. Die vereinigten organischen Phasen werden über Natriumsulfat getrocknet und eingedampft. Das erhaltene Oel wird mit 5 % Methanol in Methylenchlorid über 26 g Kieselgel chromatographiert. Das Produkt wird mit 1 molaren Aequivalent Fumarsäure, gelöst in Ethanol, behandelt, und man erhält 5H-5-(4-Cyanphenyl)-6,7,8,9-tetrahydroimidazo-[1,5-a]azepin-Fumarat, Smp. 153-155°.

Die Ausgangsverbindung wird aus 5-(1-Tritylimidazol-4-yl)-1-pentansäureethylester hergestellt, und zwar analog zur oben beschriebenen Herstellung von 5H-5-(4-tert-Butylaminocarbonylphenyl)-6,7-dihydropyrrolo-[1,2-c]imidazol aus 3-(1-Tritylimidazol-4-yl)propionsäureethylester. 5-(1-Tritylimidazol-4-yl)-1-pentansäureethylester wird wie folgt hergestellt:

a) Eine Lösung von 5,6 ml Diisopropylamin in 150 ml Tetrahydrofuran bei -70° unter Stickstoff wird mit 14,5 ml einer 2,5 M n-Butyllithiumlösung 30 min behandelt; dann werden 7,2 ml Triethylphosphonoacetat zugetropft. Nach 30 min wird eine Lösung von 10,09 g 3-(1-Tritylimidazol-4-yl)propionaldehyd in 50 ml Tetrahydrofuran langsam zugegeben. Man lässt das Reaktionsgemisch langsam auf Raumtemperatur erwärmen, rührt 15 h und bricht die Reaktion durch Zugabe von überschüssiger gesättigter Ammoniumchloridlösung ab. Die wässrige Phase wird abgetrennt und mit Essigsäureethylester (2x50 ml) extrahiert. Die vereinigten organischen Extrakte werden über Natriumsulfat getrocknet und zu einem Oel (15,35 g) eingedampft, das mit Ether über 430 g Kieselgel chromatographiert wird und 9,61 g 5-(1-Tritylimidazol-4-yl)-1-pent-2-ensäureethylester vom Smp. 86-88° ergibt.

b) Eine Lösung von 9,20 g 5-(1-Tritylimidazol-4-yl)-1-pent-2-ensäureethylester in 460 ml wasserfreiem Ethanol wird mit 1,88 g 10 % Palladium-auf-Kohle bei atmosphärischem Druck 20 min hydriert. Der Katalysator wird mittels Filtration durch Celite® entfernt. Nach dem Eindampfen erhält man einen Feststoff, der aus Hexan umkristallisiert wird; es ergeben sich 8,64 g 5-(1-Tritylimidazol-4-yl)-1-pentansäureethylester vom Smp. 84-86°.

Beispiel 33: Eine Lösung von 1,27 g 5-[1-(4-Cyanbenzyl)imidazol-5-yl]-1-pent-2-ensäureethylester in 27 ml Tetrahydrofuran bei 5° unter Stickstoff wird mit 0,52 g Kalium-tert-butoxid behandelt. Das Reaktionsgemisch wird 2 h bei 5° gerührt, dann 10 ml 1N Salzsäure hinzugegeben. Die Phasen werden getrennt. Die organische Phase wird mit 1N Salzsäure (2x10 ml) extrahiert. Die vereinigten wässrigen Phasen werden mit Ether extrahiert, auf pH=8 eingestellt und mit Methylenchlorid (3x15 ml) extrahiert. Die organische Phase wird getrocknet und eingedampft, wobei man das Endprodukt erhält, das mit 1 molaren Aequivalent etherischem Chlorwasserstoff behandelt wird. Der erhaltene Feststoff wird aus Aceton umkristallisiert, und man erhält 5-(4-Cyanphenyl)-6-ethoxycarbonylmethyl-5,6,7,8-tetrahydroimidazo[1,5-a]pyridin, Smp. 126-127°.

Herstellung der Ausgangsverbindungen:

a) Eine Lösung von 2,9 ml trockenem Dimethylsulfoxid in 250 ml Methylenchlorid wird unter Stickstoff auf -78° gekühlt und 2,1 ml Oxalylchlorid tropfenweise hinzugegeben. Nach 30 min bei -78° wird eine Lösung von 5.0 g 3-[1-(4-Cyanbenzyl)imidazol-5-yl]-1-propanol in 18 ml Dimethylsulfoxid langsam hinzugegeben. Das Reaktionsgemisch wird 2 h gerührt, dann werden 10,4 ml Triethylamin zugegeben. Man lässt das Reaktionsgemisch auf Raumtemperatur erwärmen und wäscht mit Wasser (4x100 ml). Die organische Phase wird über Natriumsulfat getrocknet und eingedampft, wobei man 4,13 g 3-[1-(4-Cyanbenzyl)imidazol-5-yl]-1-propionaldehyd erhält. IR ($CH_2Cl_2$): 2750, 2250, 1732 $cm^{-1}$.

b) Eine Lösung von 23 mmol Lithiumdiisopropylamid, hergestellt aus 3,2 ml Diisopropylamin und 9,2 ml 2,5 M n-Butyllithium in 170 ml Tetrahydrofuran bei 0° unter Stickstoff, wird auf -78° gekühlt und 4,2 ml Triethylphosphonoacetat tropfenweise hinzugegeben. Nach 30 min wird langsam eine Lösung von 4,1 g

3-[1-(4-Cyanbenzyl)imidazol-5-yl]-1-propionaldehyd in 30 ml Tetrahydrofuran zugegeben. Das Reaktionsgemisch wird 2 h bei -78° gerührt; man lässt es auf Raumtemperatur erwärmen und rührt weitere 15 h, bevor man die Reaktion durch Zugabe überschüssiger gesättigter Ammoniumchloridlösung abbricht. Die wässrige Phase wird abgetrennt und mit Essigsäureethylester (2x50 ml) extrahiert. Die vereinigten organischen Phasen werden über Natriumsulfat getrocknet und eingedampft, wobei man ein gelbes Oel erhält, das mit Ether:Essigsäureethylester 1:1 über 20 g Kieselgel chromatographiert wird und 3,56 g 5-[1-(4-Cyanbenzyl)imidazol-5-yl]-1-pent-2-ensäureethylester ergibt. IR ($CH_2Cl_2$): 2240, 1720 $cm^{-1}$.

Beispiel 34: Eine Lösung von 0,21 g 5-(4-Cyanphenyl)-6-ethoxycarbonylmethyl-5,6,7,8-tetrahydroimidazo[1,5-a]pyridin-Hydrochlorid in 1,2 ml Ethanol und 1,2 ml 1N Natronlauge wird 15 h bei Raumtemperatur gerührt, dann eingedampft, und der Rückstand in Wasser gelöst. Die wässrige Phase wird mit Essigsäureethylester extrahiert, auf pH=2 eingestellt, neutralisiert und eingedampft. Der Rückstand wird mit Tetrahydrofuran verrieben. Die organische Phase wird mit etherischem Chlorwasserstoff behandelt, wobei 0,12 g 5-(4-Cyanphenyl)-6-carboxymethyl-5,6,7,8-tetrahydroimidazo[1,5-a]pyridin-Hydrochlorid, Smp. 209-211°, erhalten werden.

Beispiel 35: Eine Lösung von 0,80 mmol Lithiumdiisopropylamid, hergestellt aus 0,12 ml Diisopropylamin und 0,32 ml 2,5 M n-Butyllithium in 6 ml Tetrahydrofuran bei 0°, wird langsam zu einer Lösung von 0,17 g 5-(4-Cyanphenyl)-5,6,7,8-tetrahydroimidazo[1,5-a]pyridin in 2 ml Tetrahydrofuran bei -78° gegeben. Nach 0,5 h werden 0,1 ml Benzylbromid zugetropft. Das Reaktionsgemisch wird eine weitere h gerührt, dann die Reaktion durch Zugabe von 5 ml Wasser abgebrochen, das Gemisch mit 1N Salzsäure angesäuert und mit 20 ml Ether verdünnt, worauf die Phasen getrennt werden. Die wässrige Phase wird auf pH=7 eingestellt und mit Essigsäureethylester (3x15 ml) extrahiert. Die organischen Extrakte werden über Natriumsulfat getrocknet. Nach Filtrieren und Eindampfen erhält man einen Schaum, der mit 1 molaren Aequivalent etherischem Chlorwasserstoff behandelt wird und 5-Benzyl-5-(4-Cyanphenyl)-5,6,7,8-tetrahydroimidazo[1,5-a]pyridin-Hydrochlorid, Smp. 249-251°, ergibt.

Beispiel 36: 7-(p-Cyanphenyl)-5,6,7,8-tetrahydroimidazo[1,5-a]pyridin-Hydrochlorid, Smp. 253-254°, wird aus 4-(p-Ethoxycarbonylphenyl)pyridin in analoger Weise wie in den Beispielen 4, 8-10 und 7 beschrieben hergestellt.

Beispiel 37: 7-(p-Carbamoylphenyl)-5,6,7,8-tetrahydroimidazo[1,5-a]pyridin-Fumarat, Smp. 193-195°, wird aus 4-(p-Ethoxycarbonylphenyl)pyridin in analoger Weise wie in den Beispielen 4 und 8-10 beschrieben hergestellt.

Beispiel 38: Eine Lösung von 1,65 g 5-(p-Cyanphenyl)-3-ethoxycarbonyl-5,6,7,8-tetrahydroimidazo[1,5-a]pyridin in 10 ml Methanol, das 0,2 g Natriumhydroxid enthält, wird 3 h bei Raumtemperatur gerührt. Die Lösung wird zum Sieden erwärmt und 5 ml 1N Salzsäure zugegeben. Nach 1 h wird das Reaktionsgemisch abgekühlt und eingedampft. Der Rückstand wird zwischen Wasser und Essigsäureethylester verteilt. Die organische Phase wird abgetrennt, über Natriumsulfat getrocknet und eingedampft, wobei man 5-(p-Cyanphenyl)-5,6,7,8-tetrahydroimidazo[1,5-a]pyridin, Smp. 128-131°, erhält.

Herstellung der Ausgangsverbindungen:

a) Eine Lösung von 2,0 g 2-Aminomethyl-6-(p-cyanphenyl)pyridin in 20 ml Methylenchlorid wird unter Stickstoff bei -15° mit 1,4 g Oxalsäuremonoethylesterchlorid behandelt. Man lässt das Reaktionsgemisch innerhalb von 2 h auf Raumtemperatur erwärmen und verdampft das Lösungsmittel. Der Rückstand wird in 30 ml Phosphoroxychlorid gelöst, das Gemisch 15 h unter Rückfluss gekocht und dann zur Trockene eingedampft. Der Rückstand wird zwischen Methylenchlorid und einer Natriumhydrogencarbonatlösung verteilt. Die organische Phase wird abgetrennt, über Natriumsulfat getrocknet und eingedampft, wobei man ein Oel erhält, das mit Essigsäureethylester als Elutionsmittel über 100 g Kieselgel chromatographiert wird und 5-(p-Cyanphenyl)-3-ethoxycarbonyl-imidazo[1,5-a]pyridin ergibt.

b) Eine Lösung von 1,1 g 5-(p-Cyanphenyl)-3-ethoxycarbonyl-imidazo[1,5-a]pyridin in 30 ml Ethanol wird mit 0,1 g 10% Palladium-auf-Kohle 2 h bei einem Druck von 1 bar hydriert, filtriert und zur Trockene eingedampft. Das erhaltene Oel wird zwischen Wasser und Essigsäureethylester verteilt. Die organische Phase wird abgetrennt, über Natriumsulfat getrocknet und eingedampft. Der Rückstand wird mit Essigsäureethylester über 40 g Kieselgel chromatographiert, und man erhält 5-(p-Cyanphenyl)-3-ethoxy carbonyl-5,6,7,8-tetrahydroimidazo[1,5-a]pyridin.

Beispiel 39: Eine Lösung von 0,24 g 1-(p-Cyanphenyl)-4-(4-imid-azolyl)-1-butanon in 20 ml Methanol bei Raumtemperatur wird mit 0,2 g Natriumcyanoborhydrid behandelt. Der pH-Wert wird durch Zugabe von konzentrierter Salzsäure auf 5,5-6,0 eingestellt und in diesem Bereich gehalten. Das Reaktionsgemisch wird 2 h gerührt, auf pH=2 eingestellt und zur Trockene eingedampft. Der Rückstand wird in Methylenchlorid

aufgenommen und mit gesättigter Natriumhydrogencarbonatlösung gewaschen. Die organische Phase wird über Natriumsulfat getrocknet und eingedampft, wobei man 5-(p-Cyanphenyl)-5,6,7,8-tetrahydroimidazo[1,5-a]pyridin erhält.

Herstellung der Ausgangsverbindungen:

a) 6,95 g N-tert-Butyl-p-brombenzamid werden in 175 ml Tetrahydrofuran bei -70° unter Stickstoff gelöst und 20,1 ml n-Butyl-lithium (2,7M) tropfenweise zugegeben. Nach 30 min wird langsam eine Lösung von 5,35 g 4-(1-Trityl-4-imidazolyl)-butansäure in 10 ml Tetrahydrofuran hinzugegeben. Man lässt das Reaktionsgemisch langsam auf Raumtemperatur erwärmen und fügt 20 ml einer Ammoniumchloridlösung hinzu. Die organische Phase wird abgetrennt, über Natriumsulfat getrocknet und eingedampft, wobei man 1-(p-N-tert-Butylaminocarbonylphenyl)-4-(1-trityl-4-imidazolyl)-1-butanon erhält.

b) Eine Lösung von 0,5 g 1-(p-N-tert-Butylaminocarbonylphenyl)-4-(1-trityl-4-imidazolyl)-1-butanon in 20 ml Thionylchlorid wird 3 h unter Rückfluss gekocht, dann in 100 ml Eiswasser gegossen. Die wässrige Phase wird mit Ether (3x20 ml) extrahiert, auf pH=10 eingestellt und mit Methylenchlorid erneut extrahiert. Die organische Phase wird getrocknet und eingedampft, wobei man 1-(p-Cyanphenyl)-4-(4-imidazolyl)-1-butanon erhält.

Beispiel 40: Racemisches 5-(p-Cyanphenyl)-5-6,7,8-tetrahydroimidazo[1,5-a]pyridin-Hydrochlorid wird in 20 mg-Portionen auf einer 4,6x250 mm Säule, die mit $\beta$-Cyclodextrin-gebundenem Kieselgel beschickt ist, chromatographiert, wobei ein 7:3 Wasser:Methanol-Gemisch als Elutionsmittel bei einer Tropfgeschwindigkeit von 0,8 ml/min verwendet wird. Getrennt werden die Fraktionen unter Vakuum eingedampft, wobei man (-)-5-(p-Cyanphenyl)-5,6,7,8-tetrahydroimidazo[1,5-a]pyridin, $[\alpha]_n^{25}$ = -89,2°, und (+)-5-(p-Cyanphenyl)-5,6,7,8-tetrahydroimidazo[1,5-a]pyridin, $[\alpha]_n^{25}$ = +85,02°, erhält. Beide Verbindungen werden separat in Aceton gelöst und jeweils mit 1 molaren Aequivalent etherischem Chlorwasserstoff behandelt, wobei man die entsprechenden Hydrochloride vom Smp. 82-83° (amorph) bzw. Smp. 218-220° erhält.

Beispiel 41: In analoger Weise wie in den vorhergehenden Beispielen beschrieben können auch die folgenden Verbindungen hergestellt werden:

5-(m-Cyanphenyl)-5-6,7,8-tetrahydroimidazo[1,5-a]pyridin,
5-(o-Cyanphenyl)-5,6,7,8-tetrahydroimidazo[1,5-a]pyridin,
5H-5-( 3-Cyanphenyl)-6,7-dihydropyrrolo[1,2-c]imidazol,
5H-5-(2-Cyanphenyl)-6,7-dihydropyrrolo[1,2-c]imidazol,
5-(m-Cyanphenyl)imidazo[1,5-a]pyridin,
5-(o-Cyanphenyl)imidazo[1,5-a]pyridin,
6-(p-Cyanphenyl)-5,6,7,8-tetrahydroimidazo[1,5-a]pyridin,
8-(p-Cyanphenyl)-5,6,7,8-tetrahydroimidazo[1,5-a]pyridin.

## Ansprüche

**1.** Verwendung von substituierten Imidazo[1,5-a]pyridin-Derivaten der Formel I

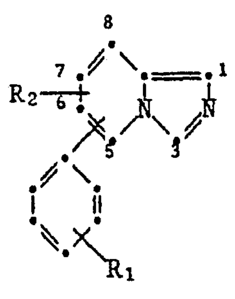

(I),

worin $R_1$ Wasserstoff, Niederalkyl; Niederalkyl, das durch Hydroxy, Niederalkoxy, Niederalkanoyloxy, Niederalkanoyl, Amino, Niederalkylamino, Diniederalkylamino, Halogen, Sulfo, Carboxy, Niederalkoxycarbonyl, Carbamoyl oder Cyan substituiert ist; Nitro, Halogen, Hydroxy, Niederalkoxy, Niederalkanoyloxy, Phenylsulfonyloxy, Niederalkylsulfonyloxy, Mercapto, Niederalkylthio, Niederalkylsulfinyl, Niederalkylsulfonyl, Niederalkanoylthio, Amino, Niederalkylamino, Diniederalkylamino, Niederalkylenamino, N-Morpholino, N-Thiomorpholino, gegebenenfalls in 4-Stellung Niederalkyl-substituiertes N-Piperazino, Triniederalkylammonio, Sulfo, Niederalkoxysulfonyl, Sulfamoyl, Niederalkylsulfamoyl, Diniederalkylsulfamoyl, Formyl; Iminomethyl, das gegebenenfalls am Stickstoff durch Hydroxy, Niederalkoxy, Niederalkanoyloxy, Niederalkyl, Phenyl oder Amino substituiert ist; $C_2$-$C_7$-Alkanoyl, Benzoyl, Carboxy, Niederalkoxycarbonyl, Carbamoyl, Niederalkylcarbamoyl, Diniederalkylcarbamoyl, Cyan, 5-Tetrazolyl, gegebenenfalls Niederalkyl-substituiertes 4,5-Dihydro-2-oxazolyl oder Hydroxycarbamoyl bedeutet; und $R_2$ für Wasserstoff, Niederalkyl, Phenyl-niederalkyl, Carboxy-niederalkyl, Niederalkoxycarbonyl-niederalkyl, Halogen, Hydroxy, Niederalkoxy, Niederalkanoyloxy, Mercapto, Niederalkylthio, Phenyl-niederalkylthio, Phenylthio, Niederalkanoylthio, Carboxy, Niederalkoxycarbonyl oder Niederalkanoyl steht; den 7,8-Dihydroderivaten davon; oder von Verbindungen der Formel I*

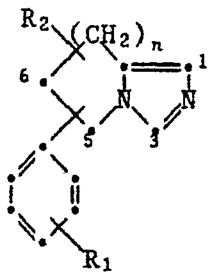

(I*),

worin n für 0, 1, 2, 3 oder 4 steht; und $R_1$ und $R_2$ wie oben unter Formel I definiert sind; wobei der Phenylring in den Resten Phenylsulfonyloxy, Phenyliminomethyl, Benzoyl, Phenyl-niederalkyl, Phenyl-niederalkylthio und Phenylthio unsubstituiert oder durch Niederalkyl, Niederalkoxy oder Halogen substituiert sein kann; wobei in einer Verbindung der Formel I* die beiden Substituenten $C_6H_4$-$R_1$ und $R_2$ an jedem der gesättigten Kohlenstoffatome des gesättigten Rings angeknüpft sein können, entweder beide am selben Kohlenstoffatom oder beide an verschiedenen Kohlenstoffatomen; worin mit "nieder" bezeichnete Reste bis zu 7 Kohlenstoffatome enthalten, Stereoisomeren, Mischungen dieser Stereoisomeren oder pharmazeutisch verwendbaren Salzen davon

2. Verwendung gemäss Anspruch 1, dadurch gekennzeichnet, dass Verbindungen der Formel I, worin $R_1$ Niederalkyl; Niederalkyl, das durch Hydroxy, Amino, Diniederalkylamino, 1-5 Fluoratome, Carboxy, Niederalkoxycarbonyl, Carbamoyl oder Cyan substituiert ist; Nitro, Halogen, Hydroxy, Niederalkoxy, Amino, Niederalkylamino, Diniederalkylamino, Sulfo, Sulfamoyl, Formyl, Iminomethyl; Iminomethyl, das am Stickstoff durch Hydroxy, Niederalkoxy, Niederalkanoyloxy, Niederalkyl oder Phenyl substituiert ist; Carboxy, Niederalkoxycarbonyl, Carbamoyl, Niederalkylcarbamoyl, Diniederalkylcarbamoyl oder Cyan bedeutet; und $R_2$ für Wasserstoff, Niederalkyl, Niederalkoxy oder Halogen steht; oder Verbindungen der Formel I*, worin n für 1, 2 oder 3 steht; $R_1$ wie oben unter Formel I definiert ist und $R_2$ Wasserstoff, Niederalkyl, Phenyl-niederalkyl, Carboxy-niederalkyl, Niederalkoxycarbonyl-niederalkyl, Halogen, Niederalkoxy, Niederalkylthio, Phenyl-niederalkylthio, Phenylthio, Carboxy, Niederalkoxycarbonyl oder Niederalkanoyl bedeutet; wobei in einer Verbindung der Formel I* die beiden Substituenten $C_6H_4$-$R_1$ und $R_2$ an jedem der gesättigten Kohlenstoffatome des gesättigten Rings angeknüpft sein können, entweder beide am selben Kohlenstoffatom oder beide an verschiedenen Kohlenstoffatomen; Stereoisomere, Mischung dieser Stereoisomeren oder pharmazeutisch verwendbare Salze davon wie oben angegeben verwendet werden.

3. Verwendung gemäss Anspruch 1, dadurch gekennzeichnet, dass Verbindungen der Formel I, worin $R_1$ Niederalkyl, Hydroxy-niederalkyl, Halogen, Amino, Formyl, Carboxy, Niederalkoxycarbonyl, Carbamoyl, N-Niederalkylcarbamoyl oder Cyan bedeutet; und $R_2$ Wasserstoff ist; oder Verbindungen der Formel I*, worin n für 1, 2 oder 3 steht; $R_1$ wie oben unter Formel I definiert ist und $R_2$ Wasserstoff, Niederalkylthio, Niederalkoxycarbonyl, Phenyl-niederalkyl, Carboxy-niederalkyl oder Niederalkoxycarbonyl-niederalkyl bedeutet; wobei in einer Verbindung der Formel I* die beiden Substituenten $C_6H_4$-$R_1$ und $R_2$ an jedem der gesättigten Kohlenstoffatome des gesättigten Rings angeknüpft sein können, entweder beide am selben Kohlenstoffatom oder beide an verschiedenen Kohlenstoffatomen; Stereoisomere, Mischungen dieser Stereoisomeren oder pharmazeutisch verwendbare Salze davon verwendet werden.

4. Pharmazeutische Präparate die eine Verbindung der Formel wie in Anspruch 1 wiedergegeben , worin

$R_1$ Halogen, Hydroxy, Mercapto oder Niederalkyl, das durch Niederalkoxy, Niederalkanoyloxy oder Halogen substituiert ist, bedeutet; und $R_2$ für Wasserstoff, Niederalkyl, Niederalkoxy oder Halogen steht; oder eine Verbindung der Formel I* wie in Anspruch 1 wiedergegeben worin n für 2 steht, $R_1$ wie oben unter Formel I definiert ist und $R_2$ Wasserstoff, Niederalkyl, Phenyl-niederalkyl, Carboxy-niederalkyl, Niederalkoxycarbonyl-niederalkyl, Halogen, Niederalkoxy, Niederalkylthio, Phenyl-niederalkylthio, Phenylthio, Carboxy, Niederalkoxycarbonyl oder Niederalkanoyl bedeutet; wobei in einer Verbindung der Formel I* die beiden Substituenten $C_6H_4$-$R_1$ und $R_2$ an jedem der gesättigten Kohlenstoffatome des gesättigten Rings angeknüpft sein können, entweder beide am selben Kohlenstoffatom oder beide an verschiedenen Kohlenstoffatomen;wobei mit "nieder"bezeichnete Reste bis zu 7 Kohlenstoffatome enthalten ein Stereoisomer, eine Mischung dieser Stereoisomeren oder ein pharmazeutisch verwendbares Salz davon zusammen mit einem oder mehreren pharmazeutisch annehmbaren Trägermaterialien enthalten.

5. Verbindungen der Formel I wie in Anspruch 1 niedergegeben worin $R_1$ Wasserstoff, Niederalkyl; $C_2$-$C_7$-Alkyl, das durch Hydroxy, Niederalkoxy, Halogen oder Niederalkanoyloxy substituiert ist; Niederalkyl, das durch Niederalkanoyl, Amino, Niederalkylamino, Diniederalkylamino, Sulfo, Niederalkoxycarbonyl, Carbamoyl oder Cyan substituiert ist; Nitro, Niederalkoxy, Niederalkanoyloxy, Phenylsulfonyloxy, Niederalkylsulfonyloxy, Niederalkylthio, Niederalkylsulfinyl, Niederalkylsulfonyl, Niederalkanoylthio, Amino, Niederalkylamino, Diniederalkylamino, Niederalkylenamino, N-Morpholino, N-Thiomorpholino, gegebenenfalls in 4-Stellung durch Niederalkyl substituiertes N-Piperazino, Triniederalkylammonio, Sulfo, Niederalkoxysulfonyl, Sulfamoyl, Niederalkylsulfamoyl, Diniederalkylsulfamoyl; Iminomethyl, das gegebenenfalls am Stickstoff durch Hydroxy, Niederalkoxy, Niederalkanoyloxy, Niederalkyl, Phenyl oder Amino substituiert ist; $C_2$-$C_7$-Alkanoyl oder Benzoyl bedeutet, und $R_2$ für Wasserstoff, Niederalkyl, Phenyl-niederalkyl, Carboxy-niederalkyl, Niederalkoxycarbonyl-niederalkyl, Halogen, Hydroxy, Niederalkoxy, Niederalkanoyloxy, Mercapto, Niederalkylthio, Phenyl-niederalkylthio, Phenylthio, Niederalkanoylthio, Carboxy, Niederalkoxycarbonyl oder Niederalkanoyl steht; die 7,8-Dihydroderivate davon und ferner solche 7,8-Dihydroderivate, worin $R_1$ Hydroxymethyl, Niederalkoxymethyl, Halogenmethyl, Niederalkanoyloxymethyl, Carboxy-neideralkyl, Halogen, Hydroxy, Mercapto, Formyl, Carboxy, Niederalkoxycarbonyl, Carbamoyl, Niederalkylcarbamoyl, Diniederalkylcarbamoyl, Cyan, 5-Tetrazolyl, gegegebenenfalls durch Niederalkyl substituiertes 4,5-Dihydro-2-oxazolyl oder Hydroxycarbamoyl bedeutet und $R_2$ wie oben unter Formel I definiert ist; und Verbindungen der Formel I* wie in Anspruch 1 wiedergegeben, worin n für 0, 1, 2, 3 oder 4 steht; $R_1$ wie oben unter Formel I definiert ist oder $R_1$ zusätzlich Hydroxymethyl, Niederalkoxymethyl, Halogenmethyl, Niederalkanoyloxymethyl, Carboxy-neideralkyl Halogen, Hydroxy, Mercapto, Formyl, Carboxy, Niederalkoxycarbonyl, Carbamoyl, Niederalkylcarbamoyl, Diniederalkylcarbamoyl, Cyan, 5-Tetrazolyl, gegebenenfalls durch Niederalkyl substituiertes 4,5-Dihydro-2-oxazolyl oder Hydroxycarbamoyl bedeuten kann, wenn n für 0, 1, 3 oder 4 steht oder wenn n für 2 steht und $R_2$ Phenyl-niederalkyl, Carboxy-niederalkyl, Niederalkoxycarbonyl-niederalkyl, Niederalkanoyloxy, Mercapto, Niederalkylthio, Phenyl-niederalkylthio, Phenylthio, Niederalkanoylthio, Carboxy, Niederalkoxycarbonyl oder Niederalkanoyl bedeutet; und $R_2$ wie oben unter Formel I definiert ist; wobei der Phenylring in den Resten Phenylsulfonyloxy, Phenyliminomethyl, Benzoyl, Phenyl-niederalkyl, Phenyl-niederalkylthio und Phenylthio unsubstituiert oder durch Niederalkyl, Niederalkoxy oder Halogen substituiert sein kann; wobei in einer Verbindung der Formel I* die beiden Substituenten $C_6H_4$-$R_1$ und $R_2$ an jedem der gesättigten Kohlenstoffatome des gesättigten Rings angeknüpft sein können, entweder beide am selben Kohlenstoffatom oder beide an verschiedenen Kohlenstoffatomen; wobei mit "neider" bezeichnete Reste bis zu 7 Kohlenstoffe enthalten; Stereoisomere, Mischungen dieser Stereoisomeren und Salze davon.

6. Verbindungen gemäss Anspruch 5 der Formel I, worin $R_1$ Niederalkyl, Hydroxy-$C_2$-$C_7$-alkyl; Niederalkyl, das durch Amino, Diniederalkylamino, 2 bis 5 Fluoratome, Niederalkoxycarbonyl, Carbamoyl oder Cyan substituiert ist; Nitro, Niederalkoxy, Amino, Niederalkylamino, Diniederalkylamino, Sulfo, Sulfamoyl, Iminomethyl oder Iminomethyl, das am Stickstoff durch Hydroxy, Niederalkoxy, Niederalkanoyloxy, Niederalkyl oder Phenyl substituiert ist, bedeutet und $R_2$ für Wasserstoff, Niederalkyl, Niederalkoxy oder Halogen steht; und Verbindungen der Formel I* worin n für 1, 2 oder 3 steht; $R_1$ wie oben unter Formel I definiert ist oder $R_1$ zusatzlich Hydroxymethyl, Carboxy-niederalkyl, Halogen, Hydroxy, Formyl, Carboxy, Niederalkoxycarbonyl, Carbamoyl, Niederalkylcarbamoyl, Diniederalkylcarbamoyl oder Cyan bedeuten kann, wenn n für 1 oder 3 steht oder wenn n für 2 steht und $R_2$ Phenylniederalkyl, Carboxy-niederalkyl, Niederalkoxycarbonyl-niederalkyl, Niederalkylthio, Phenylniederalkylthio, Phenylthio, Carboxy, Niederalkoxycarbonyl oder Niederalkanoyl bedeutet ; und $R_2$ Wasserstoff,

Niederalkyl, Phenyl-niederalkyl, Carboxy-niederalkyl, Niederalkoxycarbonyl-niederalkyl, Halogen, Niederalkoxy, Niederalkylthio, Phenyl-niederalkylthio, Phenylthio, Carboxy, Niederalkoxycarbonyl oder Niederalkanoyl bedeutet; wobei in einer Verbindung der Formel I* die beiden Substituenten $C_6H_4$-$R_1$ und $R_2$ an jedem der gesättigten Kohlenstoffatome des gesättigten Rings angeknüpft sein können, entweder beide am selben Kohlenstoffatom oder beide an verschiedenen Kohlenstoffatomen; Stereoisomere, Mischungen dieser Stereoisomeren und pharmazeutisch verwendbare Salze davon.

7. Verbindungen gemäss Anspruch 5 der Formel I*, worin n für 1, 2 oder 3 steht; $R_1$ Niederalkyl, Amino, Niederalkylamino oder Diniederalkylamino bedeutet oder $R_1$ zusätzlich Hydroxymethyl, Halogen, Formyl, Carboxy, Niederalkoxycarbonyl, Carbamoyl, Niederalkylcarbamoyl, Diniederalkylcarbamoyl oder Cyan bedeuten kann, wenn n für 1 oder 3 steht oder wenn n für 2 steht und $R_2$ Phenyl-niederalkyl, Carboxy-niederalkyl, Niederalkoxycarbonyl-niederalkyl, Niederalkylthio, Carboxy oder Niederalkoxycarbonyl bedeutet; und $R_2$ Wasserstoff, Niederalkyl, Phenyl-niederalkyl, Carboxy-niederalkyl, Niederalkoxycarbonyl-niederalkyl, Niederalkylthio, Carboxy oder Niederalkoxycarbonyl bedeutet; wobei die beiden Substituenten $C_6H_4$-$R_1$ und $R_2$ an jedem der gesättigten Kohlenstoffatome des gesättigten Rings angeknüpft sein können, entweder beide am selben Kohlenstoffatom oder beide an verschiedenen Kohlenstoffatomen; Stereoisomere, Mischungen dieser Stereoisomeren und pharmazeutisch verwendbare Salze davon.

8. Verbindungen der Formel Ia

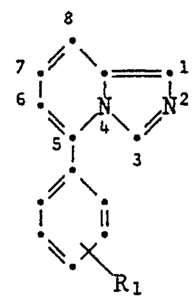

(Ia)

worin $R_1$ Cyan, Nitro oder $C_1$-$C_4$-Alkyl bedeutet, die 7,8-Dihydroderivate davon und die. 5,6,7,8-Tetrahydroderivate davon mit der Formel Ib

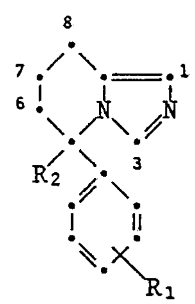

(Ib)

worin $R_1$ wie oben unter Formel Ia definiert ist und $R_2$ Wasserstoff, $C_1$-$C_4$-Alkyl, Benzyl, Halogen, $C_1$-$C_4$-Alkoxy, $C_1$-$C_4$-Alkylthio, Benzylthio, 2-Phenylethylthio, Diphenylmethylthio, Phenylthio, $C_1$-$C_4$-Alkanoyloxy, $C_1$-$C_4$-Alkanoylthio, Carboxy-$C_1$-$C_4$-alkyl, $C_1$-$C_4$-Alkoxycarbonyl-$C_1$-$C_4$-alkyl oder $C_1$-$C_4$-Alkanoyl bedeutet, Stereoisomere, Mischungen dieser Stereoisomeren und Salze dieser Verbindungen.

9. Verbindungen gemäss Anspruch 8 der Formel Ia, worin $R_1$ Cyan bedeutet, die 7,8-Dihydroderivate davon, und die 5,6,7,8-Tetrahydroderivate davon mit der Formel Ib, worin $R_1$ Cyan bedeutet und $R_2$für Wasserstoff, $C_1$-$C_4$-Alkyl, $C_1$-$C_4$-Alkoxy, $C_1$-$C_4$-Alkylthio, Benzylthio, 2-Phenylethylthio, Diphenylmethylthio, Phenylthio oder $C_1$-$C_4$-Alkanoyl steht und pharmazeutisch verwendbare Säureadditionssalze von Verbindungen der Formel Ia und Ib.

10. Verbindungen gemäss Anspruch 8 der Formel Ia, worin $R_1$ Cyan bedeutet, die 7,8-Dihydroderivate

davon und die 5,6,7,8-Tetrahydroderivate davon mit der Formel Ib, worin $R_1$ wie oben unter Formel Ia definiert ist und $R_2$ für Wasserstoff steht, und pharmazeutisch verwendbare Säureadditionssalze davon.

**11.** Verbindungen gemäss Anspruch 8 der Formel Ic

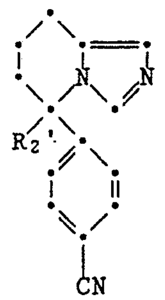

(Ic)

worin $R_2$' Wasserstoff, $C_1$-$C_4$-Alkyl, $C_1$-$C_4$-Alkoxy, $C_1$-$C_4$-Alkylthio, Benzylthio, 2-Phenylethylthio, Diphenylmethylthio, Phenylthio oder $C_1$-$C_4$-Alkanoyl bedeutet, und pharmazeutisch verwendbare Säureadditionssalze davon.

**12.** Eine Verbindung der Ic Formel gemäss Anspruch 11, worin $R_2$' Wasserstoff bedeutet, mit dem Namen 5-(p-Cyanphenyl)-5,6,7,8-tetrahydroimidazo[1,5-a]pyridin, und pharmazeutisch verwendbare Säureadditionssalze davon.

**13.** Verbindungen der Formel Ia wie in Anspruch 8 wiedergegeben, worin $R_1$ Wasserstoff, p-Toluolsulfonyloxy, Benzolsulfonyloxy, Methylsulfonyloxy, Sulfo, Amino, Carbamoyl, $C_1$-$C_4$-Alkylcarbamoyl oder Hydroxyiminomethyl bedeutet; und die 5,6,7,8-Tetrahydroverbindungen der Formel Ib wie in Anspruch 8 wiedergegeben, worin $R_1$wie oben unter Formel Ia definiert ist oder Halogen bedeutet und $R_2$ für Wasserstoff, $C_1$-$C_4$-Alkyl, Benzyl, Halogen, $C_1$-$C_4$-Alkoxy, $C_1$-$C_4$-Alkanoyloxy, $C_1$-$C_4$-Alkylthio, Benzylthio, 2-Phenylethylthio, Diphenylmethylthio, Phenylthio, $C_1$-$C_4$-Alkanoylthio, Carboxy-$C_1$-$C_4$-alkyl, $C_1$-$C_4$-Alkoxycarbonyl-$C_1$-$C_4$-alkyl oder $C_1$-$C_4$-Alkanoyl steht; und pharmazeutisch verwendbare Salze davon.

**14.** Verbindungen der Formel Ia und Ib gemäss Anspruch 13, worin $R_1$ Carbamoyl bedeutet, und Verbindungen der Formel Ib, worin $R_1$ Halogen bedeutet.

**15.** Verbindungen gemäss Anspruch 13 der Formel Ia, worin $R_1$ Wasserstoff, p-Toluolsulfonyloxy, Benzolsulfonyloxy, Methylsulfonyloxy, Sulfo, Amino oder Hydroxyiminomethyl bedeutet; und die 5,6, 7,8-Tetrahydroverbindungen der Formel Ib, worin $R_1$ wie oben unter Formel Ia angegeben definiert ist und $R_2$ für Wasserstoff steht; oder worin $R_1$ Wasserstoff, Halogen, p-Toluolsulfonyloxy, Benzolsulfonyloxy, Methylsulfonyloxy, Sulfo, Amino, Carboxy, Carbamoyl, $C_1$-$C_4$-Alkylcarbamoyl, Formyl oder Hydroxyiminomethyl bedeutet und $R_2$ für $C_1$-$C_4$-Alkyl, Benzyl, Halogen, $C_1$-$C_4$-Alkoxy, $C_1$-$C_4$-Alkanoyloxy, $C_1$-$C_4$-Alkylthio, Benzylthio, 2-Phenylethylthio, Diphenylmethylthio, Phenylthio, $C_1$-$C_4$-Alkanoylthio, Carboxy-$C_1$-$C_4$-alkyl, $C_1$-$C_4$-Alkoxycarbonyl-$C_1$-$C_4$-alkyl oder $C_1$-$C_4$-Alkanoyl steht; und pharmazeutisch verwendbare Salze davon.

**16.** (-)-(5-(p-Cyanphenyl)-5,6,7,8-tetrahydroimidazo[1,5-a]pyridin gemäss Anspruch 8 und pharmazeutisch verwendbare Salze davon.

**17.** (+)-(5-(p-Cyanphenyl)-5,6,7,8-tetrahydroimidazo[1,5-a]pyridin gemäss Anspruch 8 und pharmazeutisch verwendbare Salze davon.

**18.** 5-(p-Cyanphenyl)-7,8-dihydroimidazo[1,5-a]pyridin gemäss Anspruch 8 und pharmazeutisch verwendbare Salze davon.

**19.** 5-(p-Cyanphenyl)imidazo[1,5-a]pyridin gemäss Anspruch 8 und pharmazeutisch verwendbare Salze davon.

**20.** 5-(p-Bromphenyl)-5,6,7,8-tetrahydroimidazo[1,5-a]pyridin gemäss Anspruch 13, ein Stereoisomeres davon, Mischungen dieser Stereoisomeren und pharmazeutisch verwendbare Salze davon.

**21.** 5H-5-(4-Cyanphenyl)-6,7-dihydropyrrolo[1,2-c]imidazol gemäss Anspruch 5, ein Stereoisomeres davon, Mischungen dieser Stereoisomeren und pharmazeutisch verwendbare Salze davon.

**22.** 5H-5-(4-Cyanphenyl)-6,7,8,9-tetrahydroimidazo[1,5-a]azepin gemäss Anspruch 5, ein Stereoisomeres davon, Mischungen dieser Stereoisomeren und pharmazeutisch verwendbare Salze davon.

**23.** 5-(4-Cyanphenyl)-6-ethoxycarbonylmethyl-5,6,7,8-tetrahydroimidazo[1,5-a]pyridin gemäss Anspruch 5, ein Stereoisomeres davon, Mischungen dieser Stereoisomeren und pharmazeutisch verwendbare Salze davon.

**24.** 5-(4-Cyanphenyl)-6-carboxymethyl-5,6,7,8-tetrahydroimidazo[1,5-a]pyridin gemäss Anspruch 5, ein Stereoisomeres davon, Mischungen dieser Stereoisomeren und pharmazeutisch verwendbare Salze davon.

**25.** 5-Benzyl-5-(4-cyanphenyl)-5,6,7,8-tetrahydroimidazo[1,5-a]pyridin gemäss Anspruch 8, ein Stereoisomeres davon, Mischungen dieser Stereoisomeren und pharmazeutisch verwendbare Salze davon.

**26.** 7-(p-Cyanphenyl)-5,6,7,8-tetrahydroimidazo[1,5-a]pyridin, ein Stereoisomeres davon, Mischungen dieser Stereoisomeren und pharmazeutisch verwendbare Salze davon.

**27.** Pharmazeutische Präparate enthaltend eine Verbindung gemäss Anspruch 5 zusammen mit einem oder mehreren pharmazeutisch annehmbaren Trägermaterialien.

**28.** Pharmazeutische Präparate enthaltend eine Verbindung gemäss Anspruch 8 zusammen mit einem oder mehreren pharmazeutisch annehmbaren Trägermaterialien.

**29.** Pharmazeutische Präparate enthaltend eine Verbindung gemäss Anspruch 12 zusammen mit einem oder mehreren pharmazeutisch annehmbaren Trägermaterialien.

**30.** Pharmazeutische Präparate enthaltend eine Verbindung gemäss Anspruch 13 zusammen mit einem oder mehreren pharmazeutisch annehmbaren Trägermaterialien.

**31.** Pharmazeutische Präparate enthaltend eine Verbindung gemäss Anspruch 16 zusammen mit einem oder mehreren pharmazeutisch annehmbaren Trägermaterialien.

**32.** Pharmazeutische Präparate enthaltend eine Verbindung gemäss Anspruch 17 zusammen mit einem oder mehreren pharmazeutisch annehmbaren Trägermaterialien.

**33.** Eine Verbindung gemäss Anspruch 5 zur Anwendung in einem Verfahren zur therapeutischen Behandlung des tierischen oder menschlichen Körpers.

**34.** Eine Verbindung gemäss Anspruch 8 zur Anwendung in einem Verfahren zur therapeutischen Behandlung des tierischen oder menschlichen Körpers.

**35.** Eine Verbindung gemäss Anspruch 12 zur Anwendung in einem Verfahren zur therapeutischen Behandlung des tierischen oder menschlichen Körpers.

**36.** Eine Verbindung gemäss Anspruch 13 zur Anwendung in einem Verfahren zur therapeutischen Behandlung des tierischen oder menschlichen Körpers.

**37.** Eine Verbindung gemäss Anspruch 16 zur Anwendung in einem Verfahren zur therapeutischen Behandlung des tierischen oder menschlichen Körpers.

**38.** Eine Verbindung gemäss Anspruch 17 zur Anwendung in einem Verfahren zur therapeutischen Behandlung des tierischen oder menschlichen Körpers.

**39.** Eine Verbindung gemäss Anspruch 5 zur Verwendung als Aromatasehemmer.

**40.** Eine Verbindung gemäss Anspruch 8 zur Verwendung als Aromatasehemmer.

**41.** Eine Verbindung gemäss Anspruch 12 zur Verwendung als Aromatasehemmer.

**42.** Eine Verbindung gemäss Anspruch 16 zur Verwendung als Aromatasehemmer.

**43.** Verwendung von einer Verbindung gemäss Anspruch 5 zur Herstellung von pharmazeutischen Präparaten.

**44.** Verwendung von einer Verbindung gemäss Anspruch 8 zur Herstellung von pharmazeutischen Präparaten.

**45.** Verwendung von einer Verbindung gemäss Anspruch 12 zur Herstellung von pharmazeutischen Präparaten.

**46.** Verwendung von einer Verbindung gemäss Anspruch 16 zur Herstellung von pharmazeutischen Präparaten.

**47.** Verwendung von einer Verbindung gemäss Anspruch 17 zur Herstellung von pharmazeutischen Präparaten.

**48.** Verwendung von einer Verbindung gemäss Anspruch 5 zur Herstellung von pharmazeutischen Präparaten für die Behandlung von Krankheiten, die auf eine Hemmung der Aromatase ansprechen.

**49.** Verwendung von einer Verbindung gemäss Anspruch 8 zur Herstellung von pharmazeutischen Präparaten für die Behandlung von Krankheiten, die auf eine Hemmung der Aromatase ansprechen.

**50.** Verwendung von einer Verbindung gemäss Anspruch 12 zur Herstellung von pharmazeutischen Präparaten für die Behandlung von Krankheiten, die auf eine Hemmung der Aromatase ansprechen.

**51.** Verwendung von einer Verbindung gemäss Anspruch 13 zur Herstellung von pharmazeutischen Präparaten für die Behandlung von Krankheiten, die auf eine Hemmung der Aromatase ansprechen.

**52.** Verwendung von einer Verbindung gemäss Anspruch 16 zur Herstellung von pharmazeutischen Präparaten für die Behandlung von Krankheiten, die auf eine Hemmung der Aromatase ansprechen.

**53.** Verfahren zur Herstellung von Verbindungen der Formel I oder I* gemäss Anspruch 5, oder Salzen davon, dadurch gekennzeichnet, dass man

a) eine Verbindung der Formel II

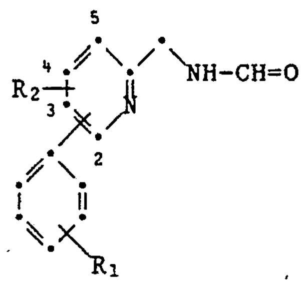

(II)

oder ein 4,5-Dihydroderivat davon, ringschliesst, um eine Verbindung der Formel I bzw. ein 7,8-Dihydroderivat davon zu erhalten, oder

b) eine Verbindung der Formel III

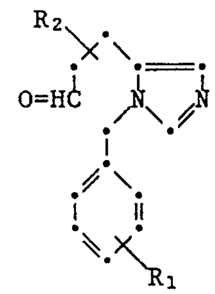

(III),

worin $R_2$ wie angedeutet an jedem der Kohlenstoffatome einschliesslich des Carbonylkohlenstoffs angeknüpft sein kann, ringschliesst, um ein 7,8-Dihydroderivat einer Verbindung der Formel I zu erhalten, worin der Substituent $C_6H_4$-$R_1$ in 5-Stellung angeknüpft ist, oder
c)/f) in einer Verbindung der Formel IV oder VII

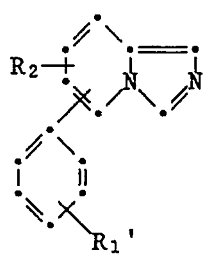

(IV)

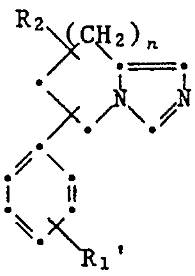

(VII)

oder in einem 7,8-Dihydroderivat der Formel IV, worin $R_1'$ jeweils eine Gruppe bedeutet, die in Cyan umgewandelt werden kann, $R_1'$ in Cyan umwandelt, um eine Verbindung der Formel I, ein 7,8-Dihydroderivat davon bzw. eine Verbindung der Formel I* zu erhalten, worin $R_1$ Cyan bedeutet, oder
d) eine Verbindung der Formel V

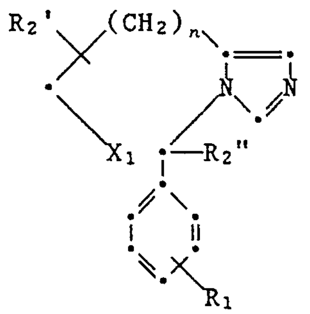

(V),

worin wenigstens einer der Reste $R_2'$ und $R_2''$ Wasserstoff und der andere einen Rest $R_2$ wie unter Formel I* definiert bedeutet, und $X_1$ eine Abgangsgruppe bedeutet, und $R_2'$ an jedem der Kohlenstoffatome wie angedeutet angeknüpft sein kann, ringschliesst, um eine Verbindung der Formel I* zu erhalten, worin der Substituent $C_6H_4$-$R_1$ in 5-Stellung angeknüpft ist; oder $X_1$ eine Gruppe =CH-COOH oder einen Niederalkylester davon bedeutet, $R_2'$ für Wasserstoff steht und $R_2''$ wie unter Formel I* definiert ist, ringschliesst, um eine Verbindung der Formel I* zu erhalten, worin der Substituent $C_6H_4$-$R_1$ in 5-Stellung angeknüpft ist und die 6-Stellung durch Carboxymethyl oder Niederalkoxycarbonylmethyl substituiert ist, oder
e) eine Verbindung der Formel VI

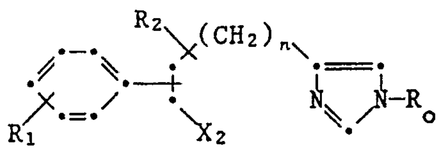

(VI)

worin die Substituenten $C_6H_4$-$R_1$ und $R_2$ an jedem der Kohlenstoffatome wie angedeutet angeknüpft sein können, entweder beide Reste am selben Kohlenstoffatom oder beide an verschiedenen Kohlenstoffatomen, $R_0$ eine NH-Schutzgruppe oder Wasserstoff bedeutet und $X_2$ eine Abgangsgruppe bedeutet, ringschliesst, um eine Verbindung der Formel I* zu erhalten; oder

g) eine Verbindung der Formel IX

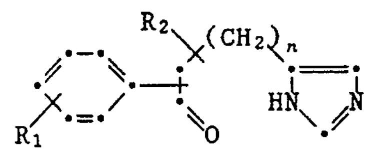

(IX)

worin die Substituenten $C_6H_4$-$R_1$ und $R_2$ an jedem der Kohlenstoffatome einschliesslich dem Carbonylkohlenstoff wie angedeutet angeknüpft sein können, entweder beide Reste am selben Kohlenstoffatom oder beide an verschiedenen Kohlenstoffatomen, gegebenenfalls unter reduktiven Bedingungen, ringschliesst, um ein 7,8-Dihydroderivat einer Verbindung der Formel I oder, im Falle von reduktiven Bedingungen, eine Verbindung der Formel I* erhält, oder

h) eine Verbindung analog zu Formel I, oder ein 7,8-Dihydroderivat davon, oder eine Verbindung analog zu Formel I*, welche jeweils eine zusätzliche Carboxygruppe in 1- oder 3-Stellung enthält, decarboxyliert, um eine Verbindung der Formel I, ein 7,8-Dihydroderivat davon oder eine Verbindung der Formel I* zu erhalten; wobei in den Ausgangsverbindungen der Formel II bis VII und IX die Symbole n, $R_1$ und $R_2$ die unter Formel I bzw. I* angegebenen Bedeutungen haben; und/oder eine Verbindung der Formel I, oder ein 7,8-Dihydroderivat davon, zu einem entsprechenden 5,6,7,8-Tetrahydroderivat der Formel I* reduziert, wobei gegebenenfalls gleichzeitig eine Reduktion des Substituenten $R_1$ und/oder $R_2$ in einen anderen Substituenten $R_1$ und/oder $R_2$ stattfindet; und/oder eine Verbindung der Formel I*, worin $R_2$ Carboxy bedeutet, decarboxyliert, um eine Verbindung der Formel I* zu erhalten, worin $R_2$ Wasserstoff ist; und/oder eine erhaltene Verbindung in eine andere Verbindung der Erfindung umwandelt und/oder ein erhaltenes Salz in die freie Verbindung oder in ein anderes Salz umwandelt und/oder eine erhaltene freie Verbindung in ein Salz umwandelt und/oder ein erhaltenes Gemisch von Isomeren oder Racematen in die einzelnen Isomeren oder Racematen auftrennt und/oder ein enantiomeren Gemisch, etwa ein Racemat, in die optischen Antipoden aufspaltet.

**54.** Verfahren zur Herstellung von Verbindungen der Formel Ia oder Ib gemäss Anspruch 8, oder Salzen davon, dadurch gekennzeichnet, dass man

a) eine Verbindung der Formel IIa

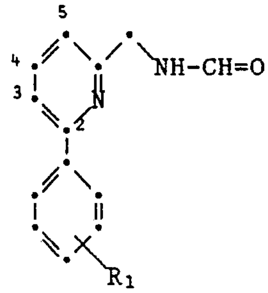

(IIa),

worin $R_1$ wie oben unter Formel Ia definiert ist, oder ein 4,5-Dihydroderivat davon, unter sauren Bedingungen cyclisiert, um eine Verbindung der Formel Ia oder ein 7,8-Dihydroderivat davon zu erhalten, oder

b) zur Herstellung eines 7,8-Dihydroderivates einer Verbindung der Formel Ia, eine Verbindung der Formel IIIa

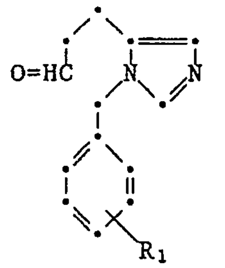

(IIIa),

worin $R_1$ wie oben unter Formel Ia definiert ist, unter basischen Bedingungen cyclisiert, oder
c) in einer Verbindung der Formel IVa

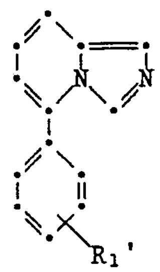

(IVa),

worin $R_1$' einen Rest bedeutet, der in Cyan umgewandelt werden kann, oder in einem 7,8-Dihydroderivat davon, $R_1$' in Cyan umwandelt, um eine Verbindung der Formel Ia, oder ein 7,8-Dihydroderivat davon, zu erhalten, oder
d) eine Verbindung der Formel Vb

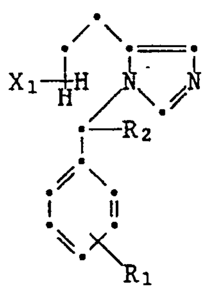

(Vb),

worin $R_1$ und $R_2$ wie oben unter Formel Ib definiert sind und $X_1$ eine Abgangsgruppe bedeutet, in Gegenwart einer Base cyclisiert, um eine Verbindung der Formel Ib zu erhalten, oder
e) eine Verbindung der Formel VIb

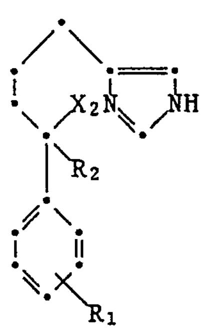

(VIb),

worin $R_1$ und $R_2$ wie oben unter Formel Ib definiert sind und $X_2$ eine Abgangsgruppe bedeutet,

worin die NH-Gruppe durch eine NH-Schutzgruppe geschützt sein kann, in Gegenwart einer Base cyclisiert, um eine Verbindung der Formel Ib zu erhalten, oder

f) in einer Verbindung der Formel VIIb

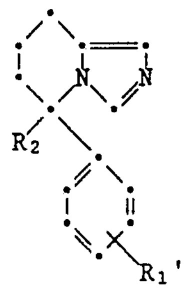

(VIIb),

worin $R_1$' einen Rest bedeutet, der in Cyan umgewandelt werden kann und worin $R_2$ wie oben unter Formel Ib definiert ist, den Rest $R_1$' in Cyan umwandelt, um eine Verbindung der Formel Ib zu erhalten, worin $R_1$ Cyan bedeutet;

g) eine Verbindung der Formel IXb

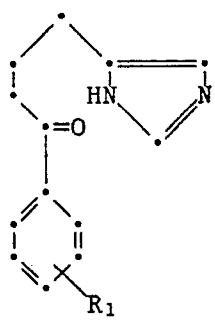

(IXb),

gegebenenfalls unter reduktiven Bedingungen, cyclisiert, um ein 7,8-Dihydroderivat einer Verbindung der Formel Ia oder, im Falle von reduktiven Bedingungen, eine Verbindung der Formel Ib zu erhalten, oder

h) eine Verbindung analog zu Formel Ia, oder ein 7,8-Dihydroderivat davon, oder eine Verbindung analog zu Formel Ib, die jeweils eine zusätzliche Carboxygruppe in 1- oder 3-Stellung enthält, decarboxyliert, um eine Verbindung der Formel Ia, ein 7,8-Dihydroderivat davon oder eine Verbindung der Formel Ib zu erhalten. und/oder eine Verbindung der Formel Ib, worin $R_2$ Carboxy ist, decarboxyliert, um eine andere Verbindung der Formel Ib zu erhalten, worin $R_2$ Wasserstoff bedeutet, und/oder eine Verbindung der Formel Ia, oder ein 7,8-Dihydroderivat davon, mit Wasserstoff in Gegenwart eines Hydrierungskatalysators zum entsprechenden 5,6,7,8-Tetrahydroderivat der Formel Ib reduziert, und/oder eine erhaltene Verbindung in eine andere Verbindung der Erfindung umwandelt und/oder ein erhaltenes Salz in die freie Verbindung oder in ein anderes Salz umwandelt und/oder eine freie Verbindung, die eine salzbildende Gruppe enthält, in ein Salz umwandelt und/oder eine erhaltenes racemisches Gemisch in die individuellen Enantiomere auftrennt.

**55.** Die nach dem Verfahren des Anspruchs 53 erhältlichen Verbindungen.

**56.** Die nach dem Verfahren des Anspruchs 54 erhältlichen Verbindungen.

Patentansprüche für Folgenden Vertragsstaat: AT

**1.** Verfahren zur Herstellung von Verbindungen der Formel I

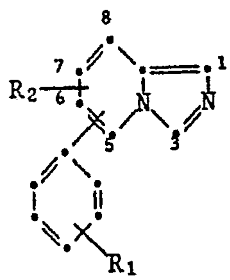

(I)

worin $R_1$ Wasserstoff, Niederalkyl; $C_2$-$C_7$-Alkyl, das durch Hydroxy, Niederalkoxy, Halogen oder Niederalkanoyloxy substituiert ist; Niederalkyl, das durch Niederalkanoyl, Amino, Niederalkylamino, Diniederalkylamino, Sulfo, Niederalkoxycarbonyl, Carbamoyl oder Cyan substituiert ist; Nitro, Niederalkoxy, Niederalkanoyloxy, Phenylsulfonyloxy, Niederalkylsulfonyloxy, Niederalkylthio, Niederalkylsulfinyl, Niederalkylsulfonyl, Niederalkanoylthio, Amino, Niederalkylamino, Diniederalkylamino, Niederalkylenamino, N-Morpholino, N-Thiomorpholino, gegebenenfalls in 4-Stellung durch Niederalkyl substituiertes N-Piperazino, Triniederalkylammonio, Sulfo, Niederalkoxysulfonyl, Sulfamoyl, Niederalkylsulfamoyl, Diniederalkylsulfamoyl: Iminomethyl, das gegebenenfalls am Stickstoff durch Hydroxy, Niederalkoxy, Niederalkanoyloxy, Niederalkyl, Phenyl oder Amino substituiert ist; $C_2$-$C_7$-Alkanoyl oder Benzoyl bedeutet, und $R_2$ für Wasserstoff, Niederalkyl, Phenyl-niederalkyl, Carboxy-niederalkyl, Niederalkoxycarbonyl-niederalkyl, Halogen, Hydroxy, Niederalkoxy, Niederalkanoyloxy, Mercapto, Niederalkylthio, Phenyl-niederalkylthio, Phenylthio, Niederalkanoylthio, Carboxy, Niederalkoxycarbonyl oder Niederalkanoyl steht; den 7,8-Dihydroderivaten davon und ferner solchen 7,8-Dihydroderivaten, worin $R_1$ Hydroxymethyl, Niederalkoxymethyl, Halogenmethyl, Niederalkanoyloxymethyl, Carboxy-niederalkyl,Halogen, Hydroxy, Mercapto, Formyl, Carboxy, Niederalkoxycarbonyl, Carbamoyl, Niederalkylcarbamoyl, Diniederalkylcarbamoyl, Cyan, 5-Tetrazolyl, gegegebenenfalls durch Niederalkyl substituiertes 4,5-Dihydro-2-oxazolyl oder Hydroxycarbamoyl bedeutet und $R_2$ wie oben unter Formel I definiert ist; und Verbindungen der Formel I*

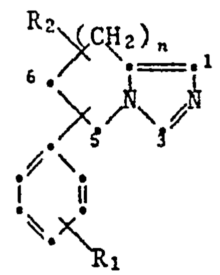

(I*)

worin n für 0, 1, 2, 3 oder 4 steht; $R_1$ wie oben unter Formel I definiert ist oder $R_1$ zusätzlich Hydroxymethyl, Niederalkoxymethyl, Halogenmethyl, Niederalkanoyloxymethyi, Carboxy-niederalkyl, Halogen, Hydroxy, Mercapto, Formyl, Carboxy, Niederalkoxycarbonyl, Carbamoyl, Niederalkylcarbamoyl, Diniederalkylcarbamoyl, Cyan, 5-Tetrazolyl, gegebenenfalls durch Niederalkyl substituiertes 4, 5-Dihydro-2-oxazolyl oder Hydroxycarbamoyl bedeuten kann, wenn n für 0, 1, 3 oder 4 steht oder wenn n für 2 steht und $R_2$ Phenyl-niederalkyl, Carboxy-niederalkyl, Niederalkoxycarbonyl-niederalkyl, Niederalkanoyloxy, Mercapto, Niederalkylthio, Phenyl-niederalkylthio, Phenylthio, Niederalkanoylthio, Carboxy, Niederalkoxycarbonyl oder Niederalkanoyl bedeutet; und $R_2$ wie oben unter Formel I definiert ist; wobei der Phenylring in den Resten Phenylsulfonyloxy, Phenyliminomethyl, Benzoyl, Phenyl-niederalkyl, Phenyl-niederalkylthio und Phenylthio unsubstituiert oder durch Niederalkyl, Niederalkoxy oder Halogen substituiert sein kann; wobei in einer Verbindung der Formel I* die beiden Substituenten $C_6H_4$-$R_1$ und $R_2$ an jedem der gesättigten Kohlenstoffatome des gesättigten Rings angeknüpft sein können, entweder beide am selben Kohlenstoffatom oder beide an verschiedenen Kohlenstoffatom; wobei mit "nieder" bezeichnete Reste bis zu 7 Kohlenstoffe enthalten; Stereoisomeren, Mischungen dieser Stereoisomeren und Salzen davon dadurch gekennzeichnet, dass man

a) eine Verbindung der Formel II

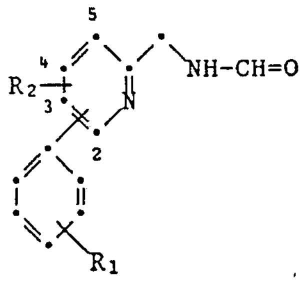

(II)

oder ein 4,5-Dihydroderivat davon, ringschliesst, um eine Verbindung der Formel I bzw. ein 7,8-Dihydroderivat davon zu erhalten, oder

b) eine Verbindung der Formel III

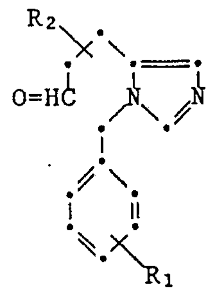

(III),

worin $R_2$ wie angedeutet an jedem der Kohlenstoffatome einschliesslich des Carbonylkohlenstoffs angeknüpft sein kann, ringschliesst, um ein 7,8-Dihydroderivat einer Verbindung der Formel I zu erhalten, worin der Substituent $C_6H_4$-$R_1$ in 5-Stellung angeknüpft ist, oder

c)/f) in einer Verbindung der Formel IV oder VII

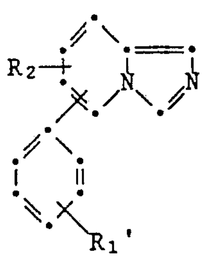

(IV)

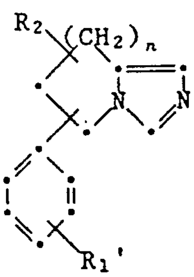

(VII)

oder in einem 7,8-Dihydroderivat der Formel IV, worin $R_1$' jeweils eine Gruppe bedeutet, die in Cyan umgewandelt werden kann, $R_1$' in Cyan umwandelt, um eine Verbindung der Formel I, ein 7,8-Dihydroderivat davon bzw. eine Verbindung der Formel I* zu erhalten, worin $R_1$ Cyan bedeutet, oder

d) eine Verbindung der Formel V

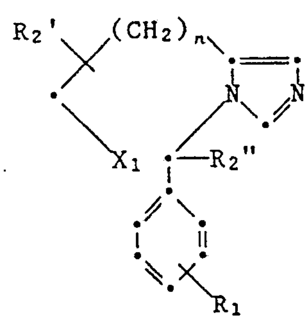

(V),

worin wenigstens einer der Reste $R_2$' und $R_2$" Wasserstoff und der andere einen Rest $R_2$ wie unter Formel I* definiert bedeutet, und $X_1$ eine Abgangsgruppe bedeutet, und $R_2$' an jedem der Kohlen-

stoffatome wie angedeutet angeknüpft sein kann, ringschliesst, um eine Verbindung der Formel I* zu erhalten, worin der Substituent $C_6H_4$-$R_1$ in 5-Stellung angeknüpft ist; oder $X_1$ eine Gruppe =CH-COOH oder einen Niederalkylester davon bedeutet, $R_2$' für Wasserstoff steht und $R_2$" wie unter Formel I* definiert ist, ringschliesst, um eine Verbindung der Formel I* zu erhalten, worin der Substituent $C_6H_4$-$R_1$ in 5-Stellung angeknüpft ist und die 6-Stellung durch Carboxymethyl oder Niederalkoxycarbonylmethyl substituiert ist, oder

e) eine Verbindung der Formel VI

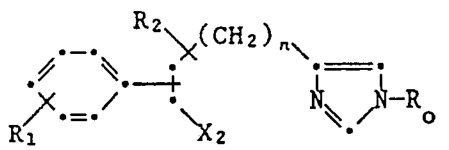

(VI)

worin die Substituenten $C_6H_4$-$R_1$ und $R_2$ an jedem der Kohlenstoffatome wie angedeutet angeknüpft sein können, entweder beide Reste am selben Kohlenstoffatom oder beide an verschiedenen Kohlenstoffatomen, $R_0$ eine NH-Schutzgruppe oder Wasserstoff bedeutet und $X_2$ eine Abgangsgruppe bedeutet, ringschliesst, um eine Verbindung der Formel I* zu erhalten; oder

g) eine Verbindung der Formel IX

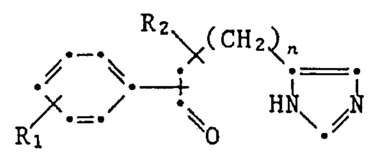

(IX)

worin die Substituenten $C_6H_4$-$R_1$ und $R_2$ an jedem der Kohlenstoffatome einschliesslich dem Carbonylkohlenstoff wie angedeutet angeknüpft sein können, entweder beide Reste am selben Kohlenstoffatom oder beide an verschiedenen Kohlenstoffatomen, gegebenenfalls unter reduktiven Bedingungen, ringschliesst, um ein 7,8-Dihydroderivat einer Verbindung der Formel I oder, im Falle von reduktiven Bedingungen, eine Verbindung der Formel I* erhält, oder

h) eine Verbindung analog zu Formel I, oder ein 7,8-Dihydroderivat davon, oder eine Verbindung analog zu Formel I*, welche jeweils eine zusätzliche Carboxygruppe in 1- oder 3-Stellung enthält, decarboxyliert, um eine Verbindung der Formel I, ein 7,8-Dihydroderivat davon oder eine Verbindung der Formel I* zu erhalten; wobei in den Ausgangsverbindungen der Formel II bis VII und IX die Symbole n, $R_1$ und $R_2$ die unter Formel I bzw. I* angegebenen Bedeutungen haben; und/oder eine Verbindung der Formel I, oder ein 7,8-Dihydroderivat davon, zu einem entsprechenden 5,6,7,8-Tetrahydroderivat der Formel I* reduziert, wobei gegebenenfalls gleichzeitig eine Reduktion des Substituenten $R_1$ und/oder $R_2$ in einen anderen Substituenten $R_1$ und/oder $R_2$ stattfindet; und/oder eine Verbindung der Formel I*, worin $R_2$ Carboxy bedeutet, decarboxyliert, um eine Verbindung der Formel I* zu erhalten, worin $R_2$ Wasserstoff ist; und/oder eine erhaltene Verbindung in eine andere Verbindung der Erfindung umwandelt und/oder ein erhaltenes Salz in die freie Verbindung oder in ein anderes Salz umwandelt und/oder eine erhaltene freie Verbindung in ein Salz umwandelt und/oder ein erhaltenes Gemisch von Isomeren oder Racematen in die einzelnen Isomeren oder Racematen auftrennt und/oder ein enantiomeren Gemisch, etwa ein Racemat, in die optischen Antipoden aufspaltet.

2. Verfahren gemäss Anspruch 1, dadurch gekennzeichnet, dass Verbindungen der Formel I, worin $R_1$ Niederalkyl, Hydroxy-$C_2$-$C_7$-alkyl; Niederalkyl, das durch Amino, Diniederalkylamino, 2 bis 5 Fluoratome, Niederalkoxycarbonyl, Carbamoyl oder Cyan substituiert ist; Nitro, Niederalkoxy, Amino, Niederalkylamino, Diniederalkylamino, Sulfo, Sulfamoyl, Iminomethyl oder Iminomethyl, das am Stickstoff durch Hydroxy, Niederalkoxy, Niederalkanoyloxy, Niederalkyl oder Phenyl substituiert ist, bedeutet und $R_2$ für Wasserstoff, Niederalkyl, Niederalkoxy oder Halogen steht; oder Verbindungen der Formel I*, worin n für 1, 2 oder 3 steht; $R_1$ wie oben unter Formel I definiert ist oder $R_1$ zusätzlich Hydroxymethyl, Carboxy-niederalkyl, Halogen, Hydroxy, Formyl, Carboxy, Niederalkoxycarbonyl, Carbamoyl, Niederalkylcarbamoyl, Diniederalkylcarbamoyl oder Cyan bedeuten kann, wenn n für 1 oder 3 steht oder wenn n für 2 steht und $R_2$ Phenyl-niederalkyl, Carboxy-niederalkyl, Niederalkoxycarbonyl-niederalkyl, Niederalkylthio, Phenyl-niederalkylthio, Phenylthio, Carboxy, Niederalkoxycarbonyl oder Niederalkanoyl bedeutet ; und $R_2$ Wasserstoff, Niederalkyl, Phenyl-niederalkyl, Carboxy-niederalkyl, Niederalkoxycarbonyl-niederalkyl, Halogen, Niederalkoxy, Niederalkylthio, Phenyl-niederalkylthio, Phenylthio, Carboxy, Niederalkoxycarbonyl oder Niederalkanoyl bedeutet; wobei in einer Verbindung der Formel $I^*$ die beiden Substituenten $C_6H_4$-$R_1$ und $R_2$ an jedem der gesättigten Kohlenstoffatome des gesättigten Rings angeknüpft sein können, entweder beide am selben Kohlenstoffatom oder beide an verschiedenen Kohlenstoffatomen; Stereoisomere, Mischungen dieser Stereoisomeren oder pharmazeutisch verwendbare Salze davon hergestellt werden.

**3.** Verfahren gemäss Anspruch 1, dadurch gekennzeichnet, dass Verbindungen der Formel $I^*$, worin n für 1, 2 oder 3 steht; $R_1$ Niederalkyl, Amine, Niederalkylamino oder Diniederalkylamino bedeutet oder $R_1$ zusätzlich Hydroxymethyl, Halogen, Formyl, Carboxy, Niederalkoxycarbonyl, Carbamoyl, Niederalkylcarbamoyl, Diniederalkylcarbamoyl oder Cyan bedeuten kann, wenn n für 1 oder 3 steht oder wenn n für 2 steht und $R_2$ Phenyl-niederalkyl, Carboxyniederalkyl, Niederalkoxycarbonyl-niederalkyl, Niederalkylthio, Carboxy oder Niederalkoxycarbonyl bedeutet; und $R_2$ Wasserstoff, Niederalkyl, Phenyl-niederalkyl, Carboxy-niederalkyl, Niederalkoxycarbonyl-niederalkyl, Niederalkylthio, Carboxy oder Niederalkoxycarbonyl bedeutet; wobei die beiden Substituenten $C_6H_4$-$R_1$ und $R_2$ an jedem der gesättigten Kohlenstoffatome des gesättigten Rings angeknüpft sein können, entweder beide am selben Kohlenstoffatom oder beide an verschiedenen Kohlenstoffatomen; Stereoisomere, Mischungen dieser Stereoisomeren oder pharmazeutisch verwendbare Salze davon hergestellt werden.

**4.** Verfahren zur Herstellung von Verbindungen Formel Ia

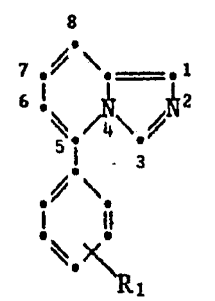

(Ia)

worin $R_1$ Cyan, Nitro oder $C_1$-$C_4$-Alkyl bedeutet, den 7,8-Dihydroderivaten davon oder den 5,6,7,8-Tetrahydroderivaten davon mit der Formel Ib

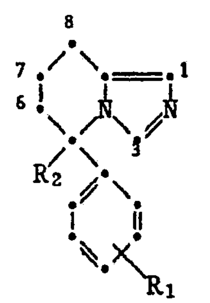

(Ib)

worin $R_1$ wie oben unter Formel Ia definiert ist und $R_2$ Wasserstoff, $C_1$-$C_4$-Alkyl, Benzyl, Halogen, $C_1$-$C_4$-Alkoxy, $C_1$-$C_4$-Alkylthio, Benzylthio, 2-Phenylethylthio, Diphenylmethylthio, Phenylthio, $C_1$-$C_4$-Alkanoyloxy, $C_1$-$C_4$-Alkanoylthio, Carboxy-$C_1$-$C_4$-alkyl, $C_1$-$C_4$-Alkoxycarbonyl-$C_1$-$C_4$-alkyl oder $C_1$-$C_4$-Alkanoyl bedeutet, Stereoisomeren, Mischungen dieser Stereoisomeren und Salzen dieser Verbindungen, dadurch gekennzeichnet, dass man

a) eine Verbindung der Formel IIa

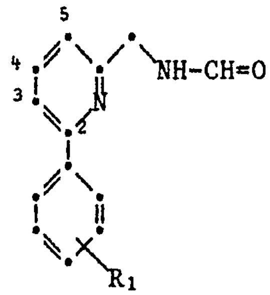

(IIa),

worin $R_1$ wie oben unter Formel Ia definiert ist, oder ein 4,5-Dihydroderivat davon, unter sauren Bedingungen cyclisiert, um eine Verbindung der Formel Ia oder ein 7,8-Dihydroderivat davon zu erhalten, oder

b) zur Herstellung eines 7,8-Dihydroderivates einer Verbindung der Formel Ia, eine Verbindung der Formel IIIa

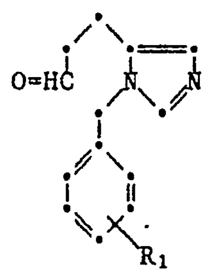

(IIIa),

worin $R_1$ wie oben unter Formel Ia definiert ist, unter basischen Bedingungen cyclisiert, oder

c) in einer Verbindung der Formel IVa

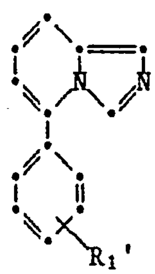

(IVa),

worin $R_1'$ einen Rest bedeutet, der in Cyan umgewandelt werden kann, oder in einem 7,8-Dihydroderivat davon, $R_1'$ in Cyan umwandelt, um eine Verbindung der Formel Ia, oder ein 7,8-Dihydroderivat davon, zu erhalten, oder

d) eine Verbindung der Formel Vb

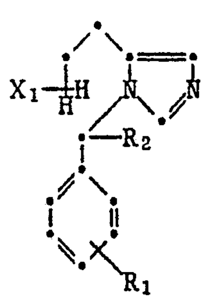

(Vb),

worin $R_1$ und $R_2$ wie oben unter Formel Ib definiert sind und $X_1$ eine Abgangsgruppe bedeutet, in Gegenwart einer Base cyclisiert, um eine Verbindung der Formel Ib zu erhalten, oder

e) eine Verbindung der Formel VIb

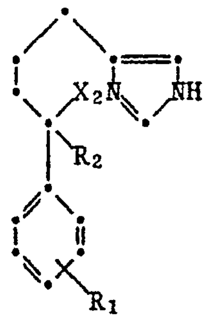

(VIb),

worin $R_1$ und $R_2$ wie oben unter Formel Ib definiert sind und $X_2$ eine Abgangsgruppe bedeutet, worin die NH-Gruppe durch eine NH-Schutzgruppe geschützt sein kann, in Gegenwart einer Base cyclisiert, um eine Verbindung der Formel Ib zu erhalten, oder

f) in einer Verbindung der Formel VIIb

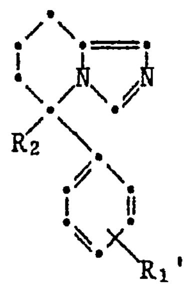

(VIIb),

worin $R_1$' einen Rest bedeutet, der in Cyan umgewandelt werden kann und worin $R_2$ wie oben unter Formel Ib definiert ist, den Rest $R_1$' in Cyan umwandelt, um eine Verbindung der Formel Ib zu erhalten, worin $R_1$ Cyan bedeutet;

g) eine Verbindung der Formel IXb

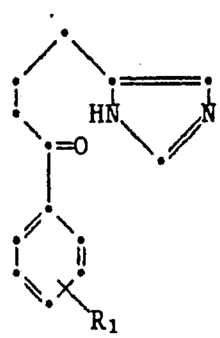

(IXb),

gegebenenfalls unter reduktiven Bedingungen, cyclisiert, um ein 7,8-Dihydroderivat einer Verbindung der Formel Ia oder, im Falle von reduktiven Bedingungen, eine Verbindung der Formel Ib zu erhalten, oder

h) eine Verbindung analog zu Formel Ia, oder ein 7,8-Dihydroderivat davon, oder eine Verbindung analog zu Formel Ib, die jeweils eine zusätzliche Carboxygruppe in 1- oder 3-Stellung enthält, decarboxyliert, um eine Verbindung der Formel Ia, ein 7,8-Dihydroderivat davon oder eine Verbindung der Formel Ib zu erhalten und/oder eine Verbindung der Formel Ib, worin $R_2$ Carboxy ist, decarboxyliert, um eine andere Verbindung der Formel Ib zu erhalten, worin $R_2$ Wasser stoff bedeutet, und/oder eine Verbindung der Formel Ia, oder ein 7,8-Dihydroderivat davon, mit Wasserstoff in Gegenwart eines Hydrierungskatalysators zum entsprechenden 5,6,7,8-Tetrahydroderivat der Formel Ib reduziert, und/oder eine erhaltene Verbindung in eine andere Verbindung der Erfindung umwandelt und/oder ein erhaltenes Salz in die freie Verbindung oder in ein anderes Salz umwandelt und/oder eine freie Verbindung, die eine salzbildende Gruppe enthält, in ein Salz umwandelt

und/oder eine erhaltenes racemisches Gemisch in die individuellen Enantiomere auftrennt.

5. Verfahren gemäss Anspruch 4, dadurch gekennzeichnet, dass man eine der dort angegebenen Verfahrensvarianten a), b), c), d), oder f) durchführt oder
e) eine Verbindung der Formel VIb

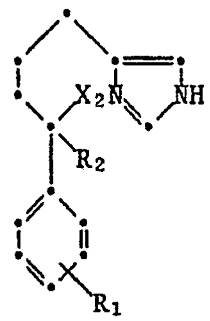

(VIb),

worin $R_1$ und $R_2$ wie oben unter Formel Ib definiert sind und $X_2$ eine Abgangsgruppe bedeutet, in Gegenwart einer Base cyclisiert, um eine Verbindung der Formel Ib zu erhalten, und/oder eine Verbindung der Formel Ia, oder ein 7,8-Dihydroderivat davon, mit Wasserstoff in Gegenwart eines Hydrierungskatalysators zum entsprechenden 5,6,7,8-Tetrahydroderivat der Formel Ib reduziert, und/oder eine erhaltene Verbindung in eine andere Verbindung der Erfindung umwandelt und/oder ein erhaltenes Salz in die freie Verbindung oder in ein anderes Salz umwandelt und/oder eine freie Verbindung, die eine salzbildende Gruppe enthält, in ein Salz umwandelt und/oder eine erhaltenes racemisches Gemisch in die individuellen Enantiomere auftrennt.

6. Verfahren gemäss Anspruch 5 dadurch gekennzeichnet, dass Verbindungen der Formel Ia, worin $R_1$ Cyan bedeutet, die 7,8-Dihydroderivate davon, oder die 5,6,7,8-Tetrahydroderivate davon mit der Formel Ib, worin $R_1$ Cyan bedeutet und $R_2$für Wasserstoff, $C_1$-$C_4$-Alkyl, $C_1$-$C_4$-Alkoxy, $C_1$-$C_4$-Alkylthio, Benzylthio, 2-Phenylethylthio, Diphenylmethylthio, Phenylthio oder $C_1$-$C_4$-Alkanoyl steht oder pharmazeutisch verwendbare Säureadditionssalze von Verbindungen der Formel Ia und

7. Verfahren gemäss Anspruch 5, dadurch gekennzeichnet das Verbindungen der Formel Ia, worin $R_1$ Cyan bedeutet, die 7,8-Dihydroderivate davon oder die 5,6,7,8-Tetrahydroderivate davon mit der Formel Ib, worin $R_1$ wie oben unter Formel Ia definiert ist und $R_2$ für Wasserstoff steht, oder pharmazeutisch verwendbare Säureadditionssalze davon hergestellt werden.

8. Verfarhen gemäss 5, dadurch gekennzeichnet, dass Verbindungen Anspruch der Formel Ic

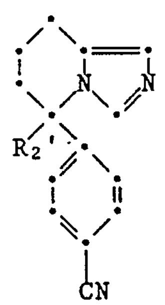

(Ic)

worin $R_2$' Wasserstoff, $C_1$-$C_4$-Alkyl, $C_1$-$C_4$-Alkoxy, $C_1$-$C_4$-Alkylthio, Benzylthio, 2-Phenylethylthio, Diphenylmethylthio, Phenylthio oder $C_1$-$C_4$-Alkanoyl bedeutet, oder pharmazeutisch verwendbare Säureadditionssalze davon hergestellt werden.

9. Verfahren gemäss Anspruch 5, dadurch gekennzeichnet,dass eine Verbindung der Formel Ic worin $R_2$' Wasserstoff bedeutet, mit dem Namen 5-(p-Cyanphenyl)-5,6,7,8-tetrahydroimidazo(1,5-a]pyridin, oder pharmazeutisch verwendbare Säureadditionssalze davon hergestellt werden.

**10.** Verfahren gemäss Anspruch 4, dadurch gekennzeichnet, dass eine Verbindung der Formel Ic worin $R_2$' Wasserstoff bedeutet, mit dem Namen 5-(p-Cyanphenyl)-5,6,7,8-tetrahydroimidazo[1,5-a]pyridin, oder pharmazeutisch verwendbare Säureadditionssalze davon hergestellt werden.

**11.** Verfahren zur Herstellung von Verbindungen der Formel Ia wie in Anspruchs 4 wiedergegeben, worin R1 Wasserstoff, p-Toluolsulfonyloxy, Benzolsulfonyloxy, Methylsulfonyloxy, Sulfo, Amino, Carbamoyl, $C_1$-$C_4$-Alkylcarbamoyl oder Hydroxyiminomethyl bedeutet; oder den 5,6,7,8-Tetrahydroverbindungen der Formel Ib wie in Anspruch 4 wiedergegeben, worin $R_1$wie oben unter Formel Ia definiert ist oder Halogen bedeutet und $R_2$ für Wasserstoff, $C_1$-$C_4$-Alkyl, Benzyl, Halogen, $C_1$-$C_4$-Alkoxy, $C_1$-$C_4$-Alkanoyloxy, $C_1$-$C_4$-Alkylthio, Benzylthio, 2-Phenylethylthio, Diphenylmethylthio, Phenylthio, $C_1$-$C_4$-Alkanoylthio, Carboxy-$C_1$-$C_4$-alkyl, $C_1$-$C_4$-Alkoxycarbonyl-$C_1$-$C_4$-alkyl oder $C_1$-$C_4$-Alkanoyl steht; oder pharmazeutisch verwendbaren Salzen davon, dadurch gekennzeichnet, dass die im Anspruch 4 angegebenen Verfahrensvarianten durchgeführt werden.

**12.** Verfahren zur Herstellung von Verbindungen der Formel Ia wie in Anspruch 4 wiedergegeben, worin $R_1$ Wasserstoff, p-Toluolsulfonyloxy, Benzolsulfonyloxy, Methylsulfonyloxy, Sulfo, Amino oder Hydroxyiminomethyl bedeutet; oder den 5,6,7,8-Tetrahydroverbindungen der Formel Ib wie in Anspruch 4 wiedergegeben, worin $R_1$ wie oben unter Formel Ia angegeben definiert ist und $R_2$ für Wasserstoff steht; oder worin $R_1$ Wasserstoff, Halogen, p-Toluolsulfonyloxy, Benzolsulfonyloxy, Methylsulfonyloxy, Sulfo, Amino, Carboxy, Carbamoyl, $C_1$-$C_4$-Alkylcarbamoyl, Formyl oder Hydroxyiminomethyl bedeutet und $R_2$ für $C_1$-$C_4$-Alkyl, Benzyl, Halogen, $C_1$-$C_4$-Alkoxy, $C_1$-$C_4$-Alkanoyloxy, $C_1$-$C_4$-Alkylthio, Benzylthio, 2-Phenylethylthio, Diphenylmethylthio, Phenylthio, $C_1$-$C_4$-Alkanoylthio, Carboxy-$C_1$-$C_4$-alkyl, $C_1$-$C_4$-Alkoxycarbonyl-$C_1$-$C_4$-alkyl oder $C_1$-$C_4$-Alkanoyl steht; oder pharmazeutisch verwendbaren Salzen davon dadurch gekennzeichnet, dass die in Anspruch 5 angegebenen Verfahrensvarianten durchgeführt werden.

**13.** Verfahren gemäss Anspruch 12, dadurch gekennzeichnet, dass Verbindungen der Formel Ia und Ib worin $R_1$ Carbamoyl bedeutet, oder Verbindungen der Formel Ib, worin $R_1$ Halogen bedeutet, hergestellt werden.

**14.** Verfahren gemäss Anspruch 4, dadurch gekennzeichnet, dass (-)-(5-(p-Cyanphenyl)-5,6,7,8-tetrahydroimidazo[1,5-a]pyridin oder pharmazeutisch verwendbare Salze davon hergestellt werden.

**15.** Verfahren gemäss Anspruch 4, dadurch gekennzeichnet, dass (+)-(5-(P-Cyanphenyl)-5,6,7,8-tetrahydroimidazo[1,5-a]pyridin oder pharmazeutisch verwendbare Salze davon hergestellt werden.

**16.** Verfahren gemäss Anspruch 5, dadurch gekennzeichnet, dass 5-(p-Cyanphenyl)-7,8-dihydroimidazo-[1,5-a]pyridin oder pharmazeutisch verwendbare Salze davon hergestellt werden.

**17.** Verfahren gemäss Anspruch 5, dadurch gekennzeichnet, dass man 5-(p-Cyanphenyl)imidazo[1,5a]-pyridin oder pharmazeutisch verwendbare Salze davon herstellt.

**18.** Verfahren gemäss Anspruch, 11 dadurch gekennzeichnet, dass man 5-(p-Bromphenyl)-5,6,7,8-tetrahydroimidazo[1,5-a]pyridin, ein Stereoisomeres davon, Mischungen dieser Stereoisomeren oder pharmazeutisch verwendbare Salze davon herstellt.

**19.** Verfahren gemäss Anspruch 1, dadurch gekennzeichnet, dass man 5H-(4-Cyanphenyl)-6,7-dihydropyrrolo[1,2-c]imidazol, ein Stereoisomeres davon, Mischungen dieser Stereoisomeren oder pharmazeutisch verwendbare Salze davon herstellt.

**20.** Verfahren gemäss Anspruch 1, dadurch gekennzeichnet, dass man 5H-5-(4-Cyanphenyl)-6,7,8,9-tetrahydroimidazo[1,5a]azepin, ein Stereoisomeres davon, Mischungen dieser Stereoisomeren oder pharmazeutisch verwendbare Salze davon herstellt.

**21.** Verfahren gemäss Anspruch 1, dadurch gekennzeichnet, dass man 5-(4-Cyanphenyl)-6-ethoxycarbonylmethyl-5,6,7,8-tetrahydroimidazo[1,5-a]pyridin, ein Stereoisomeres davon, Mischungen dieser Stereoisomeren oder pharmazeutisch verwendbare Salze davon herstellt.

22. Verfahren gemäss Anspruchs 4, dadurch gekennzeichnet, dass man 5-(p-Cyanphenyl)imidazo[1,5a]-pyridin oder pharmazeutisch verwendbare Salze davon herstellt.

23. Verfahren gemäss Anspruch 12, dadurch gekennzeichnet, dass man 5-(p-Bromphenyl)-5,6,7,8-tetrahydroimidazo[1,5-a]pyridin, ein Stereoisomeres davon, Mischungen dieser Stereoisomeren oder pharmazeutisch verwendbare Salze davon herstellt.

24. Verfahren gemäss Anspruch 1, dadurch gekennzeichnet, dass 5-(4-Cyanphenyl)-6-carboymethyl-5,6,7,8-tetrahydroimidazo[1,5-a]pyridin ein Stereoisomeres davon, Mischungen dieser Stereoisomeren oder pharmazeutisch verwendbare Salze davon hergestellt werden.

25. Verfahren gemäss Anspruch 4, dadurch gekennzeichnet, dass 5-Benzyl-5-(4-cyanphenyl)-5,6,7,8-tetrahydroimidazo[1,5-a]-pyridin ein Stereoisomeres davon, Mischungen dieser Stereoisomeren oder pharmazeutisch verwendbare Salze davon hergestellt werden.

26. Verfahren zur Herstellung von 7-(p-Cyanphenyl)-5,6,7,8-tetrahydroimidazo[1,5-a]pyridin einem Stereoisomeren davon, Mischungen dieser Stereoisomeren oder pharmazeutisch verwendbaren Salzen davon, dadurch gekennzeichnet, dass die im Anspruch 1 angegebenen Verfahrensvarianten durchgeführt werden.

27. Verfahren zur Herstellung von pharmazeutischen Präparaten, dadurch gekennzeichnet, dass man eine nach dem Verfahren gemäss einem der Ansprüche 1, 4, 10, 11, 14, oder 15 erhaltene Verbindung oder ein pharmazeutisch verwendbares Salz einer solchen Verbindung mit einem pharmazeutisch verwendbaren Trägermaterial mischt.

28. Verfahren zur Herstellung von pharmazeutischen Präparaten, dadurch gekennzeichnet, dass man eine nach dem Verfahren gemäss einem der Anspruch 5, 9 oder 12 erhaltene Verbindung oder ein pharmazeutisch verwendbares Salz einer solchen Verbindung mit einem pharmazeutisch verwendbaren Trägermaterial mischt.

## Claims

1. The use of substituted imidazo[1,5-a]pyridine derivatives of formula I

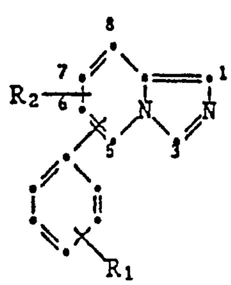

(I)

wherein $R_1$ represents hydrogen, lower alkyl; lower alkyl substituted by hydroxy, lower alkoxy, lower alkanoyloxy, lower alkanoyl, amino, lower alkylamino, di-lower alkylamino, halogen, sulfo, carboxy, lower alkoxycarbonyl, carbamoyl or cyano; nitro, halogen, hydroxy, lower alkoxy, lower alkanoyloxy, phenylsulfonyloxy, lower alkylsulfonyloxy, mercapto, lower alkylthio, lower alkylsulfinyl, lower alkylsulfonyl, lower alkanoylthio, amino, lower alkylamino, di-lower alkylamino, lower alkyleneamino, N-morpholino, N-thiomorpholino, optionally 4-lower alkyl-substituted N-piperazino, tri-lower alkylammonio, sulfo, lower alkoxysulfonyl, sulfamoyl, lower alkylsulfamoyl, di-lower alkylsulfamoyl, formyl; iminomethyl optionally N-substituted by hydroxy, lower alkoxy, lower alkanoyloxy, lower alkyl, phenyl or amino; $C_2$-$C_7$alkanoyl, benzoyl, carboxy, lower alkoxycarbonyl, carbamoyl, lower alkylcarbamoyl, di-lower alkylcarbamoyl, cyano, 5-tetrazolyl, optionally lower alkyl-substituted 4,5-dihydro-2-oxazolyl or hydroxycarbamoyl; and $R_2$ represents hydrogen, lower alkyl, phenyl-lower alkyl, carboxy-lower alkyl, lower alkoxycarbonyl-lower alkyl, halogen, hydroxy, lower alkoxy, lower alkanoyloxy, mercapto, lower alkylthio, phenyl-lower alkylthio, phenylthio, lower alkanoylthio, carboxy, lower alkoxycarbonyl or lower alkanoyl; the 7,8-dihydro derivatives thereof; or compounds of formula I*

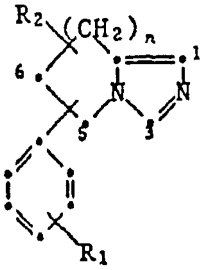

(I*)

wherein n denotes 0, 1, 2, 3 or 4; and $R_1$ and $R_2$ are as defined above under formula I; it being possible for the phenyl ring within the radicals phenylsulfonyloxy, phenyliminomethyl, benzoyl, phenyl-lower alkyl, phenyl-lower alkylthio and phenylthio to be unsubstituted or substituted by lower alkyl, lower alkoxy or halogen; and in a compound of formula I* it being possible for the two substituents $C_6H_4$-$R_1$ and $R_2$ to be attached to any of the saturated carbon atoms of the saturated ring, either both to the same carbon atom or to different carbon atoms; radicals designated "lower" containing up to 7 carbon atoms; stereoisomers, mixtures of these stereoisomers, or pharmaceutically acceptable salts thereof, for the manufacture of pharmaceutical preparations for the treatment of diseases responsive to aromatase inhibition.

2. The use according to claim 1, wherein compounds of formula I wherein $R_1$ represents lower alkyl; lower alkyl substituted by hydroxy, amino, di-lower alkylamino, 1 to 5 fluorine atoms, carboxy, lower alkoxycarbonyl, carbamoyl or cyano; nitro, halogen, hydroxy, lower alkoxy, amino, lower alkylamino, di-lower alkylamino, sulfo, sulfamoyl, formyl, iminomethyl; iminomethyl N-substituted by hydroxy, lower alkoxy, lower alkanoyloxy, lower alkyl or phenyl; carboxy, lower alkoxycarbonyl, carbamoyl, lower alkylcarbamoyl, di-lower alkylcarbamoyl or cyano; and $R_2$ is hydrogen, lower alkyl, lower alkoxy or halogen; or compounds of formula I* wherein n denotes 1, 2 or 3; $R_1$ is as defined above under formula I and $R_2$ represents hydrogen, lower alkyl, phenyl-lower alkyl, carboxy-lower alkyl, lower alkoxycarbonyl-lower alkyl, halogen, lower alkoxy, lower alkylthio, phenyl-lower alkylthio, phenylthio, carboxy, lower alkoxycarbonyl or lower alkanoyl; in a compound of formula I* it being possible for the two substituents $C_6H_4$-$R_1$ and $R_2$ to be attached to any of the saturated carbon atoms of the saturated ring, either both to the same carbon atom or to different carbon atoms; stereoisomers, mixtures of these stereoisomers, or pharmaceutically acceptable salts thereof, are used as specified above.

3. The use according to claim 1, wherein compounds of formula I wherein $R_1$ represents lower alkyl, hydroxy-lower alkyl, halogen, amino, formyl, carboxy, lower alkoxycarbonyl, carbamoyl, N-lower alkyl-carbamoyl or cyano; and $R_2$ is hydrogen; or compounds of formula I* wherein n denotes 1, 2 or 3; $R_1$ is as defined above under formula I and $R_2$ represents hydrogen, lower alkylthio, lower alkoxycarbonyl, phenyl-lower alkyl, carboxy-lower alkyl or lower alkoxycarbonyl-lower alkyl; in a compound of formula I* it being possible for the two substituents $C_6H_4$-$R_1$ and $R_2$ to be attached to any of the saturated carbon atoms of the saturated ring, either both to the same carbon atom or to different carbon atoms; stereoisomers, mixtures of these stereoisomers, or pharmaceutically acceptable salts thereof, are used.

4. Pharmaceutical preparations containing a compound of formula I as shown in claim 1, wherein $R_1$ represents halogen, hydroxy, mercapto, or lower alkyl substituted by lower alkoxy, lower alkanoyloxy or halogen; and $R_2$ is hydrogen, lower alkyl, lower alkoxy or halogen; or a compound of formula I* as shown in claim 1, wherein n denotes 2, $R_1$ is as defined above under formula I and $R_2$ represents hydrogen, lower alkyl, phenyl-lower alkyl, carboxy-lower alkyl, lower alkoxycarbonyl-lower alkyl, halogen, lower alkoxy, lower alkylthio, phenyl-lower alkylthio, phenylthio, carboxy, lower alkoxycarbonyl or lower alkanoyl; in a compound of formula I* it being possible for the two substituents $C_6H_4$-$R_1$ and $R_2$ to be attached to any of the saturated carbon atoms of the saturated ring, either both to the same carbon atom or to different carbon atoms; radicals designated "lower" containing up to 7 carbon atoms; a stereoisomer, a mixture of these stereoisomers or a pharmaceutically acceptable salt thereof, together with one or more pharmaceutically acceptable carriers.

5. Compounds of formula I as shown in claim 1, wherein $R_1$ represents hydrogen, lower alkyl; $C_2$-$C_7$alkyl

substituted by hydroxy, lower alkoxy, halogen or lower alkanoyloxy; lower alkyl substituted by lower alkanoyl, amino, lower alkylamino, di-lower alkylamino, sulfo, lower alkoxycarbonyl, carbamoyl or cyano; nitro, lower alkoxy, lower alkanoyloxy, phenylsulfonyloxy, lower alkylsulfonyloxy, lower alkylthio, lower alkylsulfinyl, lower alkylsulfonyl, lower alkanoylthio, amino, lower alkylamino, di-lower alkylamino, lower alkyleneamino, N-morpholino, N-thiomorpholino, optionally 4-lower alkyl-substituted N-piperazino, tri-lower alkylammonio, sulfo, lower alkoxysulfonyl, sulfamoyl, lower alkylsulfamoyl, di-lower alkylsulfamoyl; iminomethyl optionally N-substituted by hydroxy, lower alkoxy, lower alkanoyloxy, lower alkyl, phenyl or amino; $C_2$-$C_7$alkanoyl or benzoyl; and $R_2$ represents hydrogen, lower alkyl, phenyl-lower alkyl, carboxy-lower alkyl, lower alkoxycarbonyl-lower alkyl, halogen, hydroxy, lower alkoxy, lower alkanoyloxy, mercapto, lower alkylthio, phenyl-lower alkylthio, phenylthio, lower alkanoylthio, carboxy, lower alkoxycarbonyl or lower alkanoyl; the 7,8-dihydro derivatives thereof and also such 7,8-dihydro derivatives wherein $R_1$ is hydroxymethyl, lower alkoxymethyl, halomethyl, lower alkanoyloxymethyl, carboxy-lower alkyl, halogen, hydroxy, mercapto, formyl, carboxy, lower alkoxycarbonyl, carbamoyl, lower alkylcarbamoyl, di-lower alkylcarbamoyl, cyano, 5-tetrazolyl, optionally lower alkyl-substituted 4,5-dihydro-2-oxazolyl or hydroxycarbamoyl and $R_2$ is as defined above under formula I; and compounds of formula I* as shown in claim 1, wherein n denotes 0, 1, 2, 3 or 4; $R_1$ is as defined above under formula I or $R_1$ may be, in addition, hydroxymethyl, lower alkoxymethyl, halomethyl, lower alkanoyloxymethyl, carboxy-lower alkyl, halogen, hydroxy, mercapto, formyl, carboxy, lower alkoxycarbonyl, carbamoyl, lower alkylcarbamoyl, di-lower alkylcarbamoyl, cyano, 5-tetrazolyl, optionally lower alkyl-substituted 4,5-dihydro-2-oxazolyl or hydroxycarbamoyl if n represents 0, 1, 3 or 4 or if n represents 2 and $R_2$ is phenyl-lower alkyl, carboxy-lower alkyl, lower alkoxycarbonyl-lower alkyl, lower alkanoyloxy, mercapto, lower alkylthio, phenyl-lower alkylthio, phenylthio, lower alkanoylthio, carboxy, lower alkoxycarbonyl or lower alkanoyl; and $R_2$ is as defined above under formula I; it being possible for the phenyl ring within the radicals phenylsulfonyloxy, phenyliminomethyl, benzoyl, phenyl-lower alkyl, phenyl-lower alkylthio and phenylthio to be unsubstituted or substituted by lower alkyl, lower alkoxy or halogen; and in a compound of formula I* it being possible for the two substituents $C_6H_4$-$R_1$ and $R_2$ to be attached to any of the saturated carbon atoms of the saturated ring, either both to the same carbon atom or to different carbon atoms; radicals designated "lower" containing up to 7 carbon atoms; stereoisomers, mixtures of these stereoisomers, and salts thereof.

6. Compounds according to claim 5 of formula I wherein $R_1$ represents lower alkyl, hydroxy-$C_2$-$C_7$alkyl; lower alkyl substituted by amino, di-lower alkylamino, 2 to 5 fluorine atoms, lower alkoxycarbonyl, carbamoyl or cyano; nitro, lower alkoxy, amino, lower alkylamino, di-lower alkylamino, sulfo, sulfamoyl, iminomethyl or iminomethyl N-substituted by hydroxy, lower alkoxy, lower alkanoyloxy, lower alkyl or phenyl; and $R_2$ is hydrogen, lower alkyl, lower alkoxy or halogen; and compounds of formula I* wherein n denotes 1, 2 or 3; $R_1$ is as defined above under formula I or $R_1$ may be, in addition, hydroxymethyl, carboxy-lower alkyl, halogen, hydroxy, formyl, carboxy, lower alkoxycarbonyl, carbamoyl, lower alkylcarbamoyl, di-lower alkylcarbamoyl or cyano if n represents 1 or 3 or if n represents 2 and $R_2$ is phenyl-lower alkyl, carboxy-lower alkyl, lower alkoxycarbonyl-lower alkyl, lower alkylthio, phenyl-lower alkylthio, phenylthio, carboxy, lower alkoxycarbonyl or lower alkanoyl; and $R_2$ represents hydrogen, lower alkyl, phenyl-lower alkyl, carboxy-lower alkyl, lower alkoxycarbonyl-lower alkyl, halogen, lower alkoxy, lower alkylthio, phenyl-lower alkylthio, phenylthio, carboxy, lower alkoxycarbonyl or lower alkanoyl; in a compound of formula I* it being possible for the two substituents $C_6H_4$-$R_1$ and $R_2$ to be attached to any of the saturated carbon atoms of the saturated ring, either both to the same carbon atom or to different carbon atoms; stereoisomers, mixtures of these stereoisomers, and pharmaceutically acceptable salts thereof.

7. Compounds according to claim 5 of formula I* wherein n denotes 1, 2 or 3; $R_1$ represents lower alkyl, amino, lower alkylamino or di-lower alkylamino, or $R_1$ may be, in addition, hydroxymethyl, halogen, formyl, carboxy, lower alkoxycarbonyl, carbamoyl, lower alkylcarbamoyl, di-lower alkylcarbamoyl or cyano if n represents 1 or 3 or if n represents 2 and $R_2$ is phenyl-lower alkyl, carboxy-lower alkyl, lower alkoxycarbonyl-lower alkyl, lower alkylthio, carboxy or lower alkoxycarbonyl; and $R_2$ is hydrogen, lower alkyl, phenyl-lower alkyl, carboxy-lower alkyl, lower alkoxycarbonyl-lower alkyl, lower alkylthio, carboxy or lower alkoxycarbonyl; it being possible for the two substituents $C_6H_4$-$R_1$ and $R_2$ to be attached to any of the saturated carbon atoms of the saturated ring, either both to the same carbon atom or to different carbon atoms; stereoisomers, mixtures of these stereoisomers, and pharmaceutically acceptable salts thereof.

**8.** Compounds of formula Ia

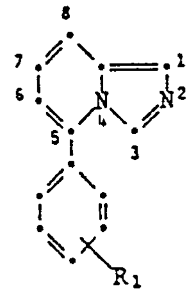

(Ia)

wherein $R_1$ represents cyano, nitro or $C_1$-$C_4$alkyl, the 7,8-dihydro derivatives thereof and the 5,6,7,8-tetra-hydro derivatives thereof of formula Ib

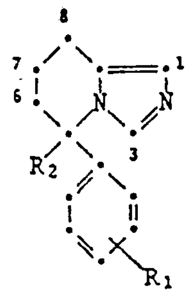

(Ib)

wherein $R_1$ is as defined above under formula Ia and $R_2$ is hydrogen, $C_1$-$C_4$alkyl, benzyl, halogen, $C_1$-$C_4$alkoxy, $C_1$-$C_4$alkylthio, benzylthio, 2-phenylethylthio, diphenylmethylthio, phenylthio, $C_1$-$C_4$-alkanoyloxy, $C_1$-$C_4$alkanoyl-thio, carboxy-$C_1$-$C_4$alkyl, $C_1$-$C_4$alkoxycarbonyl-$C_1$-$C_4$alkyl or $C_1$-$C_4$alkanoyl, stereoisomers, mixtures of these stereoisomers, and salts of these compounds.

**9.** Compounds according to claim 8 of formula Ia wherein $R_1$ represents cyano, the 7,8-dihydro derivatives thereof, and the 5,6,7,8-tetrahydro derivatives thereof of formula Ib wherein $R_1$ is cyano and $R_2$ is hydrogen, $C_1$-$C_4$alkyl, $C_1$-$C_4$alkoxy, $C_1$-$C_4$alkylthio, benzylthio, 2-phenylethylthio, diphenylmethylthio, phenylthio or $C_1$-$C_4$alkanoyl, and pharmaceutically acceptable acid addition salts of compounds of formulae Ia and Ib.

**10.** Compounds according to claim 8 of formula Ia wherein $R_1$ is cyano, the 7,8-dihydro derivatives thereof, and the 5,6,7,8-tetrahydro derivatives thereof of formula Ib wherein $R_1$ is as defined above under formula Ia and $R_2$ is hydrogen, and pharmaceutically acceptable acid addition salts thereof.

**11.** Compounds according to claim 8 of formula Ic

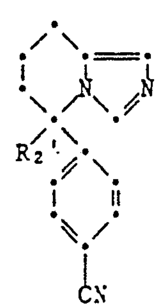

(Ic)

wherein $R_2'$ is hydrogen, $C_1$-$C_4$alkyl, $C_1$-$C_4$alkoxy, $C_1$-$C_4$alkylthio, benzylthio, 2-phenylethylthio, diphenylmethylthio, phenylthio or $C_1$-$C_4$alkanoyl, and pharmaceutically acceptable acid addition salts thereof.

12. A compound of formula Ic according to claim 11, wherein $R_2'$ is hydrogen, being 5-(p-cyanophenyl)-5,6,7,8-tetrahydroimidazo[1,5a]pyridine, and pharmaceutically acceptable acid addition salts thereof.

13. Compounds of formula Ia as shown in claim 8, wherein $R_1$ represents hydrogen, p-toluenesulfonyloxy, benzenesulfonyloxy, methylsulfonyloxy, sulfo, amino, carbamoyl, $C_1$-$C_4$alkylcarbamoyl or hydroxyiminomethyl; and the 5,6,7,8-tetrahydro compounds of formula Ib as shown in claim 8, wherein $R_1$ is as defined above under formula Ia or is halogen, and $R_2$ is hydrogen, $C_1$-$C_4$alkyl, benzyl, halogen, $C_1$-$C_4$alkoxy, $C_1$-$C_4$alkanoyloxy, $C_1$-$C_4$alkylthio, benzylthio, 2-phenylethylthio, diphenylmethylthio, phenylthio, $C_1$-$C_4$alkanoylthio, carboxy-$C_1$-$C_4$alkyl, $C_1$-$C_4$alkoxycarbonyl-$C_1$-$C_4$alkyl or $C_1$-$C_4$alkanoyl; and pharmaceutically acceptable salts thereof.

14. Compounds of formulae Ia and Ib according to claim 13, wherein $R_1$ represents carbamoyl, and compounds of formula Ib wherein $R_1$ represents halogen.

15. Compounds according to claim 13 of formula Ia wherein $R_1$ is hydrogen, p-toluenesulfonyloxy, benzenesulfonyloxy, methylsulfonyloxy, sulfo, amino or hydroximinomethyl; and the 5,6,7,8-tetrahydro compounds of formula Ib wherein $R_1$ is as defined above under formula Ia and $R_2$ is hydrogen; or wherein $R_1$ is hydrogen, halogen, p-toluenesulfonyloxy, benzenesulfonyloxy, methylsulfonyloxy, sulfo, amino, carboxy, carbamoyl, $C_1$-$C_4$alkylcarbamoyl, formyl or hydroxyiminomethyl and $R_2$ represents $C_1$-$C_4$-alkyl, benzyl, halogen, $C_1$-$C_4$alkoxy, $C_1$-$C_4$alkanoyloxy, $C_1$-$C_4$alkylthio, benzylthio, 2-phenylethylthio, diphenylmethylthio, phenylthio, $C_1$-$C_4$alkanoylthio, carboxy-$C_1$-$C_4$alkyl, $C_1$-$C_4$alkoxycarbonyl-$C_1$-$C_4$alkyl or $C_1$-$C_4$alkanoyl; and pharmaceutically acceptable salts thereof.

16. (-)-(5-(p-cyanophenyl)-5,6,7,8-tetrahydroimidazo[1,5-a]pyridine according to claim 8 and pharmaceutically acceptable salts thereof.

17. (+)-(5-(p-cyanophenyl)-5,6,7,8-tetrahydroimidazo-[1,5-a]pyridine according to claim 8 and pharmaceutically acceptable salts thereof.

18. 5-(p-cyanophenyl)-7,8-dihydroimidazo[1,5-a]pyridine according to claim 8 and pharmaceutically acceptable salts thereof.

19. 5-(p-cyanophenyl)imidazo[1,5-a]pyridine according to claim 8 and pharmaceutically acceptable salts thereof.

20. 5-(p-bromophenyl)-5,6,7,8-tetrahydroimidazo[1,5-a]-pyridine according to claim 13, a stereoisomer thereof, mixtures of these stereoisomers, and pharmaceutically acceptable salts thereof.

21. 5H-5-(4-cyanophenyl)-6,7-dihydropyrrolo[1,2-c]-imidazole according to claim 5, a stereoisomer thereof, mixtures of these stereoisomers, and pharmaceutically acceptable salts thereof.

22. 5H-5-(4-cyanophenyl)-6,7,8,9-tetrahydroimidazo-[1,5-a]azepine according to claim 5, a stereoisomer thereof, mixtures of these stereoisomers, and pharmaceutically acceptable salts thereof.

23. 5-(4-cyanophenyl)-6-ethoxycarbonylmethyl-5,6,7,8-tetrahydroimidazo[1,5-a]-pyridine according to claim 5, a stereoisomer thereof, mixtures of these stereoisomers, and pharmaceutically acceptable salts thereof.

24. 5-(4-cyanophenyl)-6-carboxymethyl-5,6,7,8-tetrahydroimidazo[1,5-a]-pyridine according to claim 5, a stereoisomer thereof, mixtures of these stereoisomers, and pharmaceutically acceptable salts thereof.

25. 5-benzyl-5-(4-cyanophenyl)-5,6,7,8-tetrahydroimidazo-[1,5-a]pyridine according to claim 8, a stereoisomer thereof, mixtures of these stereoisomers, and pharmaceutically acceptable salts thereof.

26. 7-(p-cyanophenyl)-5,6,7,8-tetrahydroimidazo[1,5-a]-pyridine, a stereoisomer thereof, mixtures of these stereoisomers, and pharmaceutically acceptable salts thereof.

27. Pharmaceutical preparations containing a compound according to claim 5 together with one or more

pharmaceutically acceptable carriers.

**28.** Pharmaceutical preparations containing a compound according to claim 8 together with one or more pharmaceutically acceptable carriers.

**29.** Pharmaceutical preparations containing a compound according to claim 12 together with one or more pharmaceutically acceptable carriers.

**30.** Pharmaceutical preparations containing a compound according to claim 13 together with one or more pharmaceutically acceptable carriers.

**31.** Pharmaceutical preparations containing a compound according to claim 16 together with one or more pharmaceutically acceptable carriers.

**32.** Pharmaceutical preparations containing a compound according to claim 17 together with one or more pharmaceutically acceptable carriers.

**33.** A compound according to claim 5 for use in a method for the therapeutic treatment of the animal or human body.

**34.** A compound according to claim 8 for use in a method for the therapeutic treatment of the animal or human body.

**35.** A compound according to claim 12 for use in a method for the therapeutic treatment of the animal or human body.

**36.** A compound according to claim 13 for use in a method for the therapeutic treatment of the animal or human body.

**37.** A compound according to claim 16 for use in a method for the therapeutic treatment of the animal or human body.

**38.** A compound according to claim 17 for use in a method for the therapeutic treatment of the animal or human body.

**39.** A compound according to claim 5 for use as aromatase inhibitor.

**40.** A compound according to claim 8 for use as aromatase inhibitor.

**41.** A compound according to claim 12 for use as aromatase inhibitor.

**42.** A compound according to claim 16 for use as aromatase inhibitor.

**43.** The use of a compound according to claim 5 for the manufacture of pharmaceutical preparations.

**44.** The use of a compound according to claim 8 for the manufacture of pharmaceutical preparations.

**45.** The use of a compound according to claim 12 for the manufacture of pharmaceutical preparations.

**46.** The use of a compound according to claim 16 for the manufacture of pharmaceutical preparations.

**47.** The use of a compound according to claim 17 for the manufacture of pharmaceutical preparations.

**48.** The use of a compound according to claim 5 for the manufacture of pharmaceutical preparations for the treatment of diseases responsive to aromatase inhibition.

**49.** The use of a compound according to claim 8 for the manufacture of pharmaceutical preparations for the treatment of diseases responsive to aromatase inhibition.

**50.** The use of a compound according to claim 12 for the manufacture of pharmaceutical preparations for the treatment of diseases responsive to aromatase inhibition.

**51.** The use of a compound according to claim 13 for the manufacture of pharmaceutical preparations for the treatment of diseases responsive to aromatase inhibition.

**52.** The use of a compound according to claim 16 for the manufacture of pharmaceutical preparations for the treatment of diseases responsive to aromatase inhibition.

**53.** A process for the manufacture of compounds of formula I or I* according to claim 5, or salts thereof, which comprises

a) cyclising a compound of formula II

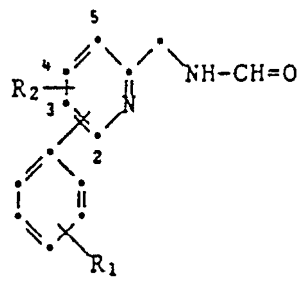

(II)

or a 4,5-dihydro derivative thereof in order to obtain a compound of formula I or a 7,8-dihydro derivative thereof, respectively, or

b) cyclising a compound of formula III

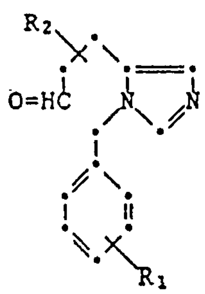

(III),

wherein $R_2$ may, as indicated, be attached to any of the carbon atoms including the carbonyl carbon, in order to obtain a 7,8-dihydro derivative of a compound of formula I wherein the substituent $C_6H_4$-$R_1$ is attached in the 5-position, or

c)/f) in a compound of formula IV or VII

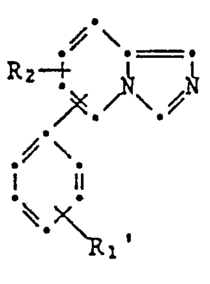

(IV)

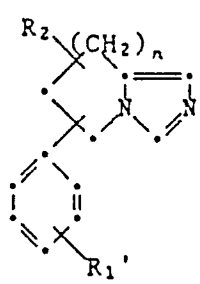

(VII)

or in a 7,8-dihydro derivative of formula IV, in each of which $R_1$' is a group that can be converted into cyano, converting $R_1$' into cyano, in order to obtain a compound of formula I, a 7,8-dihydro derivative thereof or a compound of formula I*, respectively, wherein $R_1$ represents cyano, or

d) cyclising a compound of formula V

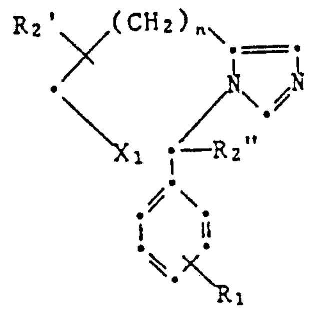

(V),

wherein at least one of the radicals $R_2$' and $R_2$" is hydrogen and the other represents a radical $R_2$ as defined under formula I*, and $X_1$ is a leaving group, and $R_2$' may, as indicated, be attached to any of the carbon atoms, in order to obtain a compound of formula I* wherein the substituent $C_6H_4$-$R_1$ is attached in the 5-position; or $X_1$ represents a =CH-COOH group or a lower alkyl ester thereof, $R_2$' is hydrogen and $R_2$" is as defined under formula I*, in order to obtain a compound of formula I* wherein the substituent $C_6H_4$-$R_1$ is attached in the 5-position and the 6-position is substituted by carboxymethyl or lower alkoxycarbonylmethyl, or

e) cyclising a compound of formula VI

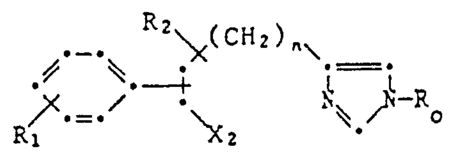

(VI)

wherein the substituents $C_6H_4$-$R_1$ and $R_2$ may, as indicated, be attached to any of the carbon atoms, either both radicals to the same carbon atom or to different carbon atoms, $R_0$ is an NH-protecting group or hydrogen, and $X_2$ is a leaving group, in order to obtain a compound of formula I*; or

g) cyclising a compound of formula IX

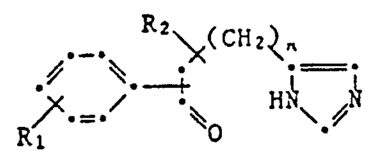

(IX)

wherein the substituents $C_6H_4$-$R_1$ and $R_2$ may, as indicated, be attached to any of the carbon atoms including the carbonyl carbon, either both radicals to the same carbon atom or to different carbon atoms, optionally under reductive conditions, in order to obtain a 7,8-dihydro derivative of a compound of formula I or, in the case of reductive conditions, a compound of formula I*, or

h) decarboxylating a compound analogous to formula I, or a 7,8-dihydro derivative thereof, or a compound analogous to formula I*, each of which contains an additional carboxy group in the 1- or 3-position, in order to obtain a compound of formula I, a 7,8-dihydro derivative thereof or a compound of formula I*; wherein, in the starting materials of formulae II to VII and IX, the symbols n, $R_1$ and $R_2$ have the meanings given under formulae I and I*, respectively; and/or reducing a compound of formula I or a 7,8-dihydro derivative thereof to a corresponding 5,6,7,8-tetrahydro derivative of formula I* optionally with simultaneous reduction of the substituent(s) $R_1$ and/or $R_2$ to (an) other substituent(s) $R_1$ and/or $R_2$; and/or decarboxylating a compound of formula I* wherein $R_2$ is carboxy, in order to obtain a compound of formula I* wherein $R_2$ is hydrogen; and/or converting a compound obtained into another compound of the invention and/or converting a salt obtained into the free compound or into another salt and/or converting a free compound obtained into a salt and/or separating a mixture of isomers or racemates obtained into the individual isomers or racemates and/or resolving an enantiomeric mixture, such as a racemate, into the optical antipodes.

**54.** A process for the manufacture of compounds of formula Ia or Ib according to claim 8, or salts thereof, which comprises

a) cyclising a compound of formula IIa

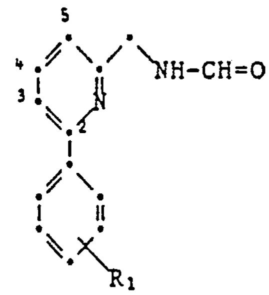

(IIa),

wherein $R_1$ is as defined above under formula Ia, or a 4,5-dihydro derivative thereof, under acidic conditions, in order to obtain a compound of formula Ia or a 7,8-dihydro derivative thereof, or
b) for the manufacture of a 7,8-dihydro derivative of a compound of formula Ia, cyclising a compound of formula IIIa

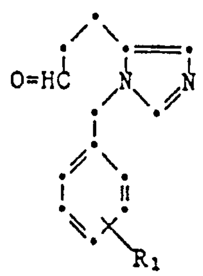

(IIIa),

wherein $R_1$ is as defined above under formula Ia, under basic conditions, or
c) in a compound of formula IVa

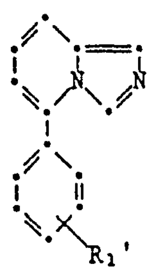

(IVa),

wherein $R_1'$ is a radical that can be converted into cyano, or in a 7,8-dihydro derivative thereof, converting $R_1'$ into cyano, in order to obtain a compound of formula Ia, or a 7,8-dihydro derivative thereof, or
d) cyclising a compound of formula Vb

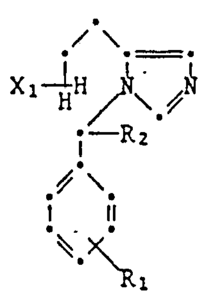

(Vb),

wherein $R_1$ and $R_2$ are as defined above under formula Ib and $X_1$ is a leaving group, in the presence of a base, in order to obtain a compound of formula Ib, or
e) cyclising a compound of formula VIb

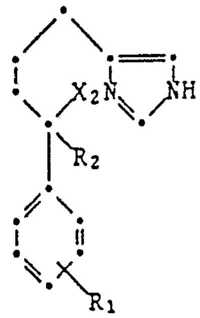

(VIb),

wherein $R_1$ and $R_2$ are as defined above under formula Ib and $X_2$ is a leaving group, and wherein the NH group may be protected by an NH-protecting group, in the presence of a base, in order to obtain a compound of formula Ib, or

f) in a compound of formula VIIb

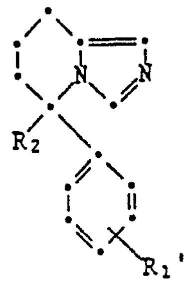

(VIIb),

wherein $R_1'$ is a radical that can be converted into cyano, and wherein $R_2$ is as defined above under formula Ib, converting the radical $R_1'$ into cyano, in order to obtain a compound of formula Ib wherein $R_1$ is cyano;

g) cyclising a compound of formula IXb

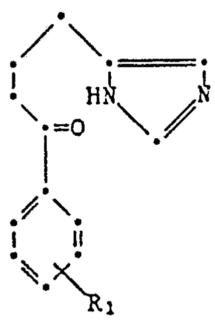

(IXb),

optionally under reductive conditions, in order to obtain a 7,8-dihydro derivative of a compound of formula Ia or, in the case of reductive conditions, a compound of formula Ib, or

h) decarboxylating a compound analogous to formula Ia, or a 7,8-dihydro derivative thereof, or a compound analogous to formula Ib, each of which contains an additional carboxy group in the 1- or 3-position, in order to obtain a compound of formula Ia, a 7,8-dihydro derivative thereof or a compound of formula Ib, and/or decarboxylating a compound of formula Ib wherein $R_2$ is carboxy in order to obtain another compound of formula Ib wherein $R_2$ is hydrogen, and/or reducing a compound of formula Ia, or a 7,8-dihydro derivative thereof, with hydrogen in the presence of a hydrogenation catalyst to the corresponding 5,6,7,8-tetrahydro derivative of formula Ib, and/or converting a compound obtained into another compound of the invention and/or converting a salt obtained into the free compound or into another salt and/or converting a free compound having a salt-forming group into a salt and/or separating a racemic mixture obtained into the individual enantiomers.

**55.** The compounds obtainable according to the process of claim 53.

**56.** The compounds obtainable according to the process of claim 54.

Claims for the following Contracting state: AT.

**1.** A process for the manufacture of compounds of formula I

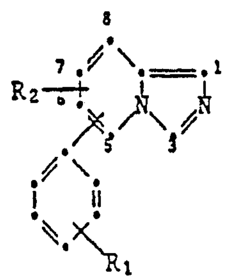

(I)

wherein $R_1$ represents hydrogen, lower alkyl; $C_2$-$C_7$alkyl substituted by hydroxy, lower alkoxy, halogen or lower alkanoyloxy; lower alkyl substituted by lower alkanoyl, amino, lower alkylamino, di-lower alkylamino, sulfo, lower alkoxycarbonyl, carbamoyl or cyano; nitro, lower alkoxy, lower alkanoyloxy, phenylsulfonyloxy, lower alkylsulfonyloxy, lower alkylthio, lower alkylsulfinyl, lower alkylsulfonyl, lower alkanoylthio, amino, lower alkylamino, di-lower alkylamino, lower alkyleneamino, N-morpholino, N-thiomorpholino, optionally 4-lower alkyl-substituted N-piperazino, tri-lower alkylammonio, sulfo, lower alkoxysulfonyl, sulfamoyl, lower alkylsulfamoyl, di-lower alkylsulfamoyl; iminomethyl optionally N-substituted by hydroxy, lower alkoxy, lower alkanoyloxy, lower alkyl, phenyl or amino; $C_2$-$C_7$alkanoyl or benzoyl; and $R_2$ represents hydrogen, lower alkyl, phenyl-lower alkyl, carboxy-lower alkyl, lower alkoxycarbonyl-lower alkyl, halogen, hydroxy, lower alkoxy, lower alkanoyloxy, mercapto, lower alkylthio, phenyl-lower alkylthio, phenylthio, lower alkanoylthio, carboxy, lower alkoxycarbonyl or lower alkanoyl; the 7,8-dihydro derivatives thereof and also such 7,8-dihydro derivatives wherein $R_1$ is hydroxymethyl, lower alkoxymethyl, halomethyl, lower alkanoyloxymethyl, carboxy-lower alkyl, halogen, hydroxy, mercapto, formyl, carboxy, lower alkoxycarbonyl, car bamoyl, lower alkylcarbamoyl, di-lower alkylcarbamoyl, cyano, 5-tetrazolyl, optionally lower alkyl-substituted 4,5-dihydro-2-oxazolyl or hydroxycarbamoyl and $R_2$ is as defined above under formula I; and compounds of formula I*

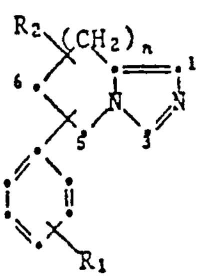

(I*)

wherein n denotes 0, 1, 2, 3 or 4; $R_1$ is as defined above under formula I or $R_1$ may be, in addition, hydroxymethyl, lower alkoxymethyl, halomethyl, lower alkanoyloxymethyl, carboxy-lower alkyl, halogen, hydroxy, mercapto, formyl, carboxy, lower alkoxycarbonyl, carbamoyl, lower alkylcarbamoyl, di-lower alkylcarbamoyl, cyano, 5-tetrazolyl, optionally lower alkyl-substituted 4,5-dihydro-2-oxazolyl or hydroxycarbamoyl if n represents 0, 1, 3 or 4 or if n represents 2 and $R_2$ is phenyl-lower alkyl, carboxy-lower alkyl, lower alkoxycarbonyl-lower alkyl, lower alkanoyloxy, mercapto, lower alkylthio, phenyl-lower alkylthio, phenylthio, lower alkanoylthio, carboxy, lower alkoxycarbonyl or lower alkanoyl; and $R_2$ is as defined above under formula I; it being possible for the phenyl ring within the radicals phenylsulfonyloxy, phenyliminomethyl, benzoyl, phenyl-lower alkyl, phenyl-lower alkylthio and phenylthio to be unsubstituted or substituted by lower alkyl, lower alkoxy or halogen; and in a compound of formula I* it being possible for the two substituents $C_6H_4$-$R_1$ and $R_2$ to be attached to any of the saturated carbon atoms of the saturated ring, either both to the same carbon atom or to different carbon atoms; radicals designated "lower" containing up to 7 carbon atoms; stereoisomers, mixtures of these stereoisomers, and salts thereof, which comprises

a) cyclising a compound of formula II

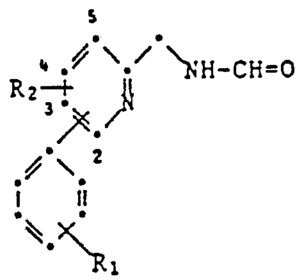

(II)

or a 4,5-dihydro derivative thereof in order to obtain a compound of formula I or a 7,8-dihydro derivative thereof, respectively, or
b) cyclising a compound of formula III

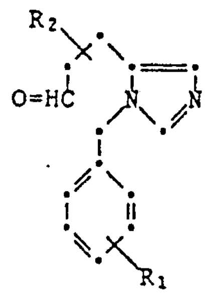

(III),

wherein $R_2$ may, as indicated, be attached to any of the carbon atoms including the carbonyl carbon, in order to obtain a 7,8-dihydro derivative of a compound of formula I wherein the substituent $C_6H_4$-$R_1$ is attached in the 5-position, or
c)/f) in a compound of formula IV or VII

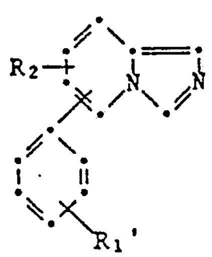

(IV)

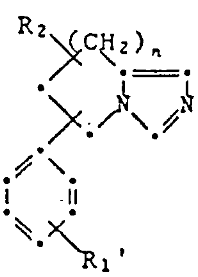

(VII)

or in a 7,8-dihydro derivative of formula IV, in each of which $R_1'$ is a group that can be converted into cyano, converting $R_1'$ into cyano, in order to obtain a compound of formula I, a 7,8-dihydro derivative thereof or a compound of formula I*, respectively, wherein $R_1$ represents cyano, or
d) cyclising a compound of formula V

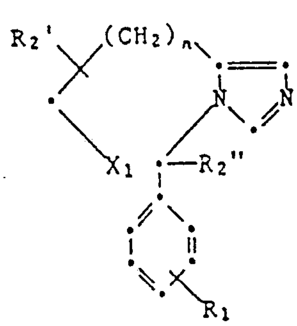

(V),

wherein at least one of the radicals $R_2'$ and $R_2''$ is hydrogen and the other represents a radical $R_2$ as defined under formula I*, and $X_1$ is a leaving group, and $R_2'$ may, as indicated, be attached to any

of the carbon atoms, in order to obtain a compound of formula I* wherein the substituent $C_6H_4$-$R_1$ is attached in the 5-position; or $X_1$ represents a =CH-COOH group or a lower alkyl ester thereof, $R_2'$ is hydrogen and $R_2''$ is as defined under formula I*, in order to obtain a compound of formula I* wherein the substituent $C_6H_4$-$R_1$ is attached in the 5-position and the 6-position is substituted by carboxymethyl or lower alkoxycarbonylmethyl, or

e) cyclising a compound of formula VI

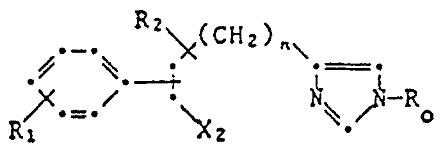

(VI)

wherein the substituents $C_6H_4$-$R_1$ and $R_2$ may, as indicated, be attached to any of the carbon atoms, either both radicals to the same carbon atom or to different carbon atoms, $R_0$ is an NH-protecting group or hydrogen, and $X_2$ is a leaving group, in order to obtain a compound of formula I*; or

g) cyclising a compound of formula IX

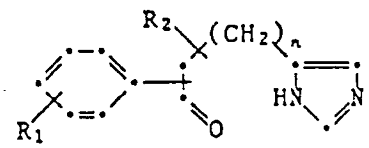

(IX)

wherein the substituents $C_6H_4$-$R_1$ and $R_2$ may, as indicated, be attached to any of the carbon atoms including the carbonyl carbon, either both radicals to the same carbon atom or to different carbon atoms, optionally under reductive conditions, in order to obtain a 7,8-dihydro derivative of a compound of formula I or, in the case of reductive conditions, a compound of formula I*, or

h) decarboxylating a compound analogous to formula I, or a 7,8-dihydro derivative thereof, or a compound analogous to formula I*, each of which contains an additional carboxy group in the 1- or 3-position, in order to obtain a compound of formula I, a 7,8-dihydro derivative thereof or a compound of formula I*; wherein, in the starting materials of formulae II to VII and IX, the symbols n, $R_1$ and $R_2$ have the meanings given under formulae I and I*, respectively; and/or reducing a compound of formula I or a 7,8-dihydro derivative thereof to a corresponding 5,6,7,8-tetrahydro derivative of formula I* optionally with simultaneous reduction of the substituent(s) $R_1$ and/or $R_2$ to (an)other substituent(s) $R_1$ and/or $R_2$; and/or decarboxylating a compound of formula I* wherein $R_2$ is carboxy, in order to obtain a compound of formula I* wherein $R_2$ is hydrogen; and/or converting a compound obtained into another compound of the invention and/or converting a salt obtained into the free compound or into another salt and/or converting a free compound obtained into a salt and/or separating a mixture of isomers or racemates obtained into the individual isomers or racemates and/or resolving an enantiomeric mixture, such as a racemate, into the optical antipodes.

**2.** A process according to claim 1, wherein compounds of formula I wherein $R_1$ represents lower alkyl, hydroxy-$C_2$-$C_7$alkyl; lower alkyl substituted by amino, di-lower alkylamino, 2 to 5 fluorine atoms, lower alkoxycarbonyl, carbamoyl or cyano; nitro, lower alkoxy, amino, lower alkylamino, di-lower alkylamino, sulfo, sulfamoyl, iminomethyl or iminomethyl N-substituted by hydroxy, lower alkoxy, lower alkanoyloxy, lower alkyl or phenyl; and $R_2$ is hydrogen, lower alkyl, lower alkoxy or halogen; or compounds of formula I* wherein n denotes 1, 2 or 3; $R_1$ is as defined above under formula I or $R_1$ may be, in addition, hydroxymethyl, carboxy-lower alkyl, halogen, hydroxy, formyl, carboxy, lower alkoxycarbonyl, carbamoyl, lower alkylcarbamoyl, di-lower alkylcarbamoyl or cyano if n represents 1 or 3 or if n represents 2 and $R_2$ is phenyl-lower alkyl, carboxy-lower alkyl, lower alkoxycarbonyl-lower alkyl, lower alkylthio, phenyl-lower alkylthio, phenylthio, carboxy, lower alkoxycarbonyl or lower alkanoyl; and $R_2$ represents hydrogen, lower alkyl, phenyl-lower alkyl, carboxy-lower alkyl, lower alkoxycarbonyl-lower alkyl, halogen, lower alkoxy, lower alkylthio, phenyl-lower alkylthio, phenylthio, carboxy, lower alkoxycarbonyl or lower alkanoyl; in a compound of formula I* it being possible for the two substituents $C_6H_4$-$R_1$ and $R_2$ to be attached to any of the saturated carbon atoms of the saturated ring, either both to the same carbon atom or to different carbon atoms; stereoisomers, mixtures of these stereoisomers, or pharmaceutically acceptable salts thereof, are manufactured.

**3.** A process according to claim 1, wherein compounds of formula I* wherein n denotes 1, 2 or 3; $R_1$ represents lower alkyl, amino, lower alkylamino or di-lower alkylamino, or $R_1$ may be, in addition, hydroxymethyl, halogen, formyl, carboxy, lower alkoxycarbonyl, carbamoyl, lower alkylcarbamoyl, di-lower alkylcarbamoyl or cyano if n represents 1 or 3 or if n represents 2 and $R_2$ is phenyl-lower alkyl, carboxy-lower alkyl, lower alkoxycarbonyl-lower alkyl, lower alkylthio, carboxy or lower alkoxycarbonyl; and $R_2$ is hydrogen, lower alkyl, phenyl-lower alkyl, carboxy-lower alkyl, lower alkoxycarbonyl-lower alkyl, lower alkylthio, carboxy or lower alkoxycarbonyl; it being possible for the two substituents $C_6H_4$-$R_1$ and $R_2$ to be attached to any of the saturated carbon atoms of the saturated ring, either both to the same carbon atom or to different carbon atoms; stereoisomers, mixtures of these stereoisomers, or pharmaceutically acceptable salts thereof, are manufactured.

**4.** A process for the manufacture of compounds of formula Ia

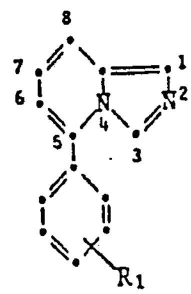

(Ia)

wherein $R_1$ represents cyano, nitro or $C_1$-$C_4$alkyl, the 7,8-dihydro derivatives thereof or the 5,6,7,8-tetrahydro derivatives thereof of formula Ib

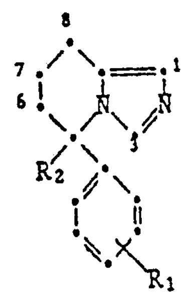

(Ib)

wherein $R_1$ is as defined above under formula Ia and $R_2$ is hydrogen, $C_1$-$C_4$alkyl, benzyl, halogen, $C_1$-$C_4$alkoxy, $C_1$-$C_4$alkylthio, benzylthio, 2-phenylethylthio, diphenylmethylthio, phenylthio, $C_1$-$C_4$-alkanoyloxy, $C_1$-$C_4$alkanoylthio, carboxy-$C_1$-$C_4$alkyl, $C_1$-$C_4$alkoxycarbonyl-$C_1$-$C_4$alkyl or $C_1$-$C_4$alkanoyl, stereoisomers, mixtures of these stereoisomers, and salts of these compounds, which comprises

a) cyclising a compound of formula IIa

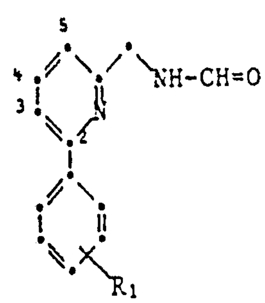

(IIa),

wherein $R_1$ is as defined above under formula Ia, or a 4,5-dihydro derivative thereof, under acidic conditions, in order to obtain a compound of formula Ia or a 7,8-dihydro derivative thereof, or

b) for the manufacture of a 7,8-dihydro derivative of a compound of formula Ia, cyclising a

compound of formula IIIa

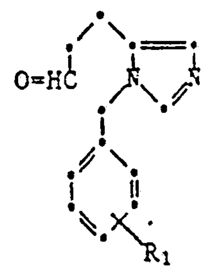

(IIIa),

wherein $R_1$ is as defined above under formula Ia, under basic conditions, or

c) in a compound of formula IVa

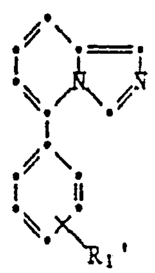

(IVa),

wherein $R_1$' is a radical that can be converted into cyano, or in a 7,8-dihydro derivative thereof , converting $R_1$' into cyano, in order to obtain a compound of formula Ia, or a 7,8-dihydro derivative thereof, or

d) cyclising a compound of formula Vb

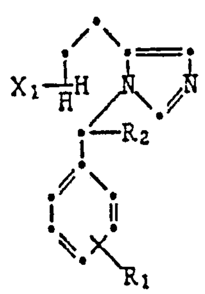

(Vb),

wherein $R_1$ and $R_2$ are as defined above under formula Ib and $X_1$ is a leaving group, in the presence of a base, in order to obtain a compound of formula Ib, or

e) cyclising a compound of formula VIb

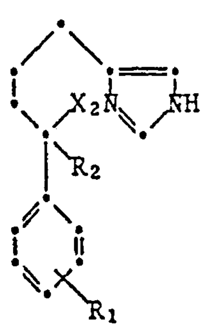

(VIb),

wherein $R_1$ and $R_2$ are as defined above under formula Ib and $X_2$ is a leaving group, and wherein the NH group may be protected by an NH-protecting group, in the presence of a base, in order to obtain a compound of formula Ib, or

f) in a compound of formula VIIb

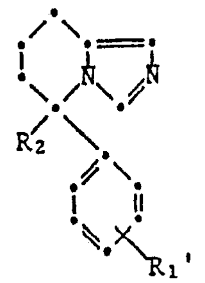

(VIIb),

wherein $R_1$' is a radical that can be converted into cyano, and wherein $R_2$ is as defined above under formula Ib, converting the radical $R_1$' into cyano, in order to obtain a compound of formula Ib wherein $R_1$ is cyano;

g) cyclising a compound of formula IXb

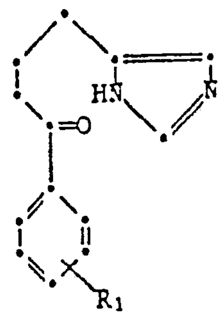

(IXb),

optionally under reductive conditions, in order to obtain a 7,8-dihydro derivative of a compound of formula Ia or, in the case of reductive conditions, a compound of formula Ib, or

h) decarboxylating a compound analogous to formula Ia, or a 7,8-dihydro derivative thereof, or a compound analogous to formula Ib, each of which contains an additional carboxy group in the 1- or 3-position, in order to obtain a compound of formula Ia, a 7,8-dihydro derivative thereof or a compound of formula Ib, and/or decarboxylating a compound of formula Ib wherein $R_2$ is carboxy in order to obtain another compound of formula Ib wherein $R_2$ is hydrogen, and/or reducing a compound of formula Ia, or a 7,8-dihydro derivative thereof, with hydrogen in the presence of a hydrogenation catalyst to the corresponding 5,6,7,8-tetrahydro derivative of formula Ib, and/or converting a compound obtained into another compound of the invention and/or converting a salt obtained into the free compound or into another salt and/or converting a free compound having a salt-forming group into a salt and/or separating a racemic mixture obtained into the individual enantiomers.

**5.** A process according to claim 4, which comprises carrying out one of the process variants a), b), c), d) or f) specified therein or e) cyclising a compound of formula VIb

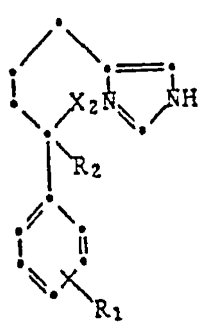

(VIb),

wherein $R_1$ and $R_2$ are as defined above under formula Ib and $X_2$ is a leaving group, in the presence of

a base, in order to obtain a compound of formula Ib, and/or reducing a compound of formula Ia, or a 7,8-dihydro derivative thereof, with hydrogen in the presence of a hydrogenation catalyst to the corresponding 5,6,7,8-tetrahydro derivative of formula Ib, and/or converting a compound obtained into another compound of the invention and/or converting a salt obtained into the free compound or into another salt and/or converting a free compound having a salt-forming group into a salt and/or separating a racemic mixture obtained into the individual enantiomers.

6. A process according to claim 5, wherein compounds of formula Ia wherein $R_1$ represents cyano, the 7,8-dihydro derivatives thereof, or the 5,6,7,8-tetrahydro derivatives thereof of formula Ib wherein $R_1$ is cyano and $R_2$ is hydrogen, $C_1$-$C_4$alkyl, $C_1$-$C_4$alkoxy, $C_1$-$C_4$alkylthio, benzylthio, 2-phenylethylthio, diphenylmethylthio, phenylthio or $C_1$-$C_4$alkanoyl, or pharmaceutically acceptable acid addition salts of compounds of formulae Ia and Ib are manufactured.

7. A process according to claim 5, wherein compounds of formula Ia wherein $R_1$ is cyano, the 7,8-dihydro derivatives thereof, or the 5,6,7,8-tetrahydro derivatives thereof of formula Ib wherein $R_1$ is as defined above under formula Ia and $R_2$ is hydrogen, or pharmaceutically acceptable acid addition salts thereof are manufactured.

8. A process according to claim 5, wherein compounds of formula Ic

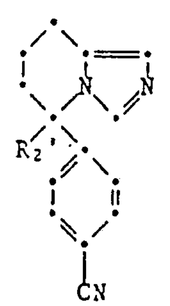

(Ic)

wherein $R_2$' is hydrogen, $C_1$-$C_4$alkyl, $C_1$-$C_4$alkoxy, $C_1$-$C_4$alkylthio, benzylthio, 2-phenylethylthio, diphenylmethylthio, phenylthio or $C_1$-$C_4$alkanoyl, or pharmaceutically acceptable acid addition salts thereof are manufactured.

9. A process according to claim 5, wherein a compound of formula Ic wherein $R_2$' is hydrogen, being 5-(p-cyanophenyl)5,6,7,8-tetrahydroimidazo[1,5-a]pyridine, or pharmaceutically acceptable acid addition salts thereof are manufactured.

10. A process according to claim 4, wherein a compound of formula Ic wherein $R_2$' is hydrogen, being 5-(p-cyanophenyl)-5,6,7,8-tetrahydroimidazo[1,5-a]pyridine, or pharmaceutically acceptable acid addition salts thereof are manufactured.

11. A process for the manufacture of compounds of formula Ia as shown in claim 4, wherein $R_1$ represents hydrogen, p-toluenesulfonyloxy, benzenesulfonyloxy, methylsulfonyloxy, sulfo, amino, carbamoyl, $C_1$-$C_4$alkylcarbamoyl or hydroxyiminomethyl; or the 5,6,7,8-tetrahydro compounds of formula Ib as shown in claim 4, wherein $R_1$ is as defined above under formula Ia or is halogen, and $R_2$ is hydrogen, $C_1$-$C_4$alkyl, benzyl, halogen, $C_1$-$C_4$alkoxy, $C_1$-$C_4$alkanoyloxy, $C_1$-$C_4$alkylthio, benzylthio, 2-phenylethylthio, diphenylmethylthio, phenylthio, $C_1$-$C_4$alkanoylthio, carboxy-$C_1$-$C_4$alkyl, $C_1$-$C_4$alkoxycarbonyl-$C_1$-$C_4$alkyl or $C_1$-$C_4$alkanoyl; or pharmaceutically acceptable salts thereof, which comprises carrying out the process variants specified in claim 4.

12. A process for the manufacture of compounds of formula Ia as shown in claim 4, wherein $R_1$ is hydrogen, p-toluenesulfonyloxy, benzenesulfonyloxy, methylsul fonyloxy, sulfo, amino or hydroximinomethyl; or the 5,6,7,8-tetrahydro compounds of formula Ib as shown in claim 4, wherein $R_1$ is as defined above under formula Ia and $R_2$ is hydrogen; or wherein $R_1$ is hydrogen, halogen, p-toluenesulfonyloxy, benzenesulfonyloxy, methylsulfonyloxy, sulfo, amino, carboxy, carbamoyl, $C_1$-$C_4$alkylcarbamoyl, formyl or hydroxyiminomethyl and $R_2$ represents $C_1$-$C_4$alkyl, benzyl, halogen, $C_1$-

$C_4$alkoxy, $C_1$-$C_4$alkanoyloxy, $C_1$-$C_4$alkylthio, benzylthio, 2-phenylethylthio, diphenylmethylthio, phenylthio, $C_1$-$C_4$alkanoylthio, carboxy-$C_1$-$C_4$alkyl, $C_1$-$C_4$alkoxycarbonyl-$C_1$-$C_4$alkyl or $C_1$-$C_4$alkanoyl; or pharmaceutically acceptable salts thereof, which comprises carrying out the process variants specified in claim 5.

**13.** A process according to claim 12, wherein compounds of formulae Ia and Ib wherein $R_1$ represents carbamoyl, or compounds of formula Ib wherein $R_1$ represents halogen are manufactured.

**14.** A process according to claim 4, wherein (-)-(5-(p-cyanophenyl)-5,6,7,8-tetrahydroimidazo[1,5-a]pyridine or pharmaceutically acceptable salts thereof are manufactured.

**15.** A process according to claim 4, wherein (+)-(5-(p-cyanophenyl)-5,6,7,8-tetrahydroimidazo[1,5-a]-pyridine or pharmaceutically acceptable salts thereof are manufactured.

**16.** A process according to claim 5, wherein 5-(p-cyanophenyl)-7,8-dihydroimidazo[1,5-a]pyridine or pharmaceutically acceptable salts thereof are manufactured.

**17.** A process according to claim 5, wherein 5-(p-cyanophenyl)imidazo[1,5-a]pyridine or pharmaceutically acceptable salts thereof are manufactured.

**18.** A process according to claim 11, wherein 5-(p-bromophenyl)-5,6,7,8-tetrahydroimidazo[1,5-a]pyridine, a stereoisomer thereof, mixtures of these stereoisomers, or pharmaceutically acceptable salts thereof are manufactured.

**19.** A process according to claim 1, wherein 5H-5-(4-cyanophenyl)-6,7-dihydropyrrolo[1,2-c]imidazole, a stereoisomer thereof, mixtures of these stereoisomers, or pharmaceutically acceptable salts thereof are manufactured.

**20.** A process according to claim 1, wherein 5H-5-(4-cyanophenyl)-6,7,8,9-tetrahydroimidazo[1,5-a]azepine, a stereoisomer thereof, mixtures of these stereoisomers, or pharmaceutically acceptable salts thereof are manufactured.

**21.** A process according to claim 1, wherein 5-(4-cyanophenyl )-6-ethoxycarbonylmethyl-5,6,7,8-tetrahydroimidazo[1,5-a]pyridine, a stereoisomer thereof, mixtures of these stereoisomers, or pharmaceutically acceptable salts thereof are manufactured.

**22.** A process according to claim 4, wherein 5-(p-cyanophenyl)imidazo[1,5-a]pyridine or pharmaceutically acceptable salts thereof are manufactured.

**23.** A process according to claim 12, wherein 5-(p-bromophenyl)-5,6,7,8-tetrahydroimidazo[1,5-a]pyridine, a stereoisomer thereof, mixtures of these stereoisomers or pharmaceutically acceptable salts thereof are manufactured.

**24.** A process according to claim 1, wherein 5-(4-cyanophenyl)-6-carboxymethyl-5,6,7,8-tetrahydroimidazo-[1,5-a]pyridine, a stereoisomer thereof, mixtures of these stereoisomers, or pharmaceutically acceptable salts thereof are manufactured.

**25.** A process according to claim 4, wherein 5-benzyl-5-(4-cyanophenyl)-5,6,7,8-tetrahydroimidazo[1,5-a]-pyridine, a stereoisomer thereof, mixtures of these stereoisomers, or pharmaceutically acceptable salts thereof are manufactured.

**26.** A process for the manufacture of 7-(p-cyanophenyl)-5,6,7,8-tetrahydroimidazo[1,5-a]pyridine, a stereoisomer thereof, mixtures of these stereoisomers or pharmaceutically acceptable salts thereof, which comprises carrying out the process variants specified in claim 1.

**27.** A process for the manufacture of pharmaceutical preparations, which comprises mixing a compound obtained by the process according to any one of claims 1, 4, 10, 11, 14 and I5, or a pharmaceutically acceptable salt of such a compound, with a pharmaceutically acceptable carrier.

28. A process for the manufacture of pharmaceutical preparations, which comprises mixing a compound obtained by the process according to any one of claims 5, 9 and 12, or a pharmaceutically acceptable salt of such a compound, with a pharmaceutically acceptable carrier.

## Revendications

1. Utilisation des dérivés substitués d'imidazo [1,5-a] pyridines de formule I

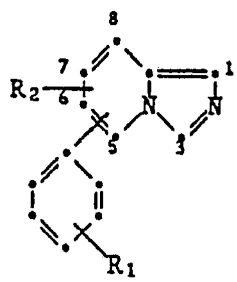

(I),

dans laquelle $R_1$ représente l'hydrogène, un groupe alkyle inférieur; un groupe alkyle inférieur substitué par des groupes hydroxy, alcoxy inférieur, alcanoyloxy inférieur, alcanoyle inférieur, amino, alkylamino inférieur, di-(alkyle inférieur)-amino, des halogènes, des groupes sulfo, carboxy, (alcoxy inférieur)-carbonyle, carbamoyle ou cyano; un groupe nitro, un halogène, un groupe hydroxy, alcoxy inférieur, alcanoyloxy inférieur, phénylsulfonyloxy, alkylsulfonyloxy inférieur, mercapto, alkylthio inférieur, alkylsulfinyle inférieur, alkylsulfonyle inférieur, alcanoylthio inférieur, amino, alkylamino inférieur, di-(alkyle inférieur)-amino, alkylène-amino inférieur, N-morpholino, N-thiomorpholino, N-pipérazino éventuellement substitué en position 4 par un groupe alkyle inférieur, tri-(alkyle inférieur)-ammonio, sulfo, alcoxysulfonyle inférieur, sulfamoyle, alkylsulfamoyle inférieur, di-(alkyle inférieur)-sulfamoyle, formyle; un groupe iminométhyle éventuellement substitué sur l'azote par un groupe hydroxy, alcoxy inférieur, alcanoyloxy inférieur, alkyle inférieur, phényle ou amino; un groupe alcénoyle en C2-C7, benzoyle, carboxy, (alcoxy inférieur)-carbonyle, carbamoyle, (alkyle inférieur)-carbamoyle, di-(alkyle inférieur)-carbamoyle, cyano, 5-tétrazolyle, 4,5-dihydro-2-oxazolyle ou hydroxycarbamoyle éventuellement substitué par un groupe alkyle inférieur; et $R_2$ représente l'hydrogène, un groupe alkyle inférieur, phényl-alkyle inférieur, carboxy-alkyle inférieur, (alcoxy inférieur)-carbonyl-alkyle inférieur, un halogène, un groupe hydroxy, alcoxy inférieur, alcanoyloxy inférieur, mercapto, alkylthio inférieur, phényl-alkylthio inférieur,-phénylthio, alcanoylthio inférieur, carboxy, (alcoxy inférieur)-carbonyle ou alcanoyle inférieur; de leurs dérivés 7,8-dihydrogénés; ou de composés de formule I*

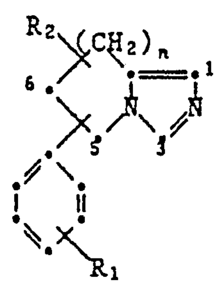

(I*),

dans laquelle n est égal à 0, 1, 2, 3 ou 4; et $R_1$ et $R_2$ ont les significations indiquées en référence à la formule I ; le noyau phényle des groupes phénylsulfonyloxy, phényliminométhyle, benzoyle, phénylalkyle inférieur, phénylalkylthio inférieur et phénylthio pouvant être non substitué ou substitué par des groupes alkyle inférieurs, alcoxy inférieurs ou des halogènes; les deux substituants $C_6H_4$-$R_1$ et $R_2$ d'un composé de formule I* pouvant être fixés sur chacun des atomes de carbone saturés du cycle saturé, tous deux sur le même atome de carbone ou sur des atomes de carbone différents; les substituants qualifiés d'"inférieurs" contenant jusqu'à 7-atomes de carbone; de leurs stéréoisomères, des mélanges de ces stéréoisomères ou des sels acceptables pour l'usage pharmaceutique de ces composés, pour la préparation de compositions pharmaceutiques pour le traitement de maladies demandant une

inhibition de l'aromatase.

2. Utilisation selon la revendication 1 caractérisée en ce que l'on utilise des composés de formule I dans laquelle $R_1$ représente un groupe alkyle inférieur; un groupe alkyle inférieur substitué par des groupes hydroxy, amino, di-(alkyle inférieur)-amino, 1 à 5 atomes de fluor, des groupes carboxy, (alcoxy inférieur)-carbonyle, carbamoyle ou cyano; un groupe nitro, un halogène, un groupe hydroxy, alcoxy inférieur, amino, alkylamino inférieur, di-(alkyle inférieur)-amino, sulfo, sulfamoyle, formyle, iminométhyle; un groupe iminométhyle substitué à l'azote par un groupe hydroxy, alcoxy inférieur alcanoyloxy inférieur, alkyle inférieur ou phényle; un groupe carboxy, (alcoxy inférieur)-carbonyle, carbamoyle, (alkyle inférieur)-carbamoyle, di-(alkyle inférieur)-carbamoyle ou cyano; et $R_2$ représente l'hydrogène, un groupe alkyle inférieur, alcoxy inférieur ou un halogène; ou des composés de formule I* dans laquelle n est égal à 1, 2 ou 3; $R_1$ a les significations indiquées en référence à la formule I et $R_2$ représente l'hydrogène, un groupe alkyle inférieur, phényl-alkyle inférieur, carboxy-alkyle inférieur, (alcoxy inférieur)-carbonyl-alkyle inférieur, un halogène,un groupe alcoxy inférieur, alkylthio inférieur, phényl-alkylthio inférieur, phénylthio, carboxy, (alcoxy inférieur)-carbonyle ou alcanoyle inférieur; les deux substituants $C_6H_4$-$R_1$ et $R_2$ d'un composé de formule I* pouvant être fixés sur chacun des atomes de carbone saturés du cycle saturé, soit tous deux sur le même atome de carbone, soit sur des atomes de carbone différents; leurs stéréoisomères, un mélange de ces stéréoisomères ou les sels acceptables pour l'usage pharmaceutique de ces composés.

3. Utilisation selon la revendication 1, caractérisée en ce que l'on utilise des composés de formule I dans laquelle $R_1$ représente un groupe alkyle inférieur, hydroxy alkyle inférieur, un halogène, un groupe amino, formyle, carboxy, (alcoxy inférieur)-carbonyle, carbamoyle, N-(alkyle inférieur)-carbamoyle ou cyano; et $R_2$ représente l'hydrogène; ou des composés de formule I* dans laquelle n est égal à 1, 2 ou 3; $R_1$ a les significations indiquées en référence à la formule I et $R_2$ représente l'hydrogène un groupe alkylthio inférieur, (alcoxy inférieur)-carbonyle, phényl-alkyle inférieur, carboxy-alkyle inférieur ou (alcoxy inférieur)-carbonyl-alkyle inférieur; les deux substituants $C_6H_4$-$R_1$ et $R_2$ d'un composé de formule I* pouvant être fixés sur chacun des atomes de carbone saturés du cycle saturé, soit tous deux sur le même atome de carbone soit sur des atomes de carbone différents; leurs stéréoisomères, les mélanges de ces stéréoisomeres ou les sels acceptables pour l'usage pharmaceutique de ces composés.

4. Compositions pharmaceutiques contenant, avec un ou plusieurs véhicules acceptables pour l'usage pharmaceutique, un composé de formule I de la revendication 1 dans laquelle $R_1$ représente un halogène, un groupe hydroxy, mercapto ou alkyle inférieur substitué par ces groupes alcoxy inférieur, alcanoyloxy inférieur ou des halogènes; et $R_2$ représente l'hydrogène, un groupe alkyle inférieur, alcoxy inférieur ou un halogène; ou un composé de formule I* de la revendication 1 dans laquelle n est égal à 2, $R_1$ a les significations indiquées en référence à la formule I et $R_2$ représente l'hydrogène, un groupe alkyle inférieur, phényl-alkyle inférieur, carboxy-alkyle inférieur, (alcoxy inférieur)-carbonyl-alkyle inférieur, un halogène, un groupe alcoxy inférieur, alkylthio inférieur, phényl-alkylthio inférieur, phénylthio, carboxy, (alcoxy inférieur)carbonyle ou alcanoyle inférieur; les deux substituants $C_6H_4$-$R_1$ et $R_2$ d'un composé de formule I* pouvant être fixés sur chacun des atomes de carbone saturés du cycle saturé, soit tous deux sur le même atome de carbone, soit sur des atomes de carbone différents; les substituants qualifiés d'"inférieurs" contenant jusqu'à 7 atomes de carbone; un stéréoisomère, un mélange de ces stéréoisomères ou un sel acceptable pour l'usage pharmaceutique de ces composés.

5. Composés de formule I de la revendication 1 dans laquelle $R_1$ représente l'hydrogène, un groupe alkyle inférieur; un groupe alkyle en C2-C7 substitué par des groupes hydroxy, alcoxy inférieurs, des halogènes ou des groupes alcanoyloxy inférieurs; un groupe alkyle inférieur substitué par des groupes alcanoyle inférieur, amino, alkylamino inférieur, di-(alkyle inférieur)-amino, sulfo, (alcoxy inférieur)-carbonyle, carbamoyle ou cyano; un groupe nitro, alcoxy inférieur, alcanoyloxy inférieur, phénylsulfonyloxy, alkylsulfonyloxy inférieur, alkylthio inférieur, alkylsulfinyle inférieur, alkylsulfonyle inférieur, alcanoylthio inférieur, amino, alkyl-amino inférieur, di-(alkyle inférieur)-amino, alkylène-amino inférieur, N-morpholino, N-thiomorpholino, N-pipérazino éventuellement substitué en position 4 par un groupe alkyle inférieur, tri-(alkyle inférieur)-ammonio, sulfo, alcoxysulfonyle inférieur, sulfamoyle, alkylsulfamoyle inférieur, di-(alkyle inférieur)-sulfamoyle; un groupe iminométhyle éventuellement substitué à l'azote par un groupe hydroxy, alcoxy inférieur, alcanoyloxy inférieur, alkyle inférieur, phényle ou amino; un groupe alcanoyle en C2-C7 ou benzoyle, et $R_2$ représente l'hydrogène, un groupe alkyle inférieur, phényl-

alkyle inférieur, carboxy-alkyle inférieur, (alcoxy inférieur)-carbonyl-alkyle inférieur, un halogène, un groupe hydroxy, alcoxy inférieur, alcanoyloxy inférieur, mercapto, alkylthio inférieur, phényl-alkylthio inférieur, phénylthio, alcanoylthio inférieur, carboxy, (alcoxy inférieur)-carbonyle ou alcanoyle inférieur; les dérivés 7,8-dihydrogénés de ces composés et en outre les dérivés 7,8-dihydrogénés dans lesquels $R_1$ représente un groupe hydroxyméthyle, (alcoxy inférieur)-méthyle, halogénométhyle, (alcanoyle inférieur)-oxyméthyle, carboxy-alkyle inférieur, un halogène, un groupe hydroxy, mercapto, formyle, carboxy, (alcoxy inférieur)-carbonyle, carbamoyle, (alkyle inférieur)-carbamoyle, di-(alkyle inférieur)-carbamoyle cyano, 5-tétrazolyle, 4,5-dihydro-2-oxazolyle ou hydroxycarbamoyle éventuellement substitué par un groupe alkyle inférieur et $R_2$ a les significations indiquées ci-dessus en référence à la formule I; et les composés de formule I* de la revendication 1 dans laquelle n est égal à 0, 1, 2, 3 ou 4; $R_1$ a les significations indiquées en référence à la formule I mais peut en outre représenter un groupe hydroxyméthyle, (alcoxy inférieur)-méthyle, halogénométhyle, (alcanoyle inférieur)-oxyméthyle, carboxy-alkyle inférieur, un halogène, un groupe hydroxy, mercapto, formyle, carboxy, (alcoxy inférieur)-carbonyle, carbamoyle, (alkyle inférieur)-carbamoyle, di-(alkyle inférieur)-carbamoyle, cyano, 5-tétrazolyle, 4,5-dihydro-2-oxazolyle ou hydroxycarbamoyle éventuellement substitué par un groupe alkyle inférieur lorsque n est égal à 0, 1, 3 ou 4 ou lorsque n est égal à 2 et que $R_2$ représente un groupe phényl-alkyle inférieur, carboxy-alkyle inférieur, (alcoxy inférieur)-carbonyl-alkyle inférieur, alcanoyloxy inférieur, mercapto, alkylthio inférieur, phényl-alkylthio inférieur, phénylthio, alcanoylthio inférieur, carboxy, (alcoxy inférieur)-carbonyle ou alcanoyle inférieur; et $R_2$ a les significations indiquées ci-dessus en-référence à la formule I; le noyau phényle des groupes phénylsulfonyloxy, phényliminométhyle, benzoyle, phényl-alkyle inférieur, phényl-alkylthio inférieur et phénylthio pouvant être non substitué ou substitué par des groupes alkyle inférieurs, alcoxy inférieurs ou des halogènes; les deux substituants $C_6H_4$-$R_1$ et $R_2$ d'un composé de formule I* pouvant être fixés sur chacun des atomes de carbone saturés du cycle saturé, soit tous deux sur le même atome de car bone, soit sur des atomes de carbone différents; les substituants qualifiés d'"inférieurs" contenant jusqu'à 7 atomes de carbone, leurs stéréoisomères, les mélanges de ces stéréoisomères et les sels de ces composés.

6. Composés selon la revendication 5, répondant à la formule I dans laquelle $R_1$ représente un groupe alkyle inférieur, hydroxyalkyle en C2-C7; alkyle inférieur substitué par des groupes amino, di-(alkyle inférieur)-amino, 2 à 5 atomes de fluor, des groupes (alcoxy inférieur)-carbonyle, carbamoyle ou cyano; un groupe nitro, alcoxy inférieur, amino, alkylamino inférieur, di-(alkyle inférieur)-amino, sulfo, sulfamoyle, iminométhyle ou iminométhyle substitué à l'azote par un groupe hydroxy, alcoxy inférieur, alcanoyloxy inférieur, alkyle inférieur ou phényle, et $R_2$ représente l'hydrogène, un groupe alkyle inférieur, alcoxy inférieur ou un halogène; et les composés de formule I* dans laquelle n est égal à 1, 2 ou 3; $R_1$ a les significations indiquées en référence à la formule I et peut en outre représenter un groupe hydroxyméthyle, carboxy-alkyle inférieur, un halogène, un groupe hydroxy, formyle, carboxy, (alcoxy inférieur)-carbonyle, carbamoyle, (alkyle inférieur)-carbamoyle, di-(alkyle inférieur)-carbamoyle ou cyano lorsque n est égal à 1 ou 3 ou lorsque n est égal à 2 et que $R_2$ représente un groupe phényl-alkyle inférieur, carboxy-alkyle inférieur (alcoxy inférieur)-carbonyl-alkyle inférieur, alkylthio inférieur, phényl-alkylthio inférieur,phénylthio, carboxy, (alcoxy inférieur)-carbonyle ou alcanoyle inférieur; et $R_2$ représente l'hydrogène, un groupe alkyle inférieur, phényl-alkyle inférieur, carboxy-alkyle inférieur, (alcoxy inférieur)-carbonyl-alkyle inférieur, un halogène, un groupe alcoxy inférieur, alkylthio inférieur, phénylalkylthio inférieur, phénylthio, carboxy, (alcoxy inférieur)-carbonyle ou alcanoyle inférieur; les deux substituants $C_6H_4$-$R_1$ et $R_2$ d'un composé de formule I* pouvant être fixés sur l'un quelconque des atomes de carbone saturés du cycle saturé, soit tous deux sur le même atome de carbone, soit sur des atomes de carbone différents; leurs stéréoisomères, les mélanges de ces stéréoisomères et les sels acceptables pour l'usage pharmaceutique de ces composés.

7. Composés selon la revendication 5 de formule I* dans laquelle n est égal à 1, 2 ou 3; $R_1$ représente un groupe alkyle inférieur, amino, alkylamino inférieur ou di-(alkyle inférieur)-amino mais peut en outre représenter un groupe méthyle, un halogène, un groupe formyle, carboxy, (alcoxy inférieur)-carbonyle, carbamoyle, (alkyle inférieur)-carbamoyle, di-(alkyle inférieur)-carbamoyle ou cyano lorsque n est égal à 1 ou 3 ou lorsque n est égal à 2 et que $R_2$ représente un groupe phényl-alkyle inférieur, carboxyalkyle inférieur, (alcoxy inférieur)-carbonyl-alkyle inférieur, alkylthio inférieur, carboxy ou (alcoxy inférieur)-carbonyle; et $R_2$ représente l'hydrogène, un groupe alkyle inférieur, phényl-alkyle inférieur, carboxy-alkyle inférieur, (alcoxy inférieur)-carbonylalkyle inférieur, alkylthio inférieur, carboxy ou (alcoxy inférieur)-carbonyle; les deux substituants $C_6H_4$-$R_1$ et $R_2$ pouvant être fixés sur l'un quelconque des atomes de carbone saturés du cycle saturé, soit tous deux sur le même atome de carbone, soit sur des

atomes de carbone différents; leurs stéréoisomères, les mélanges de ces stéréoisomères et les sels acceptables pour l'usage pharmaceutique de ces composés.

**8.** Composés de formule Ia

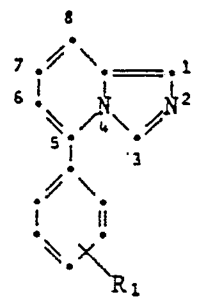

(Ia)

dans laquelle $R_1$ représente un groupe cyano, nitro ou alkyle en C1-C4 leurs dérivés 7,8-dihydrogénés et leurs dérivés 5,6,7,8-tétrahydrogénés de formule Ib

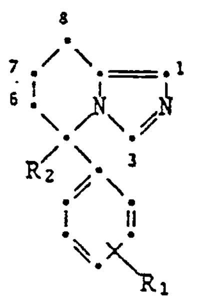

(Ib)

dans laquelle $R_1$ a les significations indiquées ci-dessus en référence à la formule Ia et $R_2$ représente l'hydrogène, un groupe alkyle en C1-C4, benzyle, un halogène, un groupe alcoxy en C1-C4, alkylthio en C1-C4, benzylthio, 2-phényléthylthio, diphénylméthylthio, phénylthio, alcanoyloxy en C1-C4, alcanoylthio en C1-C4, carboxy-alkyle en C1-C4, (alcoxy en C1-C4)-carbonyl-alkyle en C1-C4 ou alcanoyle en C1-C4, leurs stéréoisomères, les mélanges de ces stéréoisomères et les sels de ces composés.

**9.** Composés selon la revendication 8, de formule Ia dans laquelle $R_1$ représente un groupe cyano, leurs dérivés 7,8-dihydrogénés et leurs dérivés 5,6,7,8-tétrahydrogénés de formule Ib dans laquelle $R_1$ représente un groupe cyano et $R_2$ l'hydrogène, un groupe alkyle en C1-C4, alcoxy en C1-C4, alkylthio en C1-C4, benzylthio, 2-phényléthylthio, diphénylméthylthio, phénylthio ou alcanoyle en C1-C4 et les sels acceptables pour l'usage pharmaceutique, formés par addition avec des acides, des composés de formule Ia et Ib.

**10.** Composés selon la revendication 8 de formule Ia dans laquelle $R_1$ représente un groupe cyano, leurs dérivés 7,8-dihydrogénés et leurs dérivés 5,6,7,8-tétrahydrogénés de formule Ib dans laquelle $R_1$ a les significations indiquées ci-dessus en référence à la formule Ia et $R_2$ représente l'hydrogène, et leurs sels acceptables pour l'usage pharmaceutique formés par addition avec des acides.

**11.** Composés selon la revendication 8, de formule Ic

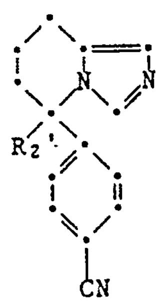

(Ic)

dans laquelle R'$_2$ représente l'hydrogène, un groupe alkyle en C1-C4, alcoxy en C1-C4, alkylthio en C1-C4, benzylthio, 2-phényléthylthio, diphénylméthylthio, phénylthio ou alcanoyle en C1-C4, et leurs sels acceptables pour l'usage pharmaceutique formés par addition avec des acides.

**12.** Un composé de formule Ic de la revendication 11, dans laquelle R'$_2$ représente l'hydrogène, à savoir la 5-(p-cyanophényl)-5,6,7,8-tétrahydroimidazo[1,5-a ]-pyridine et ses sels acceptables pour l'usage pharmaceutique formés par addition avec des acides.

**13.** Composés de formule Ia de la revendication 8 dans laquelle R$_1$ représente l'hydrogène, un groupe p-toluène-sulfonyloxy, benzène-sulfonyloxy, méthylsulfonyloxy, sulfo, amino, carbamoyle, (alkyle en C1-C4)-carbamoyle ou hydroxyiminométhyle; et les dérivés 5,6,7,8-tétrahydrogénés de formule Ib de la revendication 8, dans laquelle R$_1$ a les significations indiquées ci-dessus en référence à la formule La ou représente un halogène et R$_2$ représente l'hydrogène, un groupe alkyle en C1-C4, benzyle, un halogène, un groupe alcoxy en C1-C4, alcanoyloxy en C1-C4, alkylthio en C1-C4; benzylthio, 2-phényléthylthio, diphénylméthylthio, phénylthio, alcanoylthio en C1-C4, carboxy-alkyle en C1-C4, (alcoxy en C1-C4)-carbonyl-alkyle en C1-C4 ou alcanoyle en C1-C4; et leurs sels acceptables pour l'usage pharmaceutique.

**14.** Composés de formules Ia et Ib de la revendication 13 dans lesquelles R$_1$ représente un groupe carbamoyle, et composés de formule Ib dans laquelle R$_1$ représente un halogène.

**15.** Composés selon la revendication 13, de formule La dans laquelle R1 représente l'hydrogène, un groupe p-toluène-sulfonyloxy, benzène-sulfonyloxy, méthylsulfonyloxy, sulfo, amino ou hydroxyiminométhyle; et les dérivés 5,6,7,8-tétrahydrogénés de formule Ib dans laquelle R$_1$ a les significations indiquées ci-dessus en référence à la formule La et R$_2$ représente l'hydrogène; ou dans laquelle R$_1$ représente l'hydrogène, un halogène, un groupe p-toluènesulfonyloxy, benzène-sulfonyloxy, méthylsulfonyloxy, sulfo, amino, carboxy, carbamoyle, (alkyle en C1-C4)-carbamoyle, formyle ou hydroxyiminométhyle et R$_2$ représente un groupe alkyle en C1-C4, benzyle, un halogène, un groupe alcoxy en C1-C4, alcanoyloxy en C1-C4, alkylthio en C1-C4, benzylthio, 2-phényléthylthio, diphénylméthylthio, phénylthio, alcanoylthio en C1-C4, carboxyalkyle en C1-C4, (alcoxy en C1-C4)-carbonyl-alkyle en C1-C4 ou alcanoyle en C1-C4; et leurs sels acceptables pour l'usage pharmaceutique.

**16.** La (-)-5-(p-cyanophényl)-5,6,7,8-tétrahydroimidazo[1,5-a]pyridine selon la revendication 8 et ses sels acceptables pour l'usage pharmaceutique.

**17.** La (+)-5-(p-cyanophényl)-5,6,7,8-tétrahydroimidazo[1,5-a]pyridine selon la revendication 8 et ses sels acceptables pour l'usage pharmaceutique.

**18.** La 5-(p-cyanophényl)-7,8-dihydroimidazo[1,5-a]pyridine selon la revendication 8 et ses sels acceptables pour l'usage pharmàceutique.

**19.** La 5-(p-cyanophényl)-imidazo[1,5-a]pyridine selon la revendication 8 et ses sels acceptables pour l'usage pharmaceutique.

**20.** La 5-(p-bromophényl)-5,6,7,8-tétrahydroimidazo [1,5-a]pyridine selon la revendication 13, un stéréoisomère de ce composé, les mélanges de ces stéréoisomères et leurs sels acceptables pour l'usage pharmaceutique.

21. Le 5H-5-(4-cyanophényl)-6,7-dihydropyrrolo [1,2-c]imidazole selon la revendication 5, un stéréoisomère de ce composé, les mélanges de ces stéréoisomères et leurs sels acceptables pour l'usage pharmaceutique.

22. La 5H-5-(4-cyanophényl)-6,7,8,9-tétrahydroimidazo[1,5-a]azépine selon la revendication 5, un stéréoisomère de ce composé, les mélanges de ces stéréoisomères et leurs sels acceptables pour l'usage pharmaceutique.

23. La 5-(4-cyanophényl)-6-éthoxycarbonylméthyl-5,6,7,8-tétrahydroimidazo[1,5-a]pyridine selon la revendication 5, un stéréoisomere de ce composé les mélanges de ces stéréoisomères et leurs sels acceptables pour l'usage pharmaceutique.

24. La 5-(4-cyanophényl)-6-carboxyméthyl-5,6,7,8-tétrahydroimidazo[1,5-a]pyridine selon la revendication 5, un stéréoisomère de ce composé, les mélanges de ces stéréoisomeres et les sels acceptables pour l'usage pharmaceutique de ces composés.

25. La 5-benzyl-5-(4-cyanophényl)-5,6,7,8-tétrahydroimidazo[1,5-a]pyridine selon la revendication 8, un stéréoisomère de ce composé, les mélanges de ces stéréoisomères et leurs sels acceptables pour l'usage pharmaceutique.

26. La 7-(p-cyanophényl)-5,6,7,8-tétrahydroimidazo [1,5-a]pyridine, un stéréoisomère de ce composé les mélanges de ces stéréoisomères et leurs sels acceptables pour l'usage pharmaceutique.

27. Compositions pharmaceutiques contenant un composé selon la revendication 5, avec un ou plusieurs véhicules acceptables pour l'usage pharmaceutique.

28. Compositions pharmaceutiques contenant un composé selon la revendication 8, avec un ou plusieurs véhicules acceptables pour l'usage pharmaceutique.

29. Compositions pharmaceutiques contenant un composé selon la revendication 12, avec un ou plusieurs véhicules acceptables pour l'usage pharmaceutique.

30. Compositions pharmaceutiques contenant un composé selon la revendication 13, avec un ou plusieurs véhicules acceptables pour l'usage pharmaceutique.

31. Compositions pharmaceutiques contenant un composé selon la revendication 16, avec un ou plusieurs véhicules acceptables pour l'usage pharmaceutique.

32. Compositions pharmaceutiques contenant un composé selon la revendication 17, avec un ou plusieurs véhicules acceptables pour l'usage pharmaceutique.

33. Un composé selon la revendication 5 pour l'utilisation dans un procédé pour le traitement thérapeutique de l'organisme humain ou animal.

34. Un composé selon la revendication 8 pour l'utilisation dans un procédé pour le traitement thérapeutique de l'organisme humain ou animal.

35. Un composé selon la revendication 12 pour l'utilisation dans un procédé pour le traitement thérapeutique de l'organisme humain ou animal.

36. Un composé selon la revendication 13 pour l'utilisation dans un procédé pour le traitement thérapeutique de l'organisme humain ou animal.

37. Un composé selon la revendication 16 pour l'utilisation dans un procédé pour le traitement thérapeutique de l'organisme humain ou animal.

38. Un composé selon la revendication 17 pour l'utilisation dans un procédé pour le traitement thérapeutique de l'organisme humain ou animal.

**39.** Un composé selon la revendication 5 pour l'utilisation en tant qu'inhibiteur de l'aromatase.

**40.** Un composé selon la revendication 8 pour l'utilisation en tant qu'inhibiteur de l'aromatase.

**41.** Un composé selon la revendication 12 pour l'utilisation en tant qu'inhibiteur de l'aromatase.

**42.** Un composé selon la revendication 16 pour l'utilisation en tant qu'inhibiteur de l'aromatase.

**43.** Utilisation d'un composé selon la revendication 5 pour la préparation de compositions pharmaceutiques.

**44.** Utilisation d'un composé selon la revendication 8 pour la préparation de compositions pharmaceutiques.

**45.** Utilisation d'un composé selon la revendication 12 pour la préparation de compositions pharmaceutiques.

**46.** Utilisation d'un composé selon la revendication 16 pour la préparation de compositions pharmaceutiques.

**47.** Utilisation d'un composé selon la revendication 17 pour la préparation de compositions pharmaceutiques.

**48.** Utilisation d'un composé selon la revendication 5 pour la préparation de compositions pharmaceutiques prévues pour le traitement des maladies demandant une inhibition de l'aromatase.

**49.** Utilisation d'un composé selon la revendication 8 pour la préparation de compositions pharmaceutiques prévues pour le traitement des maladies demandant une inhibition de l'aromatase.

**50.** Utilisation d'un composé selon la revendication 12 pour la préparation de compositions pharmaceutiques prévues pour le traitement des maladies demandant une inhibition de l'aromatase.

**51.** Utilisation d'un composé selon la revendication 13 pour la préparation de compositions pharmaceutiques prévues pour le traitement des maladies demandant une inhibition de l'aromatase.

**52.** Utilisation d'un composé selon la revendication 16 pour la préparation de compositions pharmaceutiques prévues pour le traitement des maladies demandant une inhibition de l'aromatase.

**53.** Procédé de préparation des composés de formule I ou I* selon la revendication 5, ou de leurs sels, caractérisé en ce que

a) on cyclise un composé de formule II

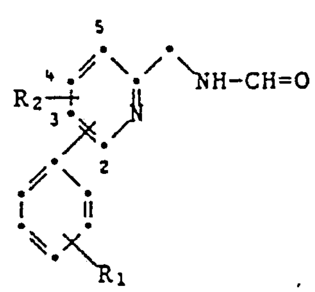

(II)

ou un dérivé 4,5-dihydrogéné d'un tel composé ce qui donne respectivement un composé de formule I ou un dérivé 7,8-dihydrogéné d'un tel composé ou bien

b) on cyclise un composé de formule III

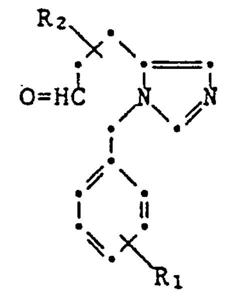

(III),

dans laquelle $R_2$, comme indiqué, peut être fixé sur l'un quelconque des atomes de carbone, y compris le carbone de carbonyle, ce qui donne un dérivé 7,8-dihydrogéné d'un composé de formule I dans lequel le substituant $C_6H_4$-$R_1$ est fixé en position 5, ou bien

c/f) dans un composé de formule IV ou VII

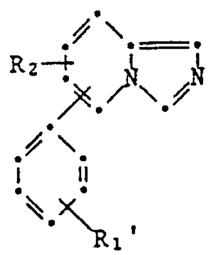

(IV)

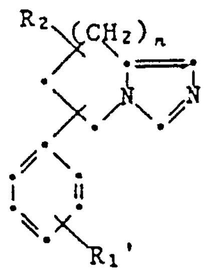

(VII)

ou dans un dérivé 7,8-dihydrogéné de formule IV pour lesquels $R'_1$ représente dans chaque cas un groupe convertible en groupe cyano, on convertit $R'_1$ en un groupe cyano, ce qui donne un composé de formule I, un dérivé 7,8-dihydrogéné d'un tel composé ou respectivement un composé de formule I*, dans lesquels $R_1$ représente un groupe cyano, ou bien

d) on cyclise un composé de formule V

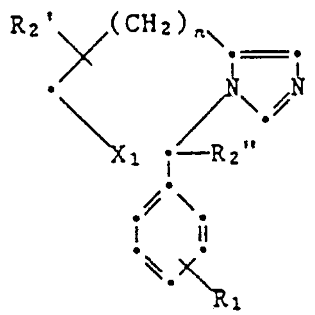

(V),

dans laquelle l'un au moins des symboles $R'_2$ et $R''_2$ représente l'hydrogène et l'autre un substituant $R_2$ tel que défini en référence à la formule I* et $X_1$ représente un groupe éliminable, $R'_2$, comme indiqué, pouvant être fixé sur l'un quelconque des atomes de carbone, ce qui donne un composé de formule I* dans lequel le substituant $C_6H_4$-$R_1$ est fixé en position 5; ou bien $X_1$ représente un groupe = CH-COOH éventuellement à l'état d'ester alkylique inférieur $R'_2$ représente l'hydrogène et $R''_2$ a les significations indiquées en référence à la formule L*, et l'on obtient par cyclisation un composé de formule I* dans laquelle le substituant $C_6H_4$-$R_1$ est fixé en position 5, et la position 6 est substituée par un groupe carboxyméthyle ou (alcoxy inférieur)-carbonylméthyle, ou bien

e) on cyclise un composé de formule VI

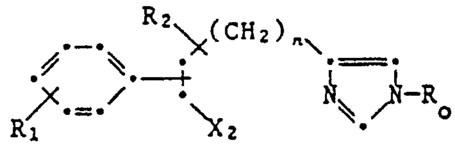

(VI)

dans laquelle les substituants $C_6H_4$-$R_1$ et $R_2$ peuvent être fixés, comme indiqué, sur l'un quelconque des atomes de carbone, soit tous deux sur le même atome de carbone, soit sur des atomes de carbone différents, $R_0$ représente un groupe protecteur du groupe NH ou l'hydrogène et $X_2$ un groupe éliminable, ce qui donne un composé de formule I*; ou bien

g) on cyclise un composé de formule IX

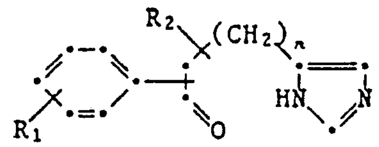

(IX)

dans laquelle les substituants $C_6H_4$-$R_1$ et $R_2$, comme indiqué, peuvent être fixés sur l'un quelconque des atomes de carbone, y compris le carbone de carbonyle, soit tous deux sur le même atome de carbone, soit sur des atomes de carbone différents, éventuellement dans des conditions réductrices, ce qui donne un dérivé 7,8-dihydrogéné d'un composé de formule I ou bien, lorsqu'on a opéré dans des conditions réductrices, un composé de formule L*, ou bien

h) on soumet à décarboxylation un composé répondant par ailleurs à la formule I, ou un dérivé 7,8-dihydrogéné d'un tel composé ou un composé répondant par ailleurs à la formule I*, mais tous ces composés portant en outre un groupe carboxy en position 1 ou 3, ce qui donne respectivement un composé de formule I un dérivé 7,8-dihydrogéné d'un tel composé ou un composé de formule I*; les symboles n $R_1$ et $R_2$ ayant, pour les composés de départ de formules II à VIL et IX, les significations indiquées respectivement en référence aux formules I et I*; et/ou on réduit un composé de formule I ou un dérivé 7,8-dihydrogéné d'un tel composé en un dérivant correspondant 5,6,7,8-tétrahydrogéné de formule I*, avec, le cas échéant, réduction simultanée des substituants $R_1$ et/ou $R_2$ en d'autres substituants $R_1$ et/ou $R_2$; et/ou on soumet à décarboxylation un composé de formule I* dans laquelle $R_2$ représente un groupe carboxy, ce qui donne un composé de formule I* dans laquelle $R_2$ représente l'hydrogène; et/ou on convertit un composé ainsi obtenu en un autre composé de l'invention et/ou un sel ainsi obtenu en le composé libre ou en un autre sel, et/ou un composé obtenu à l'état libre en un sel, et/ou on sépare un mélange d'isomères ou de racémates ainsi obtenu en les isomères ou racémates individuels et/ou on résout un mélange d'énantiomères, par exemple un recémate, en les antipodes optiques.

**54.** Procédé de préparation des composés de formule Ia ou Ib de la revendication 8 ou de leurs sels caractérisé en ce que

a) on cyclise en milieu acide un composé de formule IIa

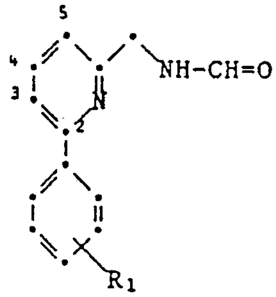

(IIa),

dans laquelle $R_1$ a les significations indiquées ci-dessus en référence à la formule Ia ou un dérivé 4,5-dihydrogéné d'un tel composé, ce qui donne respectivement un composé de formule Ia ou un dérivé 7,8-dihydrogéné d'un tel composé, ou bien

b) pour préparer un dérivé 7,8-dihydrogéné d'un composé de formule Ia, on cyclise en milieu basique un composé de formule IIIa

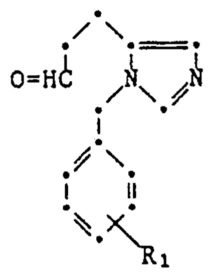

(IIIa),

dans laquelle $R_1$ a les significations indiquées ci-dessus en référence à la formule Ia, ou bien
c) dans un composé de formule IVa

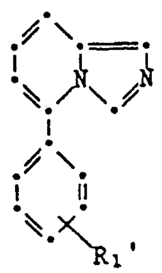

(IVa),

dans laquelle $R'_1$ représente un substituant convertible en groupe cyano, ou dans un dérivé 7,8-dihydrogéné d'un tel composé, on convertit R'1 en groupe cyano, ce qui donne un composé de formule Ia ou un dérivé 7,8-dihydrogéné d'un tel composé, ou bien
d) on cyclise en présence d'une base un composé de formule Vb

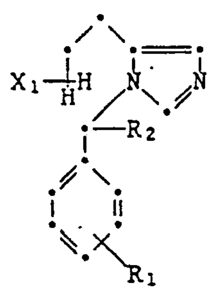

(Vb),

dans laquelle $R_1$ et $R_2$ ont les significations indiquées ci-dessus en référence à la formule Ib et $X_1$ représente un groupe éliminable, ce qui donne un composé de formule Ib, ou bien
e) on cyclise en présence d'une base un composé de formule VIb

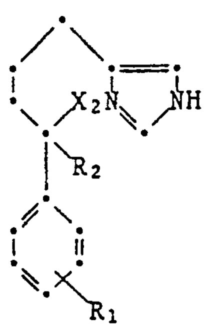

(VIb),

dans laquelle $R_1$ et $R_2$ ont les significations indiquées ci-dessus en référence à la formule Ib et $X_2$

représente un groupe éliminable, le groupe NH pouvant être protégé par un groupe protecteur approprié, ce qui donne un composé de formule Ib, ou bien

f) dans un composé de formule VIIb

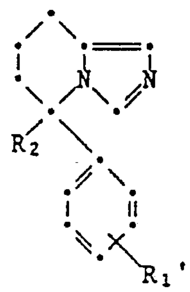

(VIIb),

dans laquelle R'$_1$ représente un substituant convertible en groupe cyano et $R_2$ a les significations indiquées ci-dessus en référence à la formule Ib, on convertit le substituant R'$_1$ en groupe cyano, ce qui donne un composé de formule Ib dans laquelle $R_1$ représente un groupe cyano;

g) on cyclise un composé de formule IXb

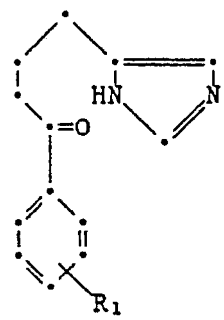

(IXb),

éventuellement dans des conditions réductrices, ce qui donne un dérivé 7,8-dihydrogéné d'un composé de formule Ia ou bien, lorsqu'on a opéré dans des conditions réductrices, un composé de formule Ib, ou bien

h) on soumet à décarboxylation un composé répondant par ailleurs à la formule Ia, ou un dérivé 7,8-dihydrogéné d'un tel composé, ou un composé répondant par ailleurs à la formule Ib tous ces composés portant toutefois un groupe carboxy supplémentaire en position 1 ou 3, ce qui donne un composé de formule Ia, un dérivé 7,8-dihydrogéné d'un tel composé ou un composé de formule Ib, et/ou on soumet à décarboxylation un composé de formule Ib dans laquelle $R_2$ représente un groupe carboxy, ce qui donne un autre composé de formule Ib dans laquelle $R_2$ représente l'hydrogène, et/ou on réduit un composé de formule Ia, ou un dérivé 7,8-dihydrogéné d'un tel composé par l'hydrogène en présence d'un catalyseur d'hydrogénation, en le dérivé correspondant 5,6,7,8-tétrahydrogéné de formule Ib, et/ou on convertit un composé ainsi obtenu en un au tre composé de l'invention, et/ou on convertit un sel ainsi obtenu en le composé libre ou en un autre sel, et/ou on convertit un composé obtenu à l'état libre et qui porte un groupe salifiable, en un sel, et/ou on sépare un mélange racémique ainsi obtenu en les énantiomères individuels.

**55.** Les composés obtenus par le procédé de la revendication 53.

**56.** Les composés obtenus par le procédé de la revendication 54.

Revendications pour l'Etat Contractant: AT

**1.** Procédé de préparation des composés de formule I

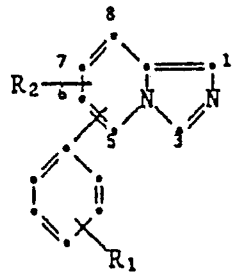

(I)

dans laquelle $R_1$ représente l'hydrogène, un groupe alkyle inférieur; un groupe alkyle en C2-C7 substitué par des groupes hydroxy, alcoxy inférieurs, des halogènes ou des groupes alcanoyloxy inférieurs; un groupe alkyle inférieur substitué par des groupes alcanoyle inférieurs, amino, alkylamino inférieurs, di-(alkyle inférieur)-amino, sulfo,(alcoxy inférieur)-carbonyle, carbamoyle ou cyano; un groupe nitro, alcoxy inférieur, alcanoyloxy inférieur, phénylsulfonyloxy, alkylsulfonyloxy inférieur, alkylthio inférieur, alkylsulfinyle inférieur, alkylsulfonyle inférieur, alcanoylthio inférieurs, amino, alkylamino inférieur, di-(alkyle inférieur)-amino alkylène-amino inférieur, N-morpholino, N-thiomorpholino, un groupe N-pipérazino éventuellement substitué en position 4 par un groupe alkyle inférieur, un groupe tri-(alkyle inférieur)-ammonio, sulfo, alcoxysulfonyle inférieur, sulfamoyle, alkylsulfamoyle inférieur, di-(alkyle inférieur)-sulfamoyle; un groupe iminométhyle éventuellement substitué sur l'azote par un groupe hydroxy, alcoxy inférieur, alcanoyloxy inférieur; alkyle inférieur, phényle ou amino; un groupe alcanoyle en C2-C7 ou benzoyle, et $R_2$ représente l'hydrogène, un groupe alkyle inférieur, phényl-alkyle inférieur, carboxy-alkyle inférieur, (alcoxy inférieur)-carbonyl-alkyle inférieur, un halogène, un groupe hydroxy, alcoxy inférieur, alcanoyloxy inférieur, mercapto, alkylthio inférieur, phényl-alkylthio inférieur, phénylthio, alcanoylthio inférieur, carboxy, (alcoxy inférieur)-carbonyle ou alcanoyle inférieur; de leurs dérivés 7,8-dihydrogénés et également des dérivés 7,8-dihydrogénés pour lesquels $R_1$ représente un groupe hydroxyméthyle, (alcoxy inférieur)-méthyle, halogénométhyle, (alcanoyle inférieur)-oxyméthyle, carboxy-alkyle inférieur, un halogène, un groupe hydroxy, mercapto, formyle, carboxy, (alcoxy inférieur)-carbonyle, carbamoyle, (alkyle inférieur)-carbamoyle, di-(alkyle inférieur)-carbamoyle, cyano, 5-tétrazolyle, 4,5-dihydro-2-oxazolyle ou hydroxycarbamoyle éventuellement substitué par un groupe alkyle inférieur et $R_2$ a les significations indiquées ci-dessus en référence à la formule I; et des composés de formule I*

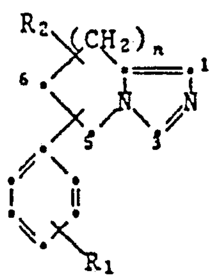

(I*)

dans laquelle n est égal à 0, 1, 2, 3 ou 4; $R_1$ a les significations indiquées en référence à la formule I mais peut en outre représenter un groupe hydroxyméthyle, (alcoxy inférieur)-méthyle, halogénométhyle, (alcanoyle inférieur)-oxyméthyle, carboxy-alkyle inférieur, un halogène, un groupe hydroxy, mercapto, formyle, carboxy, (alcoxy inférieur)-carbonyle carbamoyle, (alkyle inférieur)-carbamoyle, di-(alkyle inférieur)-carbamoyle, cyano, 5-tétrazolyle, 4,5-dihydro-2-oxazolyle ou hydroxycarbamoyle éventuellement substitué par un groupe alkyle inférieur lorsque n est égal à 0, 1, 3 ou 4 ou lorsque n est égal à 2 et que $R_2$ représente un groupe phényl-alkyle inférieur, carboxy-alkyle inférieur, (alcoxy inférieur)-carbonylalkyle inférieur, alcanoyloxy inférieur, mercapto, alkylthio inférieur, phényl-alkylthio inférieur, phénylthio, alcanoylthio inférieur, carboxy, (alcoxy inférieur)-carbonyle ou alcanoyle inférieur; et $R_2$ a les significations indiquées ci-dessus en référence à la formule I; le noyau phényle des groupes phénylsulfonyloxy, phényliminométhyle, benzoyle, phényl-alkyle inférieur, phénylalkylthio inférieur et phénylthio pouvant être non substitué ou substitué par des groupes alkyle inférieurs, alcoxy inférieurs ou des halogènes; les deux substituants $C_6H_4$-$R_1$ et $R_2$ d'un composé de formule L* pouvant être fixés

sur l'un quelconque des atomes de carbone saturés du cycle saturé, soit tous deux sur le même atome de carbone, soit sur des atomes de carbone différents; les substituants qualifiés d'"inférieurs" contenant jusqu'à 7 atomes de carbone; de leurs stéréoisomères, des mélanges de ces stéréoisomères et des sels de ces composés, caractérisé en ce que :

a) on cyclise un composé de formule II

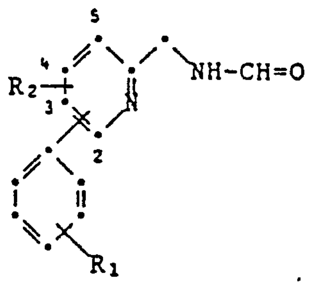

(II)

ou un dérivé 4,5-dihydrogéné d'un tel composé, ce qui donne un composé de formule I ou un dérivé 7,8-dihydrogéné d'un tel composé, ou bien

b) on cyclise un composé de formule III

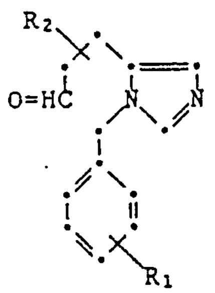

(III),

dans laquelle $R_2$,comme indiqué sur la figure, peut être fixé sur l'un quelconque des atomes de carbone, y compris le carbone de carbonyle, ce qui donne un dérivé 7,8-dihydrogéné d'un composé de formule I pour lequel le substituant $C_6H_4$-$R_1$ est fixé en position 5, ou bien

c/f) dans un composé de formule IV ou VII

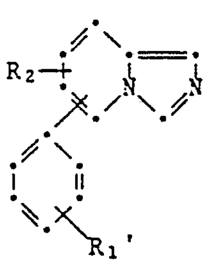

(IV)

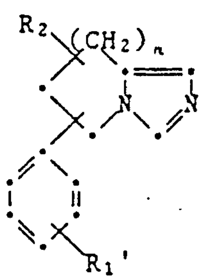

(VII)

ou dans un dérivé 7,8-dihydrogéné de formule IV, pour lesquels $R'_1$ représente un groupe convertible en groupe cyano, on convertit $R'_1$ en groupe cyano, ce qui donne un composé de formule I, un dérivé 7,8-dihydrogéné d'un tel composé ou respectivement un composé de formule I*, pour lesquels $R_1$ représente un groupe cyano, ou bien

d) on cyclise un composé de formule V

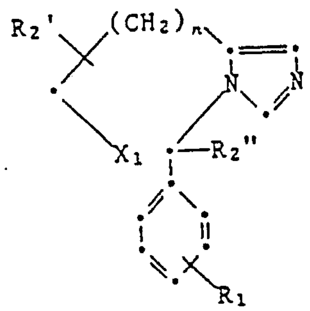

(V),

dans laquelle l'un au moins des symboles $R'_2$ et $R''_2$ représente l'hydrogène et l'autre un groupe $R_2$ tel que défini en référence à la formule I*, et $X_1$ représente un groupe éliminable, $R'_2$, comme représenté sur la figure, pouvant être fixé sur l'un quelconque des atomes de carbone, ce qui donne un composé de formule I* pour lequel le substituant $C_6H_4$-$R_1$ est fixé en position 5; ou bien $X_1$ représente un groupe =CH-COOH éventuellement à l'état d'ester alkylique inférieur, $R'_2$ représente l'hydrogène et $R''_2$ a les significations indiquées en référence à la formule I*, et l'on obtient un composé de formule I* pour lequel le substituant $C_6H_4$-$R_1$ est fixé en position 5, et la position 6 est substituée par un groupe carboxyméthyle ou (alcoxy inférieur)-carbonylméthyle, ou bien

e) on cyclise un composé de formule VI

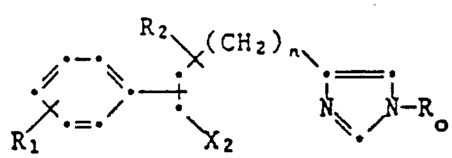

(VI)

dans laquelle les substituants $C_6H_4$-$R_1$ et $R_2$ peuvent être fixés, comme indiqué, sur l'un quelconque des atomes de carbone, soit tous deux sur le même atome de carbone, soit sur des atomes de carbone différents, $R_0$ représente un groupe protecteur du groupe NH ou l'hydrogène et $X_2$ un groupe éliminable, ce qui donne un composé de formule I*; ou bien

g) on cyclise un composé de formule IX

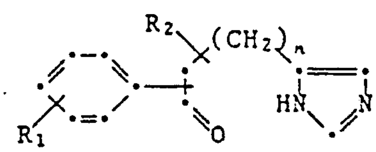

(IX)

dans laquelle les substituants $C_6H_4$-$R_1$ et $R_2$ peuvent être fixés, comme indiqué, sur l'un quelconque des atomes de carbone, y compris le carbone de carbonyle, soit tous deux sur le même atome de carbone, soit sur des atomes de carbone différents, le cas échéant dans des conditions réductrices, et l'on obtient un dérivé 7,8-dihydrogéné d'un composé de formule I ou bien, dans le cas où on opère dans des conditions réductrices, un composé de formule I*, ou bien

h) partant d'un composé répondant à la formule I, ou d'un dérivé 7,8-dihydrogéné d'un tel composé, ou d'un composé répondant à la formule I* mais qui-portent tous un groupe carboxy supplémentaire en position 1 ou 3, on soumet à décarboxylation, et l'on obtient donc un composé de formule I, un composé 7,8-dihydrogéné d'un tel composé ou un composé de formule I*; les symboles n, $R_1$ et $R_2$ des formules II à VII et IX des composés de départ ayant les significations indiquées respectivement en référence aux formules I et I*; et/ou on réduit un composé de formule I ou un dérivé 7,8-dihydrogéné d'un tel composé en le dérivé correspondant 5,6,7,8-tétrahydrogéné de formule I*, avec le cas échéant, simultanément, une réduction des substituants $R_1$ et/ou $R_2$ en d'autres substituants $R_1$ et/ou $R_2$; et/ou on soumet à décarboxylation un composé de formule I* dans laquelle $R_2$ représente un groupe carboxy, ce qui donne un composé de formule I* dans laquelle $R_2$ représente

l'hydrogène; et/ou on convertit un composé obtenu en un autre composé selon l'invention et/ou un sel obtenu en le composé libre ou en un autre sel et/ou un composé obtenu à l'état libre en un sel et/ou on sépare un mélange d'isomères ou de racémates en les isomères ou racémates individuels et/ou on résout un mélange d'énantiomères, par exemple un racémate, en les antipodes optiques.

2. Procédé selon la revendication 1, caractérisé en ce que l'on prépare des composés de formule I dans laquelle $R_1$ représente un groupe alkyle inférieur hydroxyalkyle en C2-C7; un groupe alkyle inférieur substitué par des groupes amino, di-(alkyle inférieur)-amino, deux à cinq atomes de fluor, des groupes (alcoxy inférieur) carbonyle, carbamoyle ou cyano; un groupe nitro, alcoxy inférieur, amino, alkylamino inférieur, di-(alkyle inférieur)-amino, sulfo, sulfamoyle, iminométhyle ou un groupe iminométhyle substitué à l'azote par un groupe hydroxy, alcoxy inférieur, alcanoyloxy inférieur, alkyle inférieur ou phényle, et $R_2$ représente l'hydrogène, un groupe alkyle inférieur, alcoxy inférieur ou un halogène; ou bien des composés de formule I* dans laquelle n est égal à 1, 2 ou 3; $R_1$ a les significations indiquées en référence à la formule I mais peut en outre représenter un groupe hydroxyméthyle, carboxy-alkyle inférieur, un halogène, un groupe hydroxy, formyle, carboxy; (alcoxy inférieur)-carbonyle, carbamoyle, (alkyle inférieur)-carbamoyle, di-(alkyle inférieur)-carbamoyle ou cyano lorsque n est égal à 1 ou 3 ou bien lorsque n est égal à 2 et que $R_2$ représente un groupe phényl-alkyle inférieur, carboxy-alkyle inférieur, (alcoxy inférieur)-carbonylalkyle inférieur, alkylthio inférieur, phényl-alkylthio inférieur, phénylthio, carboxy, (alcoxy inférieur)-carbonyle ou alcanoyle inférieur; et $R_2$ représente l'hydrogène, un groupe alkyle inférieur, phényl-alkyle inférieur, carboxy-alkyle inférieur, (alcoxy inférieur)-carbonyl-alkyle inférieur, un halogène, un groupe alcoxy inférieur, alkylthio inférieur, phényl-alkylthio inférieur, phénylthio, carboxy, (alcoxy inférieur)-carbonyle ou alcanoyle inférieur; les deux substituants $C_6H_4$-$R_1$ et $R_2$ d'un composé de formule I* pouvant être fixés sur un atome de carbone saturé quelconque du cycle saturé, soit tous deux sur le même atome de carbone, soit sur des atomes de carbone différents; leursstéréoisomères, les mélanges de ces stéréoisomères ou des sels acceptables pour l'usage pharmaceutique de ces composés.

3. Procédé selon la revendication 1, caractérisé en ce que l'on prépare des composés de formule I* dans laquelle n est égal à 1, 2 ou 3; $R_1$ représente un groupe alkyle inférieur amino, alkylamino inférieur ou di-(alkyle inférieur)-amino mais peut en outre représenter un groupe hydroxyméthyle, un halogène, un groupe formyle, carboxy, (alcoxy inférieur)-carbonyle, carbamoyle, (alkyle inférieur)-carbamoyle, di-(alkyle inférieur)-carbamoyle ou cyano lorsque n est égal à 1 ou 3 ou bien lorsque n est égal à 2 et que $R_2$ représente un groupe phénylalkyle inférieur, carboxyalkyle inférieur, (alcoxy inférieur)-carbonylalkyle inférieur, alkylthio inférieur, carboxy ou (alcoxy inférieur)-carbonyle; et $R_2$ représente l'hydrogène, un groupe alkyle inférieur, phénylalkyle inférieur, carboxy-alkyle inférieur, (alcoxy inférieur)-carbonyl-alkyle inférieur, alkylthio inférieur, carboxy ou (alcoxy inférieur)-carbonyle; les deux substituants $C_6H_4$-$R_1$ et $R_2$ pouvant être fixés sur l'un quelconque des atomes de carbone saturés du cycle saturé, soit tous deux sur le même atome de carbone, soit sur des atomes de carbone différents; leurs stéréoisomères, les mélanges de ces stéréoisomères ou les sels acceptables pour l'usage pharmaceutique de ces composés.

4. Procédé de préparation des composés de formule Ia

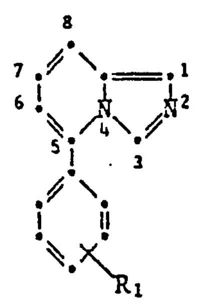

(Ia)

dans laquelle $R_1$ représente un groupe cyano, nitro ou alkyle en C1-C4, de leurs dérivés 7,8-dihydrogénés ou de leurs dérivés 5,6,7,8-tétrahydrogénés répondant à la formule Ib

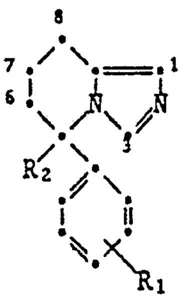

(Ib)

dans laquelle $R_1$ a les significations indiquées en référence à la formule Ia et $R_2$ représente l'hydrogène, un groupe alkyle en C1-C4, benzyle, un halogène, un groupe alcoxy en C1-C4, alkylthio en C1-C4, benzylthio, 2-phényléthylthio, diphénylméthylthio, phénylthio, alcanoyloxy en C1-C4, alcanoylthio en C1-C4, carboxy-alkyle en C1-C4, (alcoxy en C1-C4)-carbonyl-alkyle en C1-C4 ou alcanoyle en C1-C4, de leurs stéréoisomères, des mélanges de ces stéréoisomères et des sels de ces composés, caractérisé en ce que :

a) on cyclise un composé de formule IIa

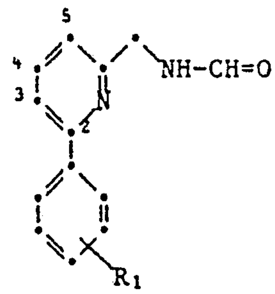

(IIa),

dans laquelle $R_1$ a les significations indiquées en référence à la formule Ia, ou un dérivé 4,5-dihydrogéné d'un tel composé, en milieu acide, et l'on obtient un composé de formule Ia ou un dérivé 7,8-dihydrogéné d'un tel composé, ou bien

b) pour préparer un dérivé 7,8-dihydrogéné d'un composé de formule Ia, on cyclise un composé de formule IIIa

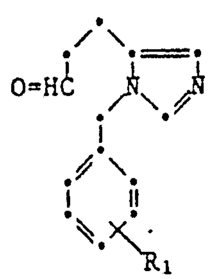

(IIIa),

dans laquelle $R_1$ a les significations indiquées en référence à la formule Ia, en milieu basique, ou bien

c) dans un composé de formule IVa

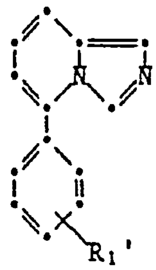

(IVa),

dans laquelle R'$_1$ représente un substituant convertible en groupe cyano, ou dans un dérivé 7,8-dihydrogéné d'un tel composé, on convertit R'$_1$ en groupe cyano, et l'on obtient un composé de formule Ia ou un dérivé 7,8-dihydrogéné d'un tel composé, ou bien

d) on cyclise un composé de formule Vb

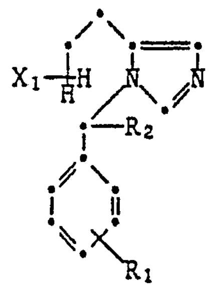

(Vb),

dans laquelle $R_1$ et $R_2$ ont les significations indiquées ci-dessus en référence à la formule Ib et $X_1$ représente un groupe éliminable, en présence d'une base, ce qui donne un composé de formule Ib, ou bien

e) on cyclise un composé de formule VIb

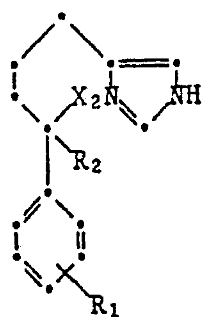

(VIb),

dans laquelle $R_1$ et $R_2$ ont les significations indiquées ci-dessus en référence à la formule Ib et $X_2$ représente un groupe éliminable, et dans lequel le groupe NH peut être protégé par un groupe protecteur approprié, en présence d'une base, ce qui donne un composé de formule Ib, ou bien

f) dans un composé de formule VIIb

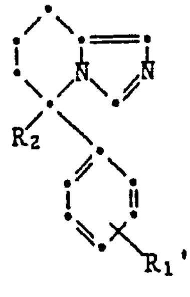

(VIIb),

dans laquelle $R'_1$ représente un groupe convertible en groupe cyano et $R_2$ a les significations indiquées ci-dessus en référence à la formule Ib, on convertit $R'_1$ en groupe cyano et l'on obtient un composé de formule Ib dans laquelle $R_1$ représente un groupe cyano;
g) on cyclise un composé de formule IXb

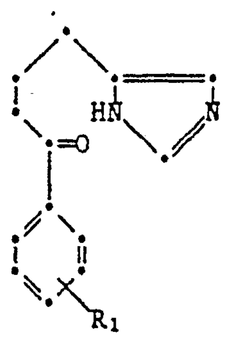

(IXb),

éventuellement dans des conditions réductrices, et l'on obtient un dérivé 7,8-dihydrogéné d'un composé de formule La ou bien, lorsqu'on opère dans des conditions réductrices, un composé de formule Ib, ou bien
h) on soumet un composé répondant par ailleurs à la formule Ia ou un dérivé 7,8-dihydrogéné d'un tel composé, ou un composé répondant par ailleurs à la formule Ib mais qui contiennent tous un groupe carboxy supplémentaire en position 1 ou 3, à décarboxylation, ce qui donne respectivement un composé de formule Ia, un dérivé 7,8-dihydrogéné d'un tel composé ou un composé de formule Ib, et/ou on soumet à décarboxylation un composé de formule Ib dans laquelle $R_2$ représente un groupe carboxy, ce qui donne un autre composé de formule Ib dans laquelle $R_2$ représente l'hydrogène, et/ou on réduit un composé de formule Ia ou un dérivé 7,8-dihydrogéné d'un tel composé, par l'hydrogène en présence d'un catalyseur d'hydrogénation, en le dérivé correspondant 5,6,7,8-tétrahydrogéné de formule Ib, et/ou on convertit un composé obtenu en un autre composé de l'invention et/ou un sel ainsi obtenu en le composé libre ou en un autre sel, et/ou un composé libre contenant un groupe salifiable, en un sel, et/ou on sépare un mélange racémique ainsi obtenu en les énantiomères individuels.

**5.** Procédé selon la revendication 4, caractérisé en ce que l'on exécute l'une des variantes opératoires a), b), c), d) ou f) de la revendication 4, ou bien e) on cyclise un composé de formule VIb

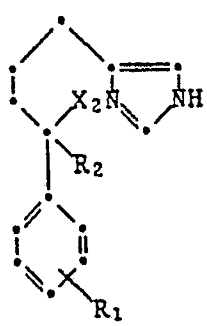

(VIb),

dans laquelle $R_1$ et $R_2$ ont les significations indiquées ci-dessus en référence à la formule Ib et $X_2$ représente un groupe éliminable, en présence d'une base, ce qui donne un composé de formule Ib, et/ou on réduit un composé de formule Ia ou un dérivé 7,8-dihydrogéné d'un tel composé, par l'hydrogène en présence d'un catalyseur d'hydrogénation, en le dérivé 5,6,7,8-tétrahydrogéné correspondant de formule Ib, et/ou on convertit un composé ainsi obtenu en un autre composé de l'invention, et/ou un sel ainsi obtenu en le composé libre ou en un autre sel, et/ou un composé libre contenant un groupe salifiable en un sel, et/ou on sépare un mélange racémique ainsi obtenu en les énantiomères individuels.

**6.** Procédé selon la revendication 5, caractérisé en ce que l'on prépare les composés de formule Ia dans

laquelle $R_1$ représente un groupe cyano, les dérivés 7,8-dihydrogénés de ces composés ou les dérivés 5,6,7,8-tétrahydrogénés de ces composés, répondant à la formule Ib dans laquelle $R_1$ représente un groupe cyano et $R_2$ représente l'hydrogène, un groupe alkyle en C1-C4, alcoxy en C1-C4, alkylthio en C1-C4, benzylthio, 2-phényléthylthio, diphénylméthylthio, phénylthio ou alcanoyle en C1-C4, ou les sels formés par addition avec des acides acceptables pour l'usage pharmaceutique des composés de formules Ia et Ib;

7. Procédé selon la revendication 5, caractérisé en ce que l'on prépare des composés de formule Ia dans laquelle $R_1$ représente un groupe cyano, leurs dérivés 7,8-dihydrogénés ou leurs dérivés 5,6,7,8-tétrahydrogénés de formule Ib dans laquelle $R_1$ a les significations indiquées ci-dessus en référence à la formule Ia et $R_2$ représente l'hydrogène, ou les sels de ces composés formés par addition avec des acides acceptables pour l'usage pharmaceutique.

8. Procédé selon la revendication 5, caractérisé en ce que l'on prépare des composés de formule Ic

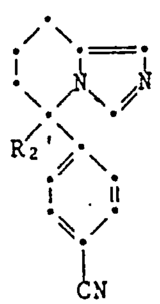

(Ic)

dans laquelle $R'_2$ représente l'hydrogène, un groupe alkyle en C1-C4, alcoxy en C1-C4, alkylthio en C1-C4, benzylthio, 2-phényléthylthio, diphénylméthylthio, phénylthio ou alcanoyle en C1-C4, ou des sels de ces composés formés par addition avec des acides acceptables pour l'usage pharmaceutique.

9. Procédé selon la revendication 5, caractérisé en ce que l'on prépare un composé de formule Ic dans laquelle $R'_2$ représente l'hydrogène, à savoir la 5-(p-cyanophényl)-5,6,7,8-tétrahydroimidazo[1,5-a]-pyridine ou ses sels formés par addition avec des acides acceptables pour l'usage pharmaceutique.

10. Procédé selon la revendication 4, caractérisé en ce que l'on prépare un composé de formule Ic dans laquelle $R'_2$ représente l'hydrogène, à savoir la 5-(p-cyanophényl)-5,6,7,8-tétrahydroimidazo[1,5-a]-pyridine ou ses sels formés par addition avec des acides acceptables pour l'usage pharmaceutique.

11. Procédé de préparation des composés de formule Ia de la revendication 4, dans laquelle $R_1$ représente l'hydrogène, un groupe p-toluène-sulfonyloxy, benzènesulfonyloxy, méthylsulfonyloxy, sulfo, amino, carbamoyle, (alkyle en C 1-C 4)-carbamoyle ou hydroxyiminométhyle; ou des dérivés 5,6,7,8-tétrahydrogénés de formule Ib de la revendication 4 dans laquelle $R_1$ a les significations indiquées en référence à la formule Ia ou représente un halogène et $R_2$ représente l'hydrogène, un groupe alkyle en C 1-C 4, benzyle, un halogène, un groupe alcoxy en C 1-C 4, alcanoyloxy en C 1-C 4, alkylthio en C 1-C 4, benzylthio, 2-phényléthylthio, diphénylméthylthio, phénylthio, alcanoylthio en C 1-C 4, carboxy-alkyle en C 1-C 4, (alcoxy en C 1-C 4)-carbonyl-alkyle en C 1-C 4 ou alcanoyle en C 1-C 4; ou de leurs sels acceptables pour l'usage pharmaceutique, caractérisé en ce que l'on exécute les variantes opératoires de la revendication 4.

12. Procédé de préparation des composés de formule Ia de la revendication 4, dans laquelle $R_1$ représente l'hydrogène, un groupe p-toluène-sulfonyloxy, benzènesulfonyloxy, méthylsulfonyloxy, sulfo, amino ou hydroxyiminométhyle; ou des dérivés 5,6,7,8-tétrahydrogénés de formule Ib de la revendication 4 dans laquelle $R_1$ a les significations indiquées ci-dessus en référence à la formule Ia et $R_2$ représente l'hydrogène; ou bien dans laquelle $R_1$ représente l'hydrogène, un halogène, un groupe p-toluène-sulfonyloxy, benzène-sulfonyloxy, méthylsulfonyloxy, sulfo, amino, carboxy, carbamoyle, (alkyle en C 1-C 4)-carbamoyle, formyle ou hydroxyiminométhyle et $R_2$ représente un groupe alkyle en C 1-C 4, benzyle, un halogène, un groupe alcoxy en C 1-C 4, alcanoyloxy en C 1-C 4, alkylthio en C 1-C 4, benzylthio, 2-phényléthylthio, diphénylméthylthio, phénylthio, alcanoylthio en C 1-C 4, carboxy-alkyle en C 1-C 4, (alcoxy en C 1-C 4)-carbonyl-alkyle en C 1-C 4 ou alcanoyle en C 1-C 4; ou de leurs sels

acceptables pour l'usage pharmaceutique, caractérisé en ce que l'on exécute les variantes opératoires de la revendication 5.

**13.** Procédé selon la revendication 12, caractérisé en ce que l'on prépare des composés de formule Ia et Ib dans lesquelles $R_1$ représente un groupe carbamoyle ou des composés de formule Ib dans laquelle $R_1$ représente un halogène.

**14.** Procédé selon la revendication 4, caractérisé en ce que l'on prépare la (-)-5-(p-cyanophényl)-5,6,7,8-tétrahydroimidazo[1,5-a]pyridine ou ses sels acceptables pour l'usage pharmaceutique.

**15.** Procédé selon la revendication 4, caractérisé en ce que l'on prépare la (+)-5-(p-cyanophényl)-5,6,7,8-tétrahydroimidazo[1,5-a]pyridine ou ses sels acceptables pour l'usage pharmaceutique.

**16.** Procédé selon la revendication 5, caractérisé en ce que l'on prépare la 5-(p-cyanophényl)-7,8-dihydroimidazo[1,5-a]pyridine ou ses sels acceptables pour l'usage pharmaceutique.

**17.** Procédé selon la revendication 5, caractérisé en ce que l'on prépare la 5-(p-cyanophényl)imidazo[1,5-a]pyridine ou ses sels acceptables pour l'usage pharmaceutique.

**18.** Procédé selon la revendication 11, caractérisé en ce que l'on prépare la 5-(p-bromophényl)-5,6,7,8-tétrahydroimidazo[1,5-a]pyridine, un stéréoisomère de ce composé, un mélange de ces stéréoisomères ou un sel acceptable pour l'usage pharmaceutique de ce composé.

**19.** Procédé selon la revendication 1, caractérisé en ce que l'on prépare le 5H-5-(4-cyanophényl)-6,7-dihydropyrrolo[1,5-a]imidazole, un stéréoisomère de ce composé, un mélange de ces stéréoisomères ou un sel acceptable pour l'usage pharmaceutique de ce composé.

**20.** Procédé selon la revendication 1, caractérisé en ce que l'on prépare la 5H-5-(4-cyanophényl)-6,7,8,9-tétrahydroimidazo[1,5-a]azépine, un stéréoisomére de ce composé, un mélange de ces stéréoisomères ou un sel acceptable pour l'usage pharmaceutique de ce composé.

**21.** Procédé selon la revendication 1, caractérisé en ce que l'on prépare la 5-(4-cyanophényl)-6-éthoxycarbonylméthyl-5,6,7,8-tétrahydroimidazo[1,5-a]pyridine, un stéréoisomère de ce composé, un mélange de ces stéréoiscmères, ou un sel acceptable pour l'usage pharmaceutique de ce composé.

**22.** Procédé selon la revendication 4, caractérisé en ce que l'on prépare la 5-(p-cyanophényl)-imidazo [1,5-a]pyridine ou ses sels acceptables pour l'usage pharmaceutique.

**23.** Procédé selon la revendication 12, caractérisé en ce que l'on prépare la 5-(p-bromophényl)-5,6,7,8-tétrahydroimidazo[1,5-a]pyridine, un stéréoisomère de ce composé, un mélange de ces stéréoisomères ou leurs sels acceptables pour l'usage pharmaceutique.

**24.** Procédé selon la revendication 1, caractérisé en ce que l'on prépare la 5-(4-cyanophényl)-6-carboxyméthyl-5,6,7,8-tétrahydroimidazo[1,5-a]pyridine, un stéréoisomère de ce composé, un mélange de ces stéréoisomères ou leurs sels acceptables pour l'usage pharmaceutique.

**25.** Procédé selon la revendication 4, caractérisé en ce que l'on prépare la 5-benzyl-5-(4-cyanophényl)-5,6,7,8-tétrahydroimidazo[1,5-a]pyridine, un stéréoisomère de ce composé, un mélange de ces stéréoisomères ou leurs sels acceptables pour l'usage pharmaceutique.

**26.** Procédé de préparation de la 7-(p-cyanophényl)-5,6,7,8-tétrahydroimidazo[1,5-a]pyridine, d'un stéréoisomère de ce composé, d'un mélange de ces stéréoisomères ou de leurs sels acceptables pour l'usage pharmaceutique, caractérisé en ce que l'on exécute les variantes opératoires de la revendication 1.

**27.** Procédé de préparation de compositions pharmaceutiques, caractérisé en ce que l'on mélange un composé obtenu par le procédé selon l'une des revendications 1, 4, 10, 11, 14 ou 15, ou un sel acceptable pour l'usage pharmaceutique d'un tel composé, avec un véhicule acceptable pour l'usage

pharmaceutique.

**28.** Procédé de préparation de compositions pharmaceutiques, caractérisé en ce que l'on mélange un composé obtenu par le procédé selon l'une des revendications 5, 9 ou 12, ou un sel acceptable pour l'usage pharmaceutique d'un tel composé, avec un véhicule acceptable pour l'usage pharmaceutique.